# EUROPEAN PATENT APPLICATION

(11) **EP 4 289 833 A1**
(43) Date of publication of application: **13.12.2023**
(21) Application number: 22749196.6
(22) Date of filing: 30.01.2022
(51) Int. Cl.: C07D 401/12, C07D 401/14, C07D 403/12, C07D 403/14, C07D 413/12, C07D 413/14, C07D 417/12, C07D 417/14, A61K 31/501, A61P 29/00, A61P 37/00

(54) **TYK2 INHIBITOR COMPOUND CONTAINING BICYCLIC RING**

(30) Priority: 06.02.2021 CN 202110165856; 16.08.2021 CN 202110935764; 10.09.2021 CN 202111063960; 18.01.2022 CN 202210056272
(71) Applicant: CHIA TAI TIANQING PHARMACEUTICAL GROUP CO., LTD., Lianyungang, Jiangsu 222062 (CN)
(72) Inventor: LIU, Fei, Lianyungang, Jiangsu 222062 (CN); LIU, Yanlong, Lianyungang, Jiangsu 222062 (CN); WANG, Bin, Lianyungang, Jiangsu 222062 (CN); XU, Hongjiang, Lianyungang, Jiangsu 222062 (CN); TANG, Xujing, Lianyungang, Jiangsu 222062 (CN)
(74) Representative: WSL Patentanwälte Partnerschaft mbB
(86) International application number: PCT/CN2022/075145
(87) International publication number: WO 2022/166917

(57) **Abstract**

A TYK2 inhibitor compound containing a bicyclic ring and the use thereof in the preparation of a drug for treating or preventing TYK2-related diseases.

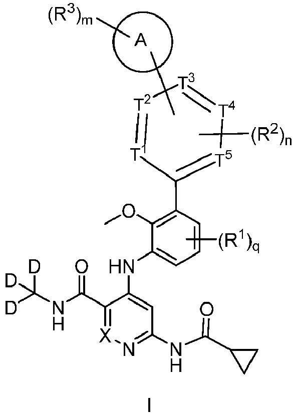

## Description

### CROSS-REFERENCE TO RELATED APPLICATIONS

The present disclosure claims the benefit and priority to the Chinese Patent Application No. 202110165856.4 filed with China National Intellectual Property Administration on February 06, 2021, the Chinese Patent Application No. 202110935764.X filed with China National Intellectual Property Administration on August 16, 2021, the Chinese Patent Application No. 202111063960.9 filed with China National Intellectual Property Administration on September 10, 2021, and the Chinese Patent Application No. 202210056272.8 filed with China National Intellectual Property Administration on January 18, 2022, the content of each of which is incorporated herein by reference in its entirety.

### TECHNICAL FIELD

The present disclosure relates to the field of pharmaceutical chemistry. Provided is a TYK2 inhibitor compound containing a bicyclic ring, a preparation method therefor, and use thereof in preparing a medicament for use in treating or preventing TYK2-related diseases.

### BACKGROUND

Tyrosine kinase 2 (TYK2) is a member of the Janus kinase (JAK) family of non-receptor tyrosine kinases and has been shown to be critically important in the regulation of signaling transduction cascades downstream of the IL-12, IL-23, and type I interferon receptors in both mice and humans. TYK2 mediates receptor-induced phosphorylation of members of the STAT family of transcription factors, which is an essential signal leading to the dimerization of STAT proteins and the transcription of STAT-dependent pro-inflammatory genes. TYK2-deficient mice serve as experimental models that are susceptible to colitis, psoriasis, and multiple sclerosis, thereby demonstrating the importance of TYK2-mediated signaling in autoimmunity and related disorders. In humans, individuals expressing an inactive variant of TYK2 are protected against the effects of multiple sclerosis and potentially other autoimmune disorders.

Given the disorders that can benefit from therapies involving modulation of cytokines and/or interferons, TYK2 inhibitor compounds capable of modulating cytokines and/or interferons such as IL-12, IL-23, and/or IFNα, as well as methods of using these compounds, may offer substantial therapeutic benefits to numerous patients in need thereof.

### BRIEF SUMMARY

In one aspect, the present disclosure provides a compound of formula I or a pharmaceutically acceptable salt thereof: 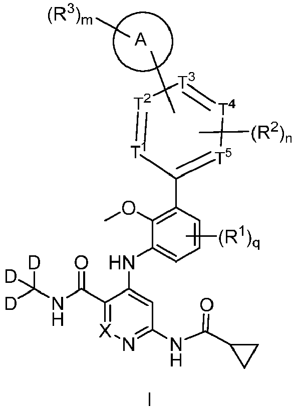 wherein,
X is selected from the group consisting of N and CH;
each R¹ is independently selected from halogen;
q is selected from the group consisting of 0, 1, and 2;
each R² is independently selected from the group consisting of halogen, hydroxy, amino, cyano, and nitro;
n is selected from the group consisting of 0, 1, and 2;
T¹, T², T³, T⁴, and T⁵ are each independently selected from the group consisting of CH and N, at least one of which is selected from CH;
ring A is selected from C₃₋₁₀ cycloalkyl, 3- to 10-membered heterocyclyl, C₆₋₁₀ aryl, and 5- to 10-membered heteroaryl;
each R³ is independently selected from the group consisting of =O, halogen, hydroxy, amino, cyano, nitro, C₁₋₈ alkyl, and C₁₋₈ alkoxy, wherein the C₁₋₈ alkyl or C₁₋₈ alkoxy is optionally substituted with one or more R^{a};
m is selected from the group consisting of 0, 1, 2, 3, and 4;
each R^{a} is independently selected from the group consisting of halogen, hydroxy, amino, and cyano.

In one aspect, the present disclosure provides a compound of formula I or a pharmaceutically acceptable salt thereof: 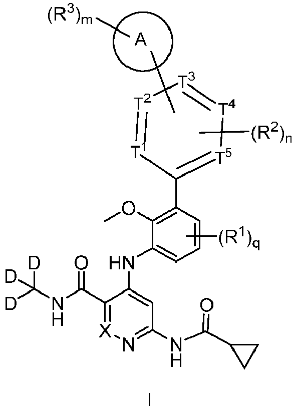 wherein,
X is selected from the group consisting of N and CH;
each R¹ is independently selected from halogen;
q is selected from the group consisting of 0, 1, and 2;
each R² is independently selected from the group consisting of halogen, hydroxy, amino, cyano, and nitro;
n is selected from the group consisting of 0, 1, and 2;
T¹, T², T³, T⁴, and T⁵ are each independently selected from the group consisting of CH and N, at least one of which is selected from CH;
ring A is selected from C₃₋₁₀ cycloalkyl, 3- to 10-membered heterocyclyl, C₆₋₁₀ aryl, and 5- to 10-membered heteroaryl;
each R³ is independently selected from the group consisting of =O, halogen, hydroxy, amino, cyano, nitro, C₁₋₈ alkyl, C₃₋₁₀ cycloalkyl, 3- to 10-membered heterocycloalkyl, 5- to 10-membered heteroaryl, C₆₋₁₀ aryl, and C₁₋₈ alkoxy, wherein the C₁₋₈ alkyl, C₃₋₁₀ cycloalkyl, 3- to 10-membered heterocycloalkyl, 5- to 10-membered heteroaryl, C₆₋₁₀ aryl, or C₁₋₈ alkoxy is optionally substituted with one or more R^{a};
m is selected from the group consisting of 0, 1, 2, 3, and 4;
each R^{a} is independently selected from the group consisting of halogen, hydroxy, amino, and cyano.

In another aspect, the present disclosure further provides a pharmaceutical composition comprising the compound or the pharmaceutically acceptable salt thereof described herein above. In some embodiments, the pharmaceutical composition disclosed herein further comprises a pharmaceutically acceptable excipient.

In another aspect, the present disclosure further provides a method for treating or preventing various TYK2-related diseases comprising administering to a mammal, preferably a human, in need of the treatment a therapeutically or prophylactically effective amount of the compound or the pharmaceutically acceptable salt thereof or the pharmaceutical composition thereof described herein above.

In another aspect, the present disclosure further provides use of the compound or the pharmaceutically acceptable salt thereof or the pharmaceutical composition thereof described herein above in preparing a medicament for use in treating or preventing various TYK2-related diseases.

In another aspect, the present disclosure further provides use of the compound or the pharmaceutically acceptable salt thereof or the pharmaceutical composition thereof described herein above in treating or preventing various TYK2-related diseases.

### DETAILED DESCRIPTION OF INVENTION

In one aspect, the present disclosure provides a compound of formula I or a pharmaceutically acceptable salt thereof: 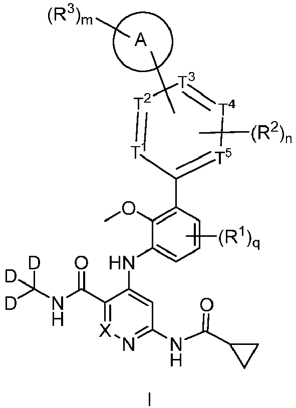 wherein,
X is selected from the group consisting of N and CH;
each R¹ is independently selected from halogen;
q is selected from the group consisting of 0, 1, and 2;
each R² is independently selected from the group consisting of halogen, hydroxy, amino, cyano, and nitro;
n is selected from the group consisting of 0, 1, and 2;
T¹, T², T³, T⁴, and T⁵ are each independently selected from the group consisting of CH and N, at least one of which is selected from CH;
ring A is selected from C₃₋₁₀ cycloalkyl, 3- to 10-membered heterocyclyl, C₆₋₁₀ aryl, and 5- to 10-membered heteroaryl;
each R³ is independently selected from the group consisting of =O, halogen, hydroxy, amino, cyano, nitro, C₁₋₈ alkyl, and C₁₋₈ alkoxy, wherein the C₁₋₈ alkyl or C₁₋₈ alkoxy is optionally substituted with one or more R^{a};
m is selected from the group consisting of 0, 1, 2, 3, and 4;
each R^{a} is independently selected from the group consisting of halogen, hydroxy, amino, and cyano.

In one aspect, the present disclosure provides a compound of formula I or a pharmaceutically acceptable salt thereof: 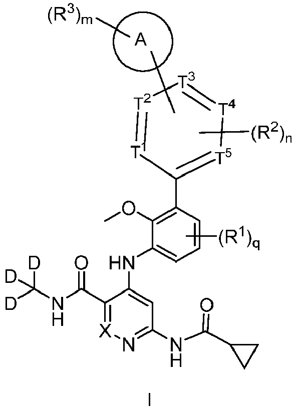 wherein,
X is selected from the group consisting of N and CH;
each R¹ is independently selected from halogen;
q is selected from the group consisting of 0, 1, and 2;
each R² is independently selected from the group consisting of halogen, hydroxy, amino, cyano, and nitro;
n is selected from the group consisting of 0, 1, and 2;
T¹, T², T³, T⁴, and T⁵ are each independently selected from the group consisting of CH and N, at least one of which is selected from CH;
ring A is selected from C₃₋₁₀ cycloalkyl, 3- to 10-membered heterocyclyl, C₆₋₁₀ aryl, and 5- to 10-membered heteroaryl;
each R³ is independently selected from the group consisting of =O, halogen, hydroxy, amino, cyano, nitro, C₁₋₈ alkyl, C₃₋₁₀ cycloalkyl, 3- to 10-membered heterocycloalkyl, 5- to 10-membered heteroaryl, C₆₋₁₀ aryl, and C₁₋₈ alkoxy, wherein the C₁₋₈ alkyl, C₃₋₁₀ cycloalkyl, 3- to 10-membered heterocycloalkyl, 5- to 10-membered heteroaryl, C₆₋₁₀ aryl, or C₁₋₈ alkoxy is optionally substituted with one or more R^{a};
m is selected from the group consisting of 0, 1, 2, 3, and 4;
each R^{a} is independently selected from the group consisting of halogen, hydroxy, amino, and cyano.

In some embodiments, ring A is selected from the group consisting of C₅₋₁₀ cycloalkyl, C₆₋₁₀ aryl, 3- to 10-membered heterocycloalkenyl, and 5- to 10-membered heteroaryl, provided that structural unit 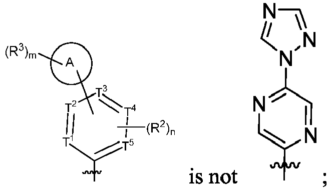 alternatively, 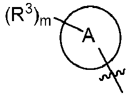 is selected from the group consisting of pyrrolidinyl, 3-hydroxypyrrolidine-1-yl-, 3-oxopyrrolidine-1-yl-, 3-methyl-2-oxopyrrolidine-1-yl-, 4,4-dimethyl-2-oxopyrrolidine-1-yl-, 3,3-dimethyl-2-oxopyrrolidine-1-yl-, oxetanyl, azetidinyl, 3-oxoazetidin-1-yl-, and 3-hydroxyazetidin-1-yl-.

In some embodiments, ring A is selected from the group consisting of C₅₋₆ cycloalkyl, C₆₋₁₀ aryl, 3- to 8-membered heterocycloalkenyl, and 5- to 8-membered heteroaryl, provided that structural unit 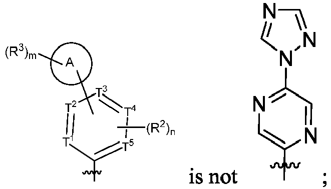 alternatively, structural unit 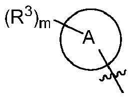 is selected from the group consisting of

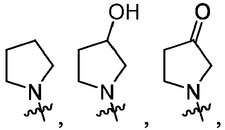

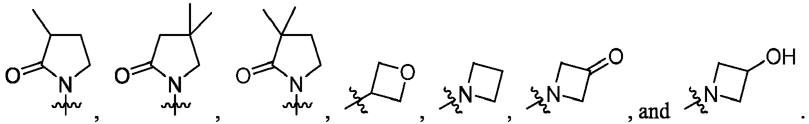

In some embodiments, ring A is selected from the group consisting of C₅₋₆ cycloalkyl, C₆₋₁₀ aryl, 5- to 6-membered heterocycloalkenyl, and 5- to 6-membered heteroaryl, provided that structural unit 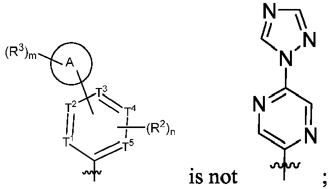 alternatively, structural unit is selected from the group consisting of

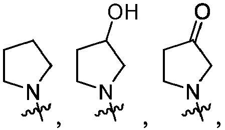

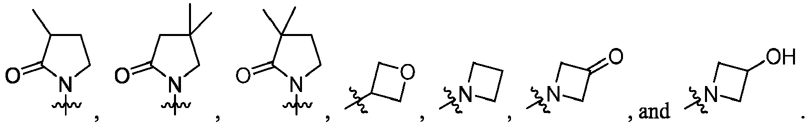

In some embodiments, ring A is selected from the group consisting of 5- to 6-membered heterocycloalkenyl and 5-to 6-membered heteroaryl, provided that structural unit 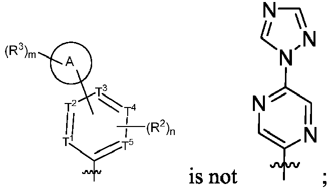 alternatively, structural unit 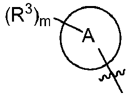 is selected from the group consisting of

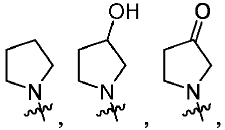

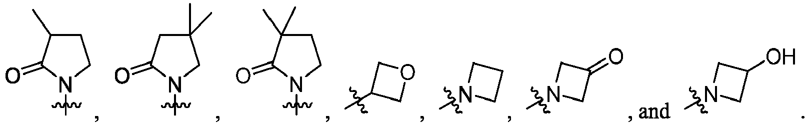

In some embodiments, structural unit

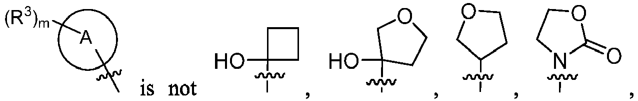

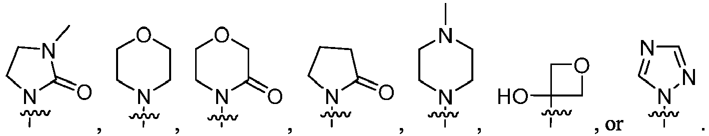

In some embodiments, X is N. In some embodiments, X is CH.

In some embodiments, each R¹ is independently selected from the group consisting of fluorine and chlorine. In some embodiments, each R¹ is independently fluorine.

In some embodiments, q is selected from the group consisting of 0 and 1. In some embodiments, q is 0.

In some embodiments, q is 1 and R¹ is fluorine.

In some embodiments, each R² is independently selected from the group consisting of fluorine, chlorine, and bromine. In some embodiments, each R² is independently selected from the group consisting of fluorine and chlorine. In some embodiments, each R² is independently fluorine.

In some embodiments, n is selected from the group consisting of 0 and 1. In some embodiments, n is 0.

In some embodiments, T¹, T², T³, T⁴, and T⁵ are each independently selected from the group consisting of CH and N, three of which are selected from N and two of which are selected from CH.

In some embodiments, T¹, T², T³, T⁴, and T⁵ are each independently selected from the group consisting of CH and N, two of which are selected from N and three of which are selected from CH.

In some embodiments, T¹ and T⁴ are not both selected from N, and T² and T⁵ are not both selected from N.

In some embodiments, T¹, T², T³, T⁴, and T⁵ are each independently selected from the group consisting of CH and N, one of which is selected from N and four of which are selected from CH.

In some embodiments, T¹, T³, and T⁵ are selected from N, and T² and T⁴ are selected from CH.

In some embodiments, T¹ and T³ are selected from N, and T², T⁴, and T⁵ are selected from CH.

In some embodiments, T¹ and T⁵ are selected from N, and T², T³, and T⁴ are selected from CH.

In some embodiments, T² and T⁴ are selected from N, and T¹, T³, and T⁵ are selected from CH.

In some embodiments, T¹ and T² are selected from N, and T³, T⁴, and T⁵ are selected from CH.

In some embodiments, T² and T³ are selected from N, and T¹, T⁴, and T⁵ are selected from CH.

In some embodiments, T¹ and T⁴ are selected from N, and T², T³, and T⁵ are selected from CH.

In some embodiments, T¹ is selected from N, and T², T³, T⁴, and T⁵ are selected from CH.

In some embodiments, T² is selected from N, and T¹, T³, T⁴, and T⁵ are selected from CH.

In some embodiments, T³ is selected from N, and T¹, T², T⁴, and T⁵ are selected from CH.

In some embodiments, ring A is selected from the group consisting of C₃₋₈ cycloalkyl, 3- to 8-membered heterocyclyl, C₆₋₁₀ aryl, and 5- to 8-membered heteroaryl.

In some embodiments, ring A is selected from the group consisting of C₃₋₆ cycloalkyl, 4- to 6-membered heterocyclyl, C₆₋₁₀ aryl, and 5- to 6-membered heteroaryl.

In some embodiments, ring A is selected from the group consisting of C₃₋₆ cycloalkyl, 4- to 6-membered heterocyclyl containing 1, 2, or 3 atoms selected from the group consisting of N, O, and S, C₆₋₁₀ aryl, and 5- to 6-membered heteroaryl containing 1, 2, or 3 atoms selected from the group consisting of N, O, and S.

In some embodiments, ring A is selected from the group consisting of C₃₋₈ cycloalkyl, 3- to 8-membered heterocycloalkyl, 3- to 8-membered heterocycloalkenyl, C₆₋₁₀ aryl, and 5- to 8-membered heteroaryl.

In some embodiments, ring A is selected from the group consisting of C₃₋₈ cycloalkyl, 4- to 6-membered heterocycloalkyl, 4- to 6-membered heterocycloalkenyl, C₆₋₁₀ aryl, and 5- to 8-membered heteroaryl.

In some embodiments, ring A is selected from the group consisting of C₃₋₆ cycloalkyl, 4- to 6-membered heterocycloalkyl containing 1, 2, or 3 atoms selected from the group consisting of N, O, and S, 4- to 6-membered heterocycloalkenyl containing 1, 2, or 3 atoms selected from the group consisting of N, O, and S, C₆₋₁₀ aryl, and 5-to 6-membered heteroaryl containing 1, 2, or 3 atoms selected from the group consisting of N, O, and S.

In some embodiments, ring A is selected from the group consisting of 4- to 6-membered heterocycloalkyl containing 1, 2, or 3 atoms selected from the group consisting of N, O, and S, 4- to 6-membered heterocycloalkenyl containing 1, 2, or 3 atoms selected from the group consisting of N, O, and S, and 5- to 6-membered heteroaryl containing 1, 2, or 3 atoms selected from the group consisting of N, O, and S.

In some embodiments, ring A is selected from the group consisting of 3- to 8-membered heterocycloalkenyl and 5-to 8-membered heteroaryl.

In some embodiments, ring A is selected from the group consisting of 4- to 6-membered heterocycloalkenyl and 5-to 8-membered heteroaryl.

In some embodiments, ring A is selected from the group consisting of 4- to 6-membered heterocycloalkenyl containing 1, 2, or 3 atoms selected from the group consisting of N, O, and S and 5- to 6-membered heteroaryl containing 1, 2, or 3 atoms selected from the group consisting of N, O, and S.

In some embodiments, ring A is selected from 5- to 6-membered heteroaryl containing 1, 2, or 3 atoms selected from the group consisting of N, O, and S.

In some embodiments, ring A is selected from 5-membered heteroaryl containing 1, 2, or 3 atoms selected from the group consisting of N, O, and S.

In some embodiments, ring A is selected from 5-membered heteroaryl containing 1, 2, or 3 atoms selected from the group consisting of N and O.

In some embodiments, ring A is selected from 6-membered heteroaryl containing 1, 2, or 3 atoms selected from the group consisting of N and O.

In some embodiments, ring A is selected from 5-membered heteroaryl containing 1, 2, or 3 atoms selected from N.

In some embodiments, ring A is selected from 6-membered heteroaryl containing 1, 2, or 3 atoms selected from N.

In some embodiments, ring A is selected from 5-membered heteroaryl containing 1 or 2 atoms selected from N.

In some embodiments, ring A is selected from 6-membered heteroaryl containing 1 or 2 atoms selected from N.

In some embodiments, ring A is selected from 5-membered heteroaryl containing 2 nitrogen atoms.

In some embodiments, ring A is selected from 6-membered heteroaryl containing 2 nitrogen atoms.

In some embodiments, ring A is selected from the group consisting of oxetanyl, azetidinyl, morpholinyl, dioxanyl, dihydropyridinyl, pyridinyl, pyrimidinyl, pyridazinyl, tetrahydropyrrolyl, triazolyl, tetrazolyl, pyrazolyl, furanyl, thienyl, dihydropyrimidinyl, oxazolyl, isoxazolyl, and imidazolyl.

In some embodiments, ring A is selected from the group consisting of dihydropyridinyl, pyridinyl, pyrimidinyl, pyridazinyl, tetrazolyl, pyrazolyl, furanyl, thienyl, dihydropyrimidinyl, oxazolyl, isoxazolyl, and imidazolyl.

In some embodiments, ring A is selected from the group consisting of tetrazolyl, pyrazolyl, furanyl, thienyl, oxazolyl, isoxazolyl, and imidazolyl.

In some embodiments, ring A is selected from the group consisting of tetrazolyl, pyrazolyl, and imidazolyl.

In some embodiments, ring A is selected from pyrazolyl.

In some embodiments, ring A is selected from the group consisting of C₅₋₁₀ cycloalkyl, C₆₋₁₀ aryl, 4-membered heterocycloalkyl, 5- to 6-membered heterocycloalkenyl, and 5- to 10-membered heteroaryl, and T¹ and T⁴ are not both selected from N, and T² and T⁵ are not both selected from N.

In some embodiments, ring A is selected from the group consisting of C₅₋₆ cycloalkyl, C₆₋₁₀ aryl, 4-membered heterocycloalkyl, 5- to 6-membered heterocycloalkenyl, and 5- to 6-membered heteroaryl, and T¹ and T⁴ are not both selected from N, and T² and T⁵ are not both selected from N.

In some embodiments, ring A is selected from the group consisting of 5- to 6-membered heterocycloalkenyl and 5-to 6-membered heteroaryl, and T¹ and T⁴ are not both selected from N, and T² and T⁵ are not both selected from N.

In some embodiments, ring A is selected from the group consisting of dihydropyridinyl, pyridinyl, pyrimidinyl, pyridazinyl, triazolyl, tetrazolyl, pyrazolyl, furanyl, thienyl, dihydropyrimidinyl, oxazolyl, isoxazolyl, and imidazolyl, and T¹ and T⁴ are not both selected from N, and T² and T⁵ are not both selected from N.

In some embodiments, ring A is selected from the group consisting of triazolyl, tetrazolyl, pyrazolyl, furanyl, thienyl, oxazolyl, isoxazolyl, and imidazolyl, and T¹ and T⁴ are not both selected from N, and T² and T⁵ are not both selected from N.

In some embodiments, ring A is selected from the group consisting of triazolyl, tetrazolyl, pyrazolyl, and imidazolyl, and T¹ and T⁴ are not both selected from N, and T² and T⁵ are not both selected from N.

In some embodiments, T¹ and T⁵ are selected from N, and T², T³, and T⁴ are selected from CH; ring A is selected from the group consisting of 4- to 6-membered heterocycloalkenyl and 5- to 6-membered heteroaryl.

In some embodiments, T¹ and T⁵ are selected from N, and T², T³, and T⁴ are selected from CH; ring A is selected from the group consisting of 5- to 6-membered heterocycloalkenyl and 5- to 6-membered heteroaryl.

In some embodiments, T¹ and T⁵ are selected from N, and T², T³, and T⁴ are selected from CH; ring A is selected from 5- to 6-membered heteroaryl.

In some embodiments, T¹ and T⁵ are selected from N, and T², T³, and T⁴ are selected from CH; ring A is selected from 6-membered heteroaryl.

In some embodiments, T¹ and T⁵ are selected from N, and T², T³, and T⁴ are selected from CH; ring A is selected from 6-membered heteroaryl containing 1 or 2 nitrogen atoms.

In some embodiments, T¹ and T⁵ are selected from N, and T², T³, and T⁴ are selected from CH; ring A is selected from 6-membered heteroaryl containing 2 nitrogen atoms.

In some embodiments, T¹ and T⁵ are selected from N, and T², T³, and T⁴ are selected from CH; ring A is selected from 5-membered heteroaryl.

In some embodiments, T¹ and T⁵ are selected from N, and T², T³, and T⁴ are selected from CH; ring A is selected from 5-membered heteroaryl containing 1 or 2 nitrogen atoms.

In some embodiments, T¹ and T⁵ are selected from N, and T², T³, and T⁴ are selected from CH; ring A is selected from 5-membered heteroaryl containing 2 nitrogen atoms.

In some embodiments, X is selected from N; ring A is selected from the group consisting of 5- to 6-membered heterocycloalkenyl and 5- to 6-membered heteroaryl.

In some embodiments, X is selected from N; ring A is selected from the group consisting of 4- to 6-membered heterocycloalkenyl and 5- to 6-membered heteroaryl.

In some embodiments, T¹ and T⁵ are selected from N, and T², T³, and T⁴ are selected from CH; X is selected from N.

In some embodiments, T¹ and T⁵ are selected from N, and T², T³, and T⁴ are selected from CH; X is selected from N; ring A is selected from the group consisting of 5- to 6-membered heterocycloalkenyl and 5- to 6-membered heteroaryl.

In some embodiments, T¹ and T⁵ are selected from N, and T², T³, and T⁴ are selected from CH; X is selected from N; ring A is selected from the group consisting of 4- to 6-membered heterocycloalkenyl and 5- to 6-membered heteroaryl.

In some embodiments, T¹ and T⁵ are selected from N, and T², T³, and T⁴ are selected from CH; X is selected from N; ring A is selected from 5- to 6-membered heteroaryl.

In some embodiments, T¹ and T⁵ are selected from N, and T², T³, and T⁴ are selected from CH; X is selected from N; ring A is selected from 6-membered heteroaryl.

In some embodiments, T¹ and T⁵ are selected from N, and T², T³, and T⁴ are selected from CH; X is selected from N; ring A is selected from 6-membered heteroaryl containing 1 or 2 nitrogen atoms.

In some embodiments, T¹ and T⁵ are selected from N, and T², T³, and T⁴ are selected from CH; X is selected from N; ring A is selected from 6-membered heteroaryl containing 2 nitrogen atoms.

In some embodiments, T¹ and T⁵ are selected from N, and T², T³, and T⁴ are selected from CH; X is selected from N; ring A is selected from 5-membered heteroaryl.

In some embodiments, T¹ and T⁵ are selected from N, and T², T³, and T⁴ are selected from CH; X is selected from N; ring A is selected from 5-membered heteroaryl containing 1 or 2 nitrogen atoms.

In some embodiments, T¹ and T⁵ are selected from N, and T², T³, and T⁴ are selected from CH; X is selected from N; ring A is selected from 5-membered heteroaryl containing 2 nitrogen atoms.

In some embodiments, T¹ and T⁵ are selected from N, and T², T³, and T⁴ are selected from CH; X is selected from N; ring A is selected from pyrazolyl.

In some other embodiments, ring A is selected from the group consisting of oxetanyl, azetidinyl, morpholinyl, dihydropyridinyl, pyridinyl, pyrimidinyl, tetrahydropyrrolyl, triazolyl, pyrazolyl, furanyl, dihydropyrimidinyl, isoxazolyl, and imidazolyl.

In some other embodiments, ring A is selected from the group consisting of oxetanyl, morpholinyl, dihydropyridinyl, pyridinyl, pyrimidinyl, tetrahydropyrrolyl, triazolyl, pyrazolyl, furanyl, dihydropyrimidinyl, isoxazolyl, and imidazolyl.

In some embodiments, ring A is selected from the group consisting of

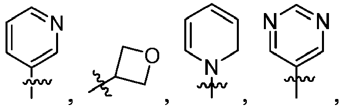

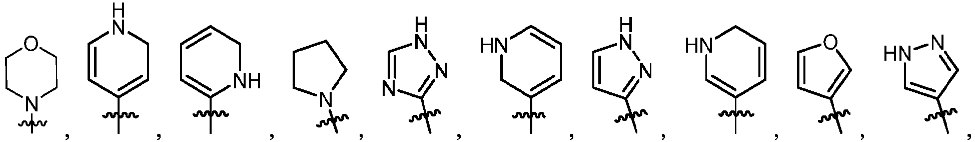

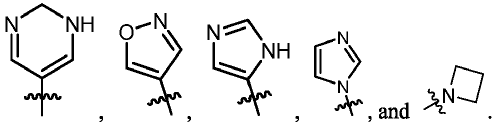

In some embodiments, ring A is selected from the group consisting of 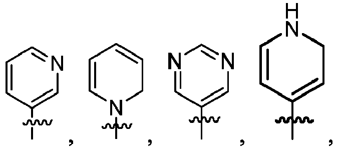 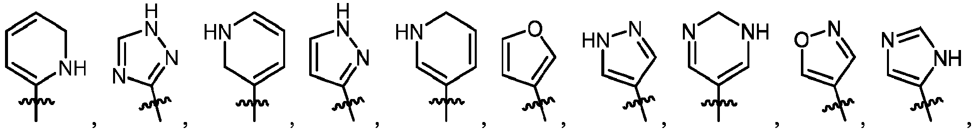 and

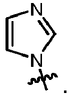

In some embodiments, ring A is selected from the group consisting of

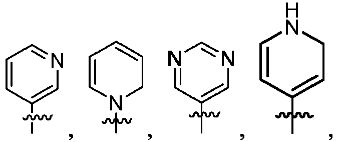

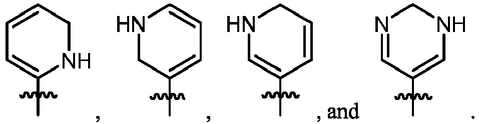

In some embodiments, ring A is selected from the group consisting of

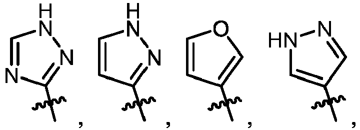

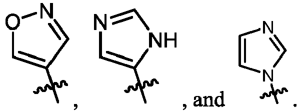

In some embodiments, ring A is selected from the group consisting of 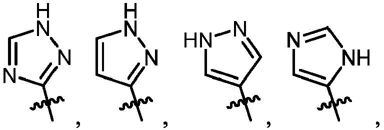 and

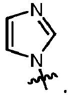

In some embodiments, ring A is selected from

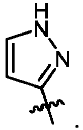

In some other embodiments, ring A is selected from the group consisting of 4- to 6-membered heterocycloalkyl containing 1 or 2 atoms selected from the group consisting of N and O, 4- to 6-membered heterocycloalkenyl containing 1 or 2 atoms selected from N, and 6-membered heteroaryl containing 1 or 2 atoms selected from N.

In some other embodiments, ring A is selected from the group consisting of 4- to 6-membered heterocyclyl containing 1 or 2 atoms selected from the group consisting of N and O and 6-membered heteroaryl containing 1 or 2 atoms selected from N.

In some other embodiments, ring A is selected from the group consisting of oxetanyl, morpholinyl, 1,2-dihydropyridinyl, pyridinyl, and pyrimidinyl.

In some other embodiments, ring A is selected from the group consisting of 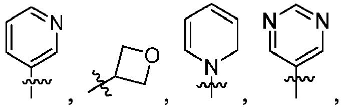 and

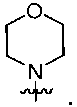

In some embodiments, m is selected from the group consisting of 0, 1, 2, 3, and 4. In some embodiments, m is selected from the group consisting of 0, 1, 2, and 3. In some embodiments, m is selected from the group consisting of 0, 1, and 2.

In some embodiments, each R³ is independently selected from the group consisting of =O, halogen, hydroxy, amino, cyano, nitro, C₁₋₆ alkyl, C₃₋₆ cycloalkyl, 3- to 6-membered heterocycloalkyl, 5- to 6-membered heteroaryl, C₆₋₁₀ aryl, and C₁₋₆ alkoxy, wherein the C₁₋₆ alkyl, C₃₋₆ cycloalkyl, 3- to 6-membered heterocycloalkyl, 5- to 6-membered heteroaryl, C₆₋₁₀ aryl, or C₁₋₆ alkoxy is optionally substituted with one or more R^{a}.

In some embodiments, each R³ is independently selected from the group consisting of =O, halogen, hydroxy, amino, cyano, nitro, C₁₋₄ alkyl, C₃₋₆ cycloalkyl, 3- to 6-membered heterocycloalkyl, and C₁₋₄ alkoxy, wherein the C₁₋₄ alkyl, C₃₋₆ cycloalkyl, 3- to 6-membered heterocycloalkyl, or C₁₋₄ alkoxy is optionally substituted with one or more R^{a}.

In some embodiments, each R³ is independently selected from the group consisting of =O, halogen, hydroxy, amino, cyano, nitro, C₁₋₃ alkyl, C₃₋₄ cycloalkyl, and C₁₋₃ alkoxy, wherein the C₁₋₃ alkyl, C₃₋₄ cycloalkyl, or C₁₋₃ alkoxy is optionally substituted with one or more R⁸.

In some embodiments, each R³ is independently selected from the group consisting of =O, fluorine, chlorine, hydroxy, amino, cyano, cyclopropyl, and C₁₋₃ alkyl optionally substituted with one or more fluorine.

In some embodiments, each R³ is independently selected from the group consisting of =O, fluorine, hydroxy, amino, cyano, methyl, trifluoromethyl, and cyclopropyl.

In some embodiments, each R³ is independently selected from the group consisting of =O, fluorine, chlorine, amino, cyano, cyclopropyl, and C₁₋₃ alkyl optionally substituted with one or more fluorine.

In some embodiments, each R³ is independently selected from the group consisting of =O, fluorine, amino, cyano, methyl, trifluoromethyl, and cyclopropyl.

In some embodiments, each R³ is independently selected from the group consisting of cyano, cyclopropyl, and C₁₋₃ alkyl optionally substituted with one or more fluorine.

In some embodiments, each R³ is independently selected from the group consisting of cyano, methyl, trifluoromethyl, and cyclopropyl.

In some other embodiments, each R³ is independently selected from the group consisting of =O, halogen, hydroxy, amino, cyano, nitro, C₁₋₈ alkyl, and C₁₋₈ alkoxy, wherein the C₁₋₈ alkyl or C₁₋₈ alkoxy is optionally substituted with one or more R^{a}.

In some other embodiments, each R³ is independently selected from the group consisting of =O, halogen, hydroxy, amino, cyano, nitro, C₁₋₆ alkyl, and C₁₋₆ alkoxy, wherein the C₁₋₆ alkyl or C₁₋₆ alkoxy is optionally substituted with one or more R^{a}.

In some other embodiments, each R³ is independently selected from the group consisting of =O, halogen, hydroxy, amino, cyano, nitro, C₁₋₃ alkyl, and C₁₋₃ alkoxy, wherein the C₁₋₃ alkyl or C₁₋₃ alkoxy is optionally substituted with one or more R^{a}.

In some embodiments, each R³ is independently selected from the group consisting of =O, fluorine, chlorine, amino, cyano, and C₁₋₃ alkyl optionally substituted with one or more fluorine.

In some embodiments, each R³ is independently selected from the group consisting of =O, fluorine, amino, cyano, methyl, and trifluoromethyl.

In some other embodiments, each R³ is independently selected from the group consisting of =O, fluorine, chlorine, bromine, and C₁₋₃ alkyl, wherein the C₁₋₃ alkyl is optionally substituted with one or more R^{a}.

In some other embodiments, each R³ is independently selected from the group consisting of =O, fluorine, and methyl, wherein the methyl is optionally substituted with one or more fluorine.

In some embodiments, each R^{a} is independently selected from the group consisting of halogen and cyano. In some embodiments, each R^{a} is independently selected from the group consisting of fluorine, chlorine, and bromine. In some embodiments, each R^{a} is independently fluorine.

In some embodiments, structural unit 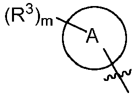 is selected from the group consisting of

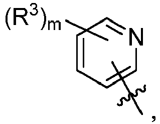

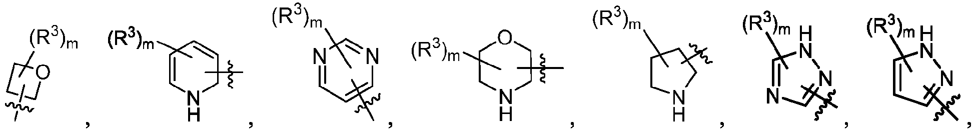

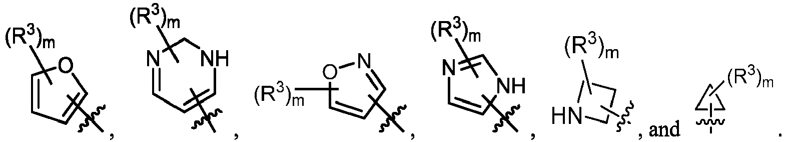

In some embodiments, structural unit 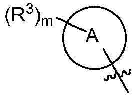 is selected from the group consisting of 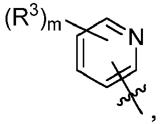 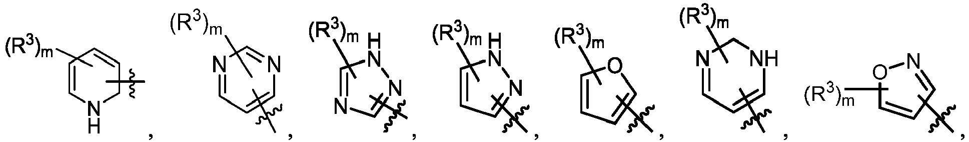 and

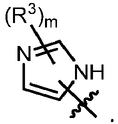

In some embodiments, structural unit 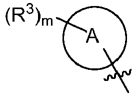 is selected from the group consisting of

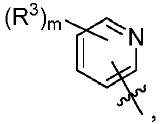

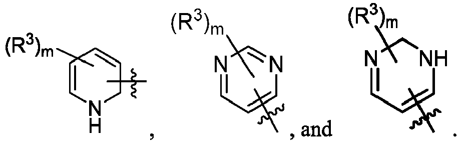

In some embodiments, structural unit 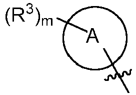 is selected from the group consisting of

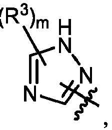

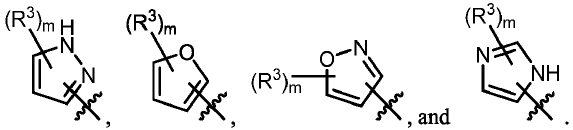

In some embodiments, structural unit 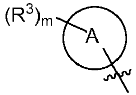 is selected from the group consisting of

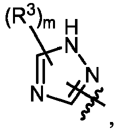

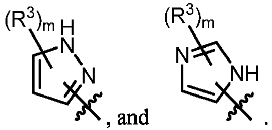

In some embodiments, structural unit 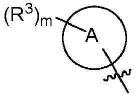 is selected from

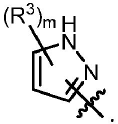

In some other embodiments, structural unit 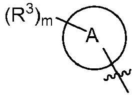 is selected from the group consisting of

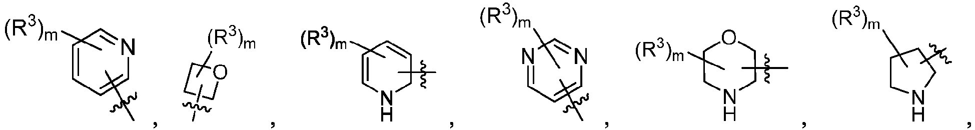

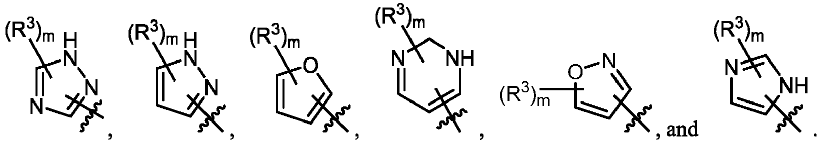

In some other embodiments, structural unit 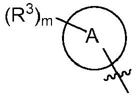 is selected from the group consisting of

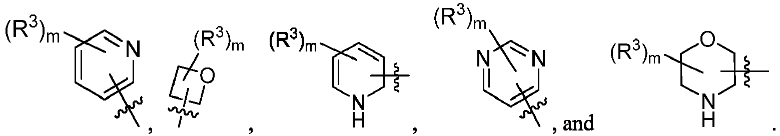

In some embodiments, structural unit 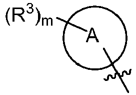 is selected from the group consisting of

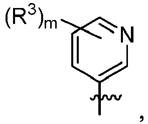

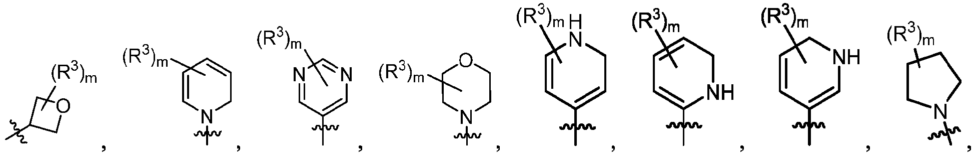

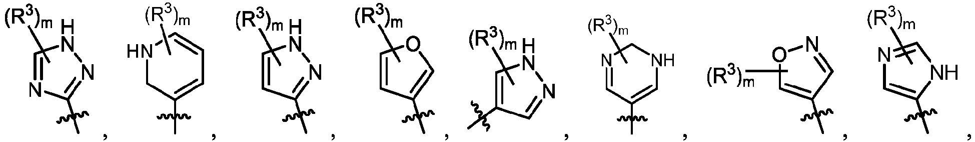

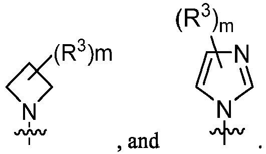

In some embodiments, structural unit 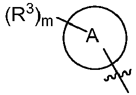 is selected from the group consisting of

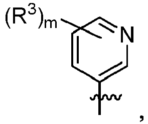

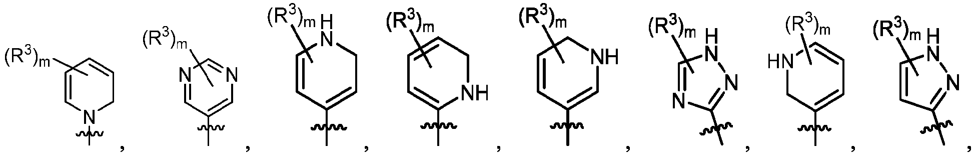

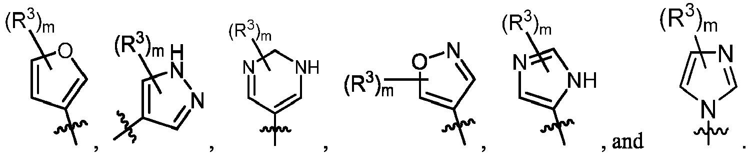

In some embodiments, structural unit 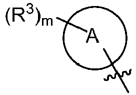 is selected from the group consisting of

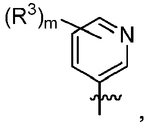

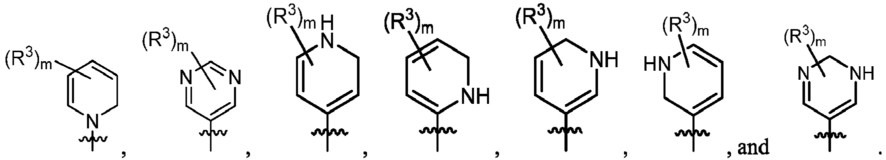

In some embodiments, structural unit 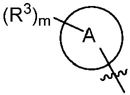 is selected from the group consisting of

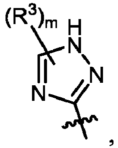

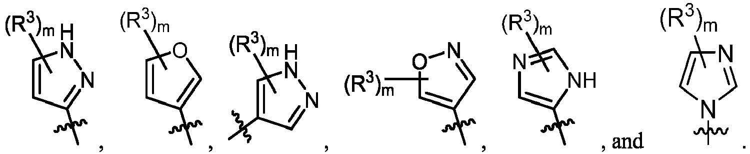

In some embodiments, structural unit 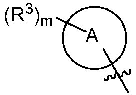 is selected from the group consisting of

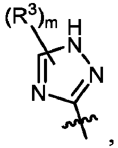

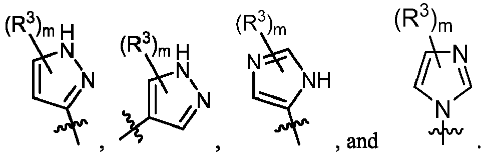

In some embodiments, structural unit 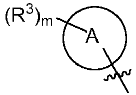 is selected from

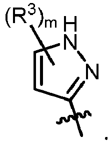

In some other embodiments, structural unit 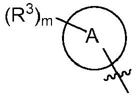 is selected from the group consisting of

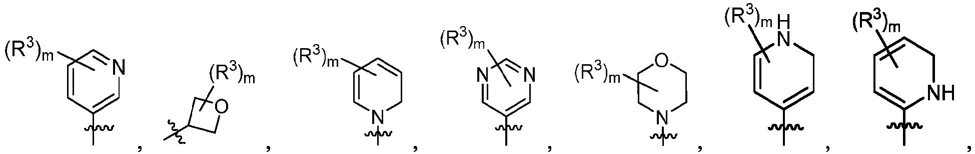

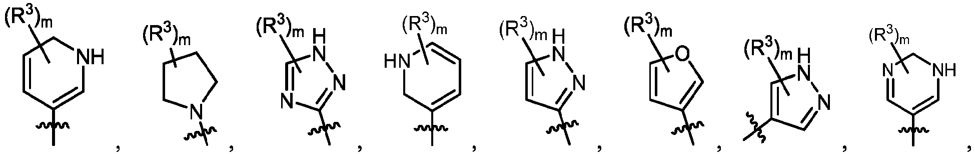

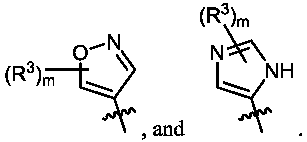

In some other embodiments, structural unit 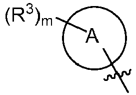 is selected from the group consisting of

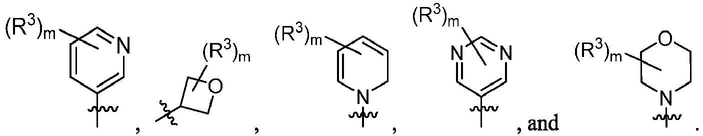

In some embodiments, structural unit 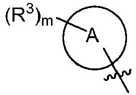 is selected from the group consisting of

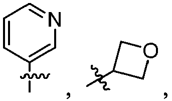

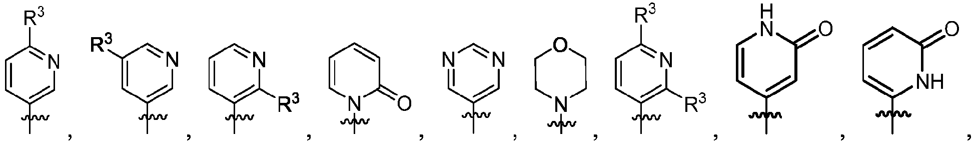

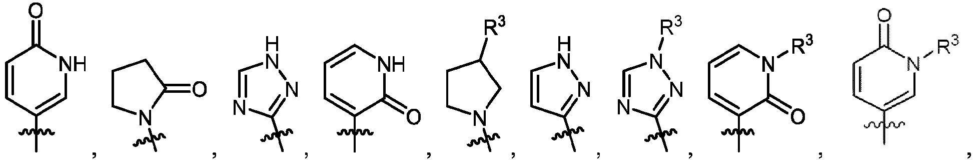

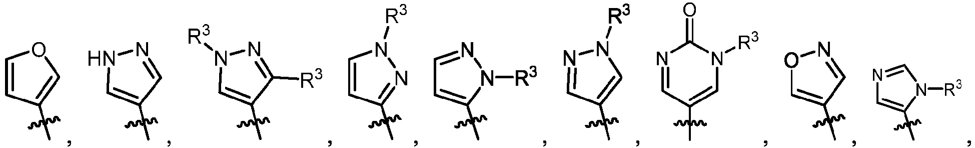

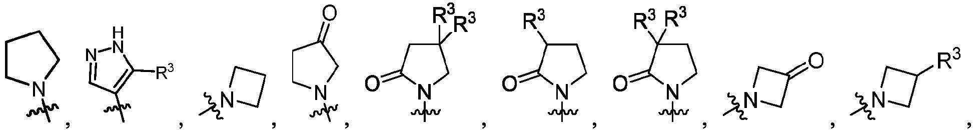

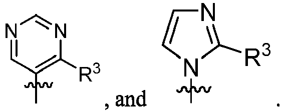

In some embodiments, structural unit 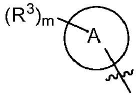 is selected from the group consisting of 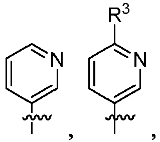 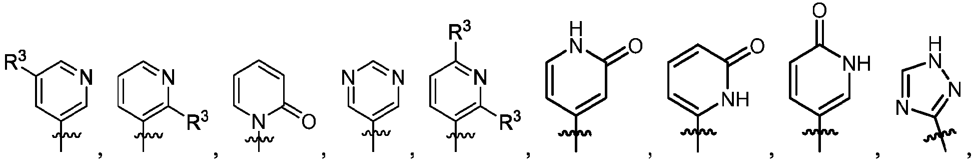 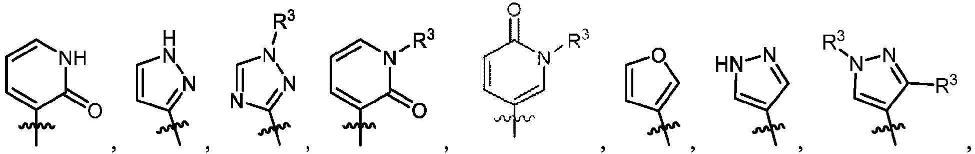 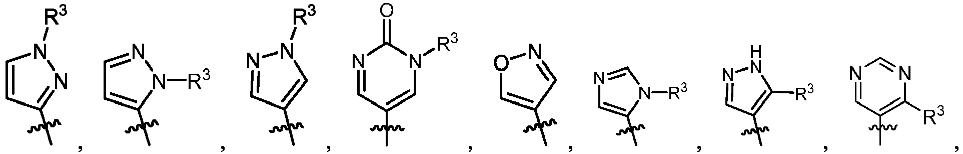 and

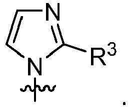

In some embodiments, structural unit 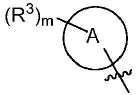 is selected from the group consisting of

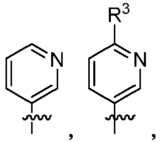

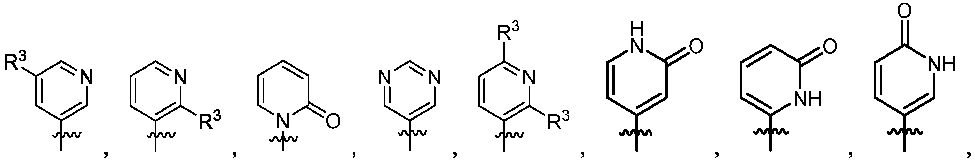

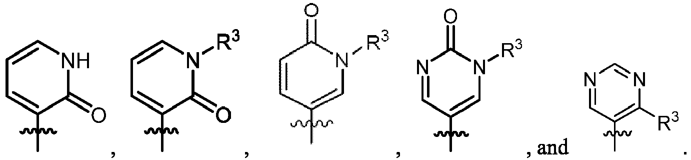

In some embodiments, structural unit 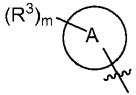 is selected from the group consisting of

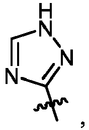

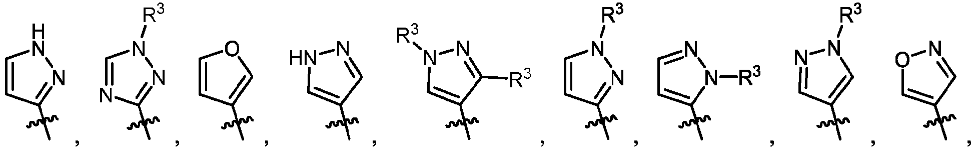

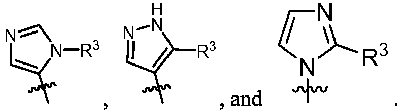

In some embodiments, structural unit 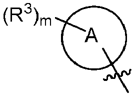 is selected from the group consisting of 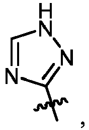 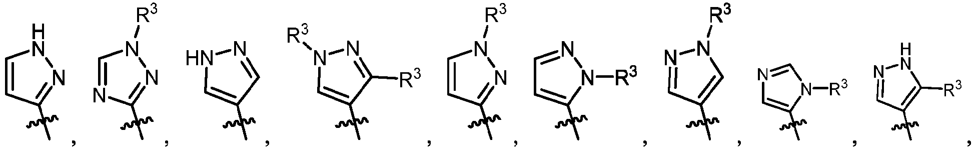 and

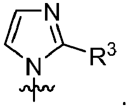

In some embodiments, structural unit 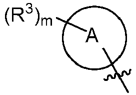 is selected from the group consisting of 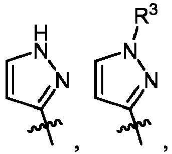 and

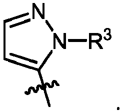

In some other embodiments, structural unit 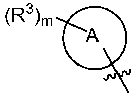 is selected from the group consisting of

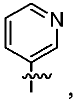

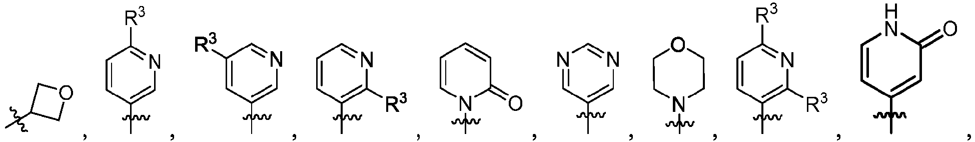

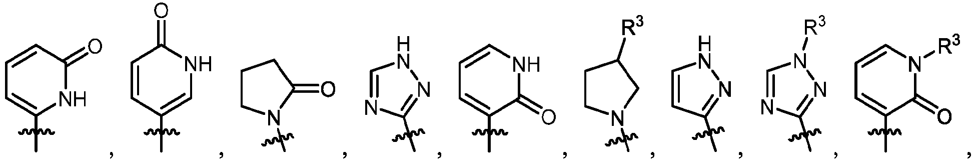

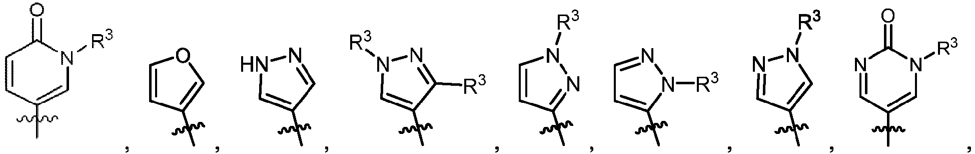

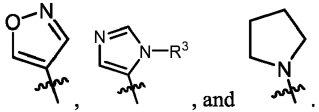

In some other embodiments, structural unit 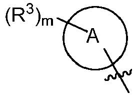 is selected from the group consisting of

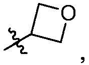

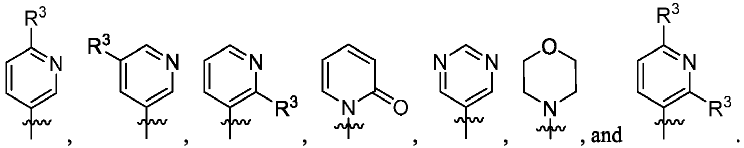

In some embodiments, structural unit 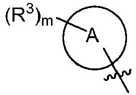 is selected from the group consisting of

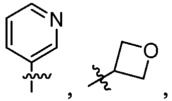

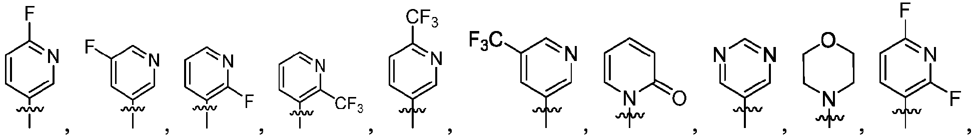

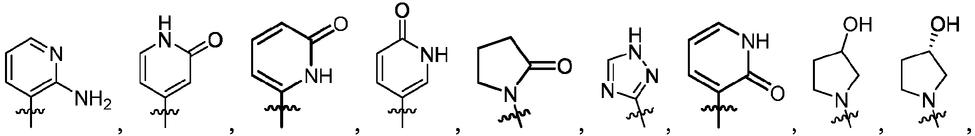

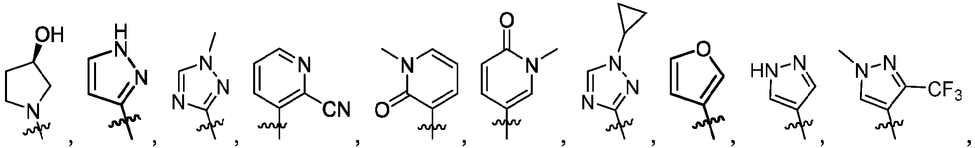

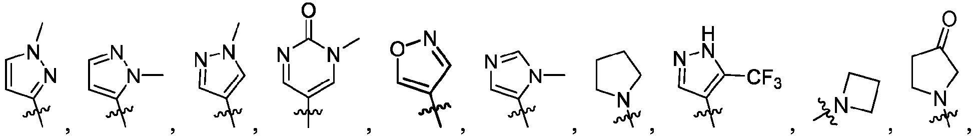

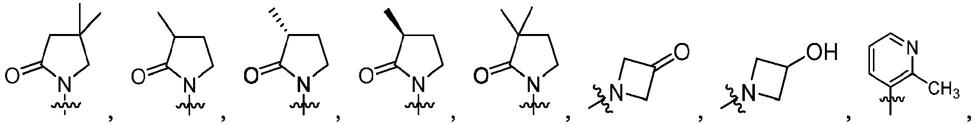

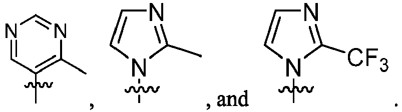

In some embodiments, structural unit 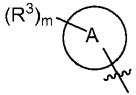 is selected from the group consisting of

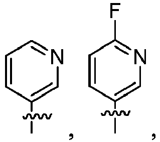

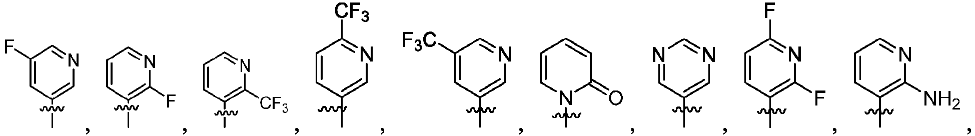

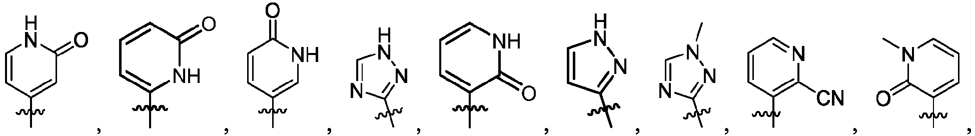

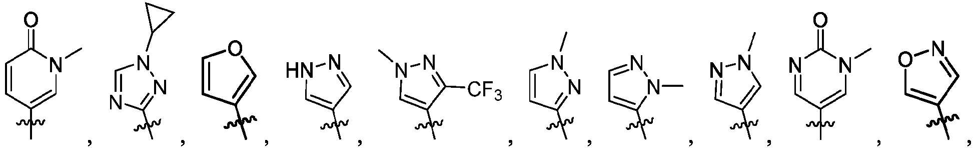

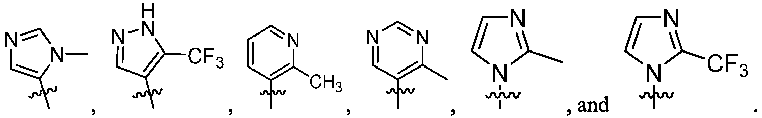

In some embodiments, structural unit 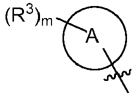 is selected from the group consisting of

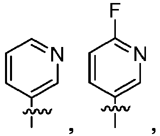

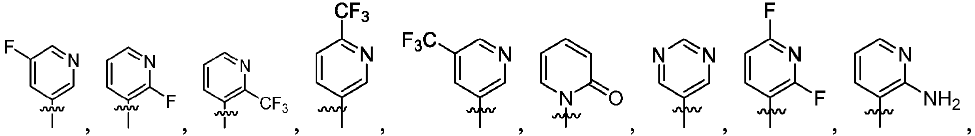

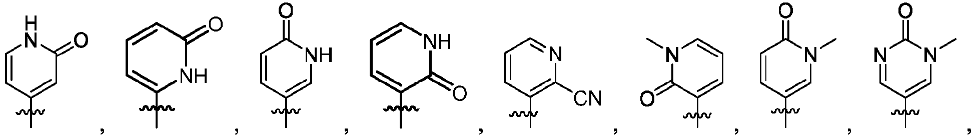

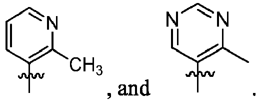

In some embodiments, structural unit 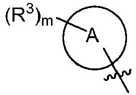 is selected from the group consisting of

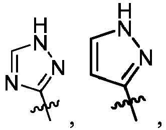

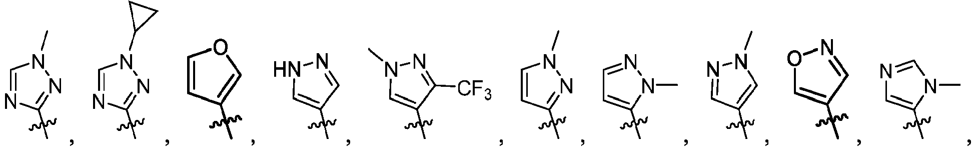

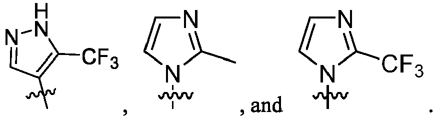

In some embodiments, structural unit 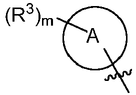 is selected from the group consisting of

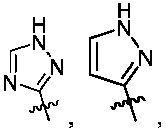

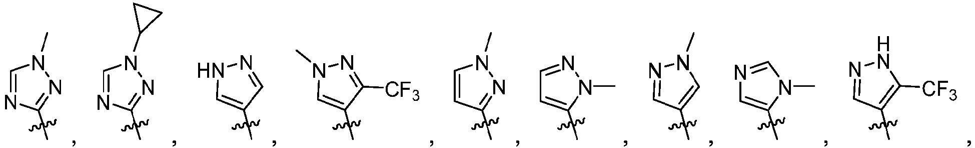

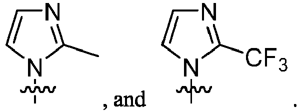

In some embodiments, structural unit 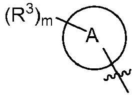 is selected from the group consisting of 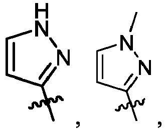 and

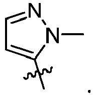

In some other embodiments, structural unit 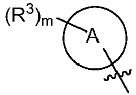 is selected from the group consisting of

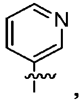

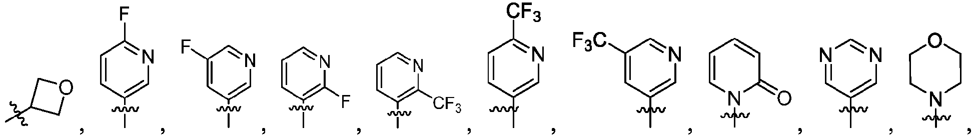

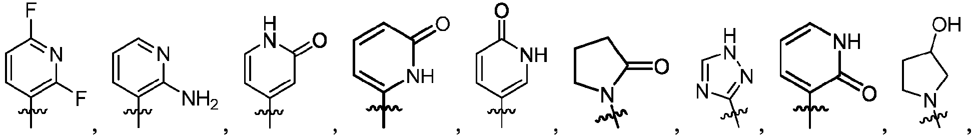

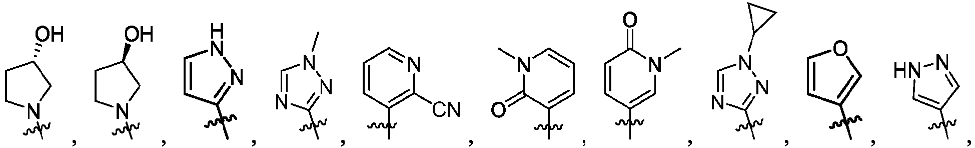

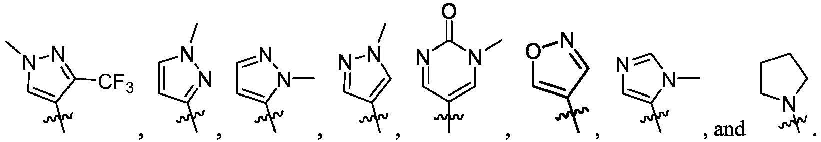

In some other embodiments, structural unit 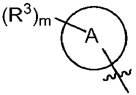 is selected from the group consisting of 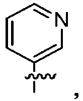 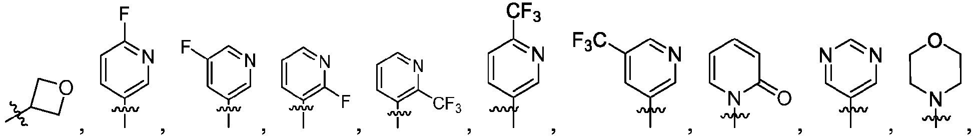 and

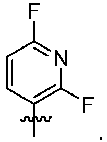

In some embodiments, structural unit 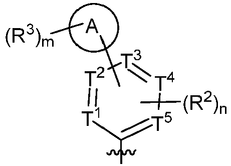 is not 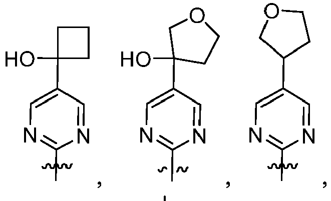 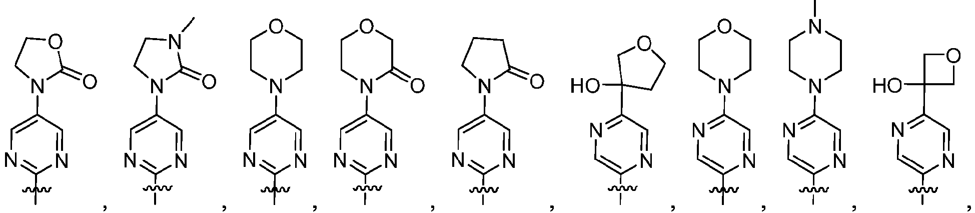 or

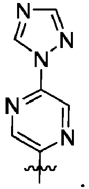

In some embodiments, structural unit 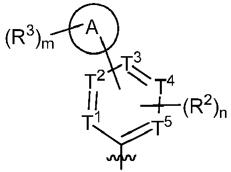 is selected from the group consisting of

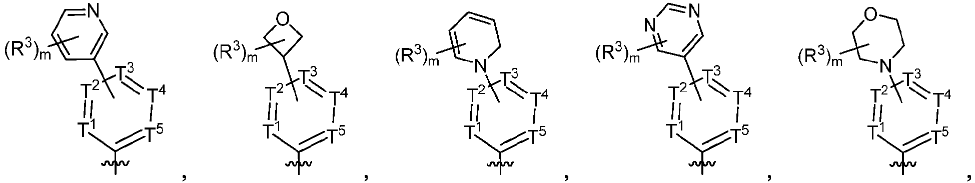

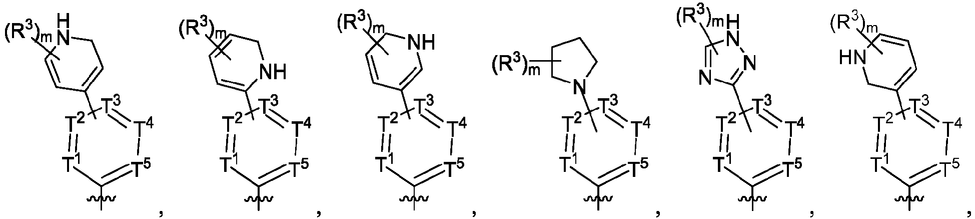

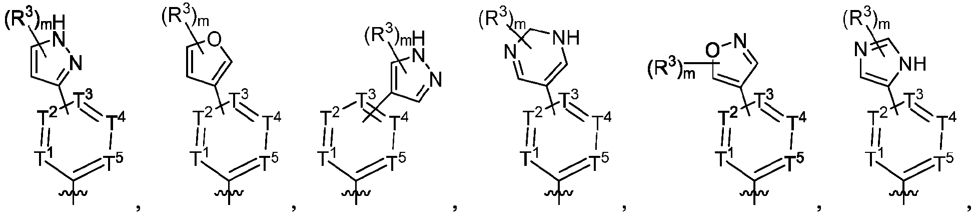

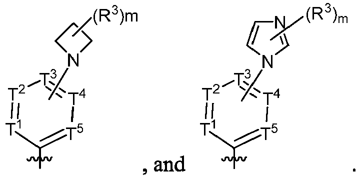

In some embodiments, structural unit 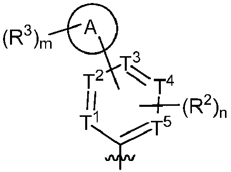 is selected from the group consisting of

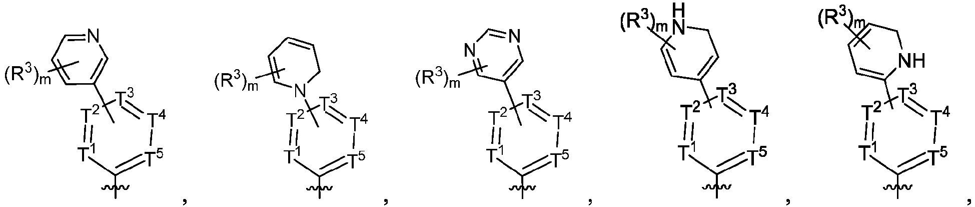

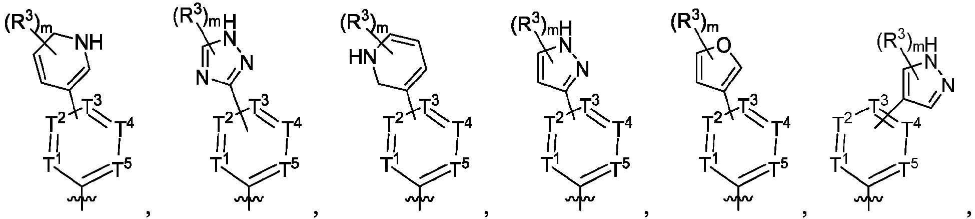

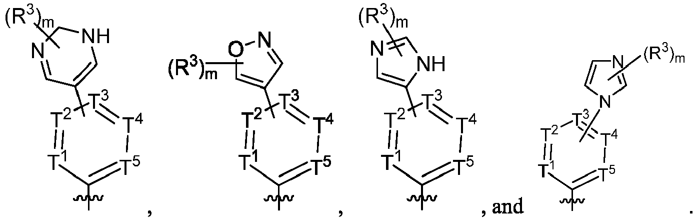

In some embodiments, structural unit 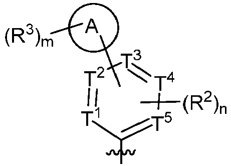 is selected from the group consisting of

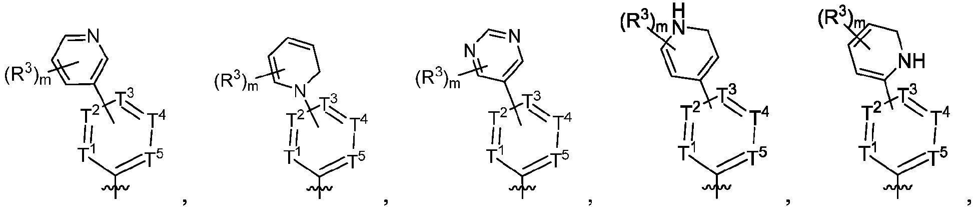

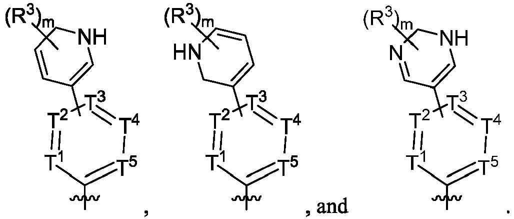

In some embodiments, structural unit 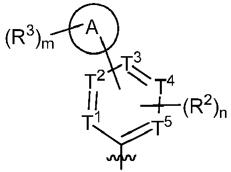 is selected from the group consisting of

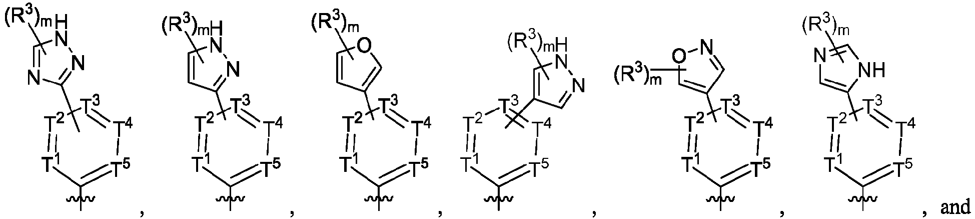

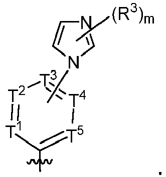

In some embodiments, structural unit 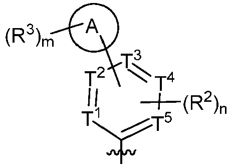 is selected from the group consisting of

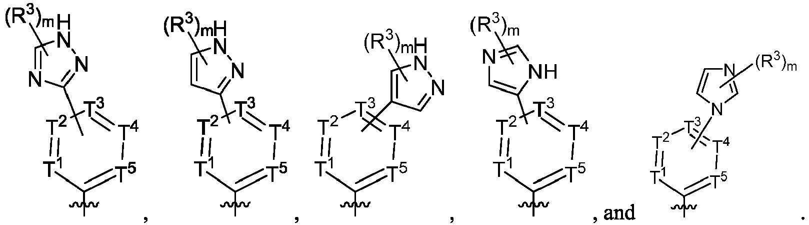

In some embodiments, structural unit 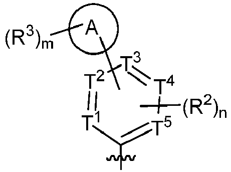 is selected from

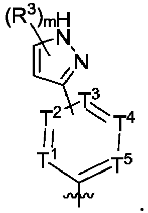

In some other embodiments, structural unit 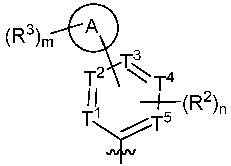 is selected from the group consisting of

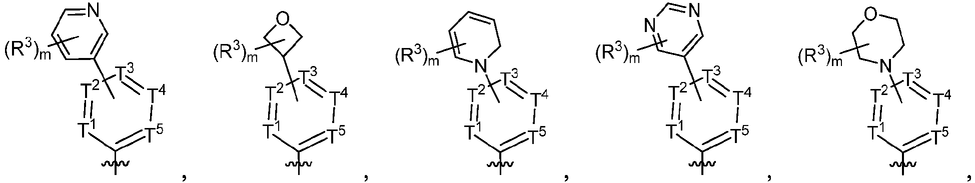

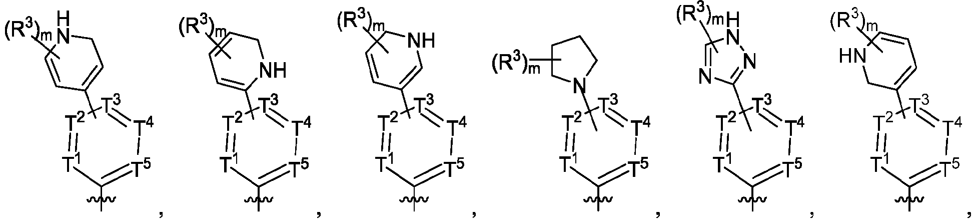

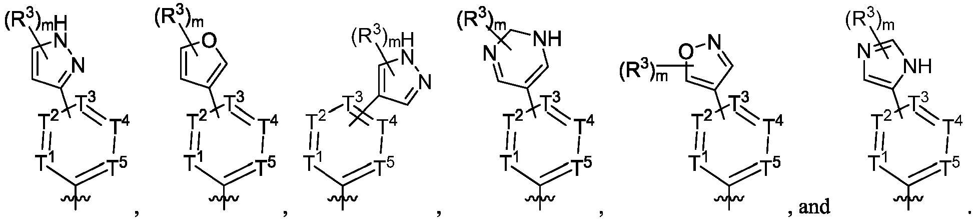

In some other embodiments, structural unit 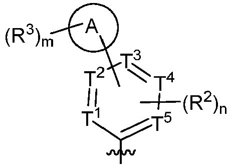 is selected from the group consisting of

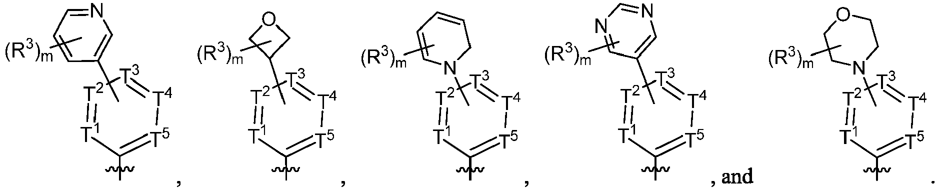

In some embodiments, structural unit 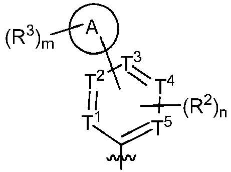 is selected from the group consisting of

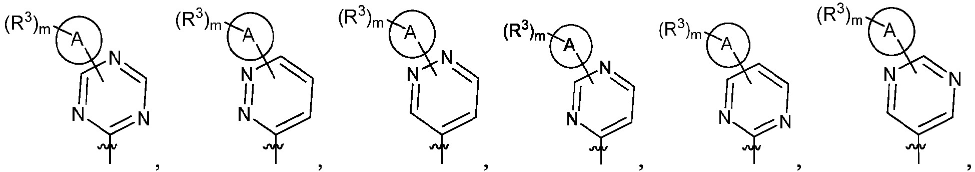

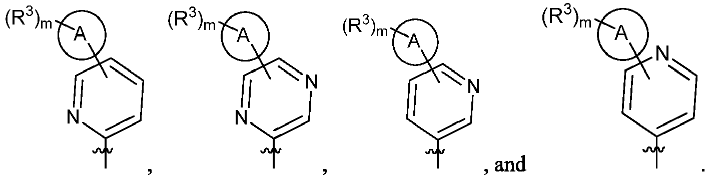

In some embodiments, structural unit 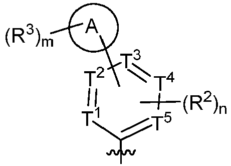 is selected from

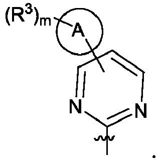

In some other embodiments, structural unit 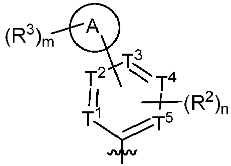 is selected from the group consisting of

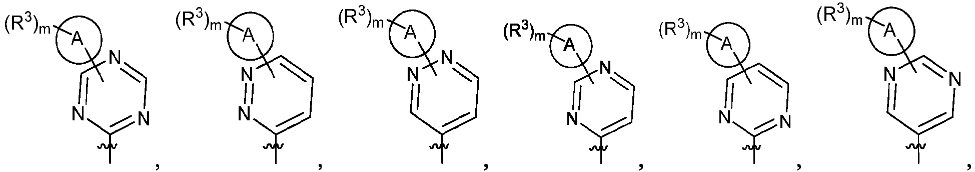

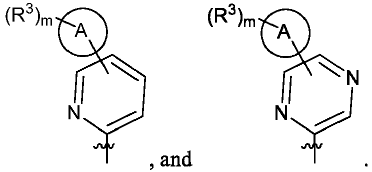

In some embodiments, structural unit 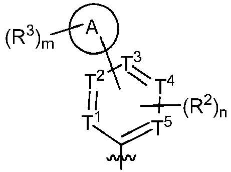 is selected from the group consisting of

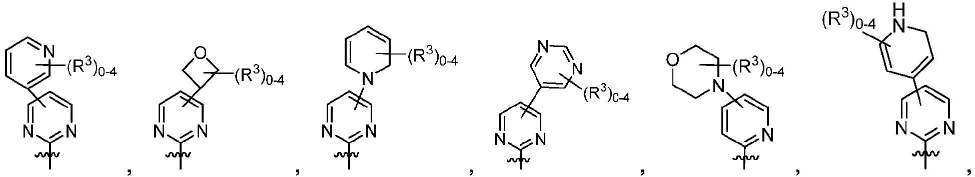

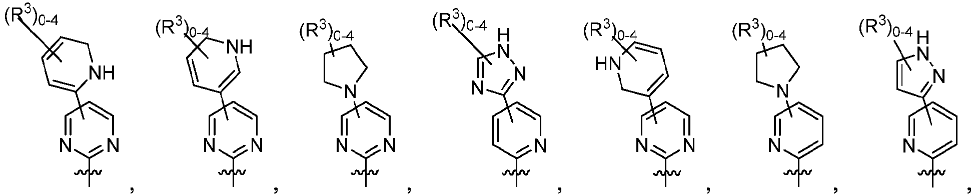

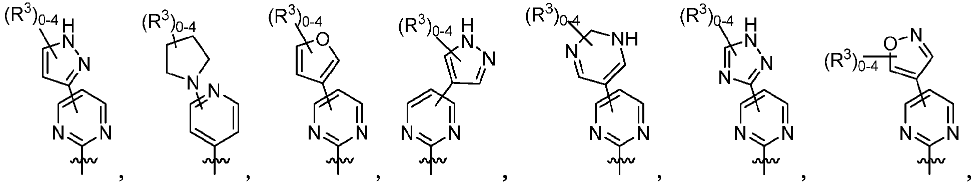

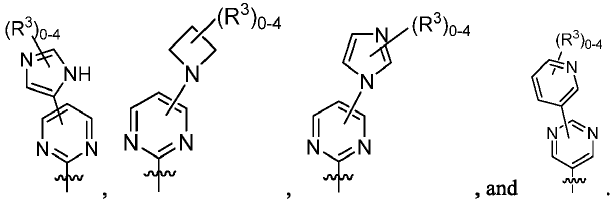

In some embodiments, structural unit 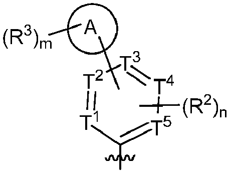 is selected from the group consisting of

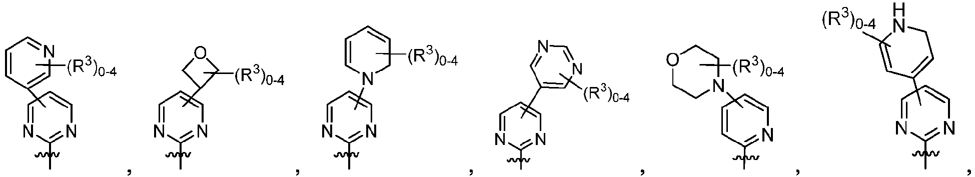

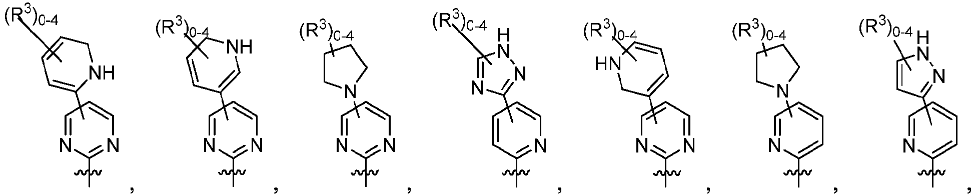

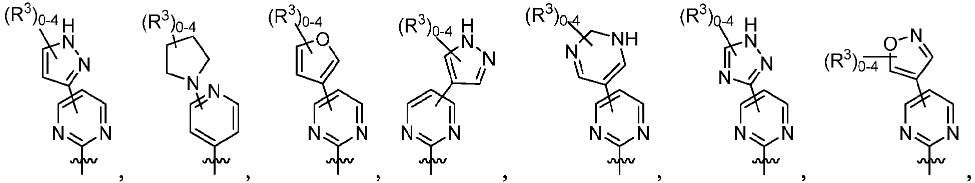

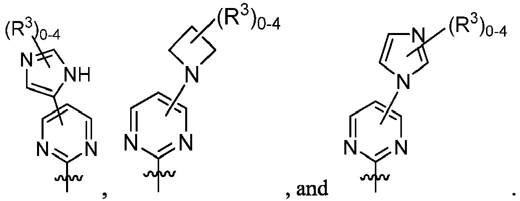

In some embodiments, structural unit 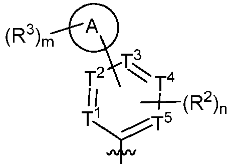 is selected from the group consisting of

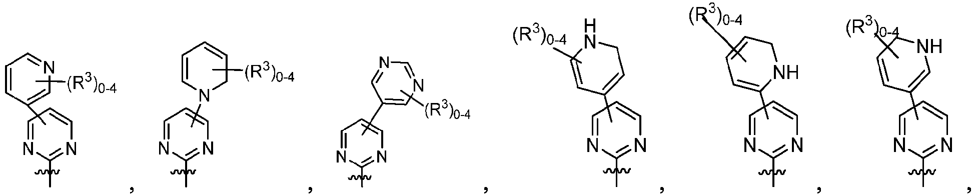

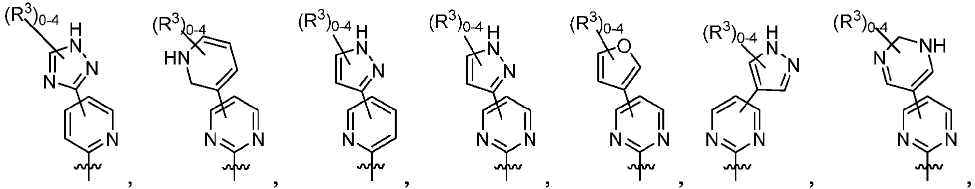

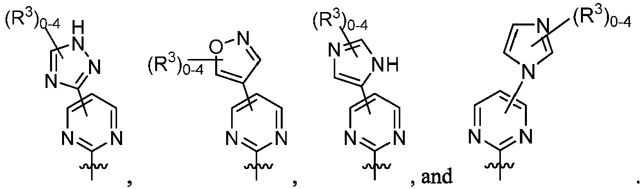

In some embodiments, structural unit 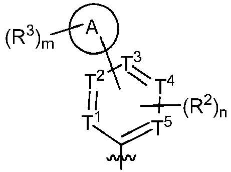 is selected from the group consisting of

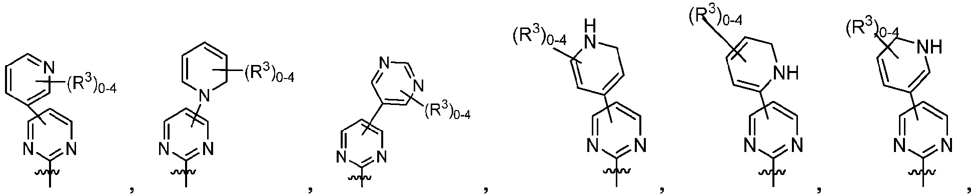

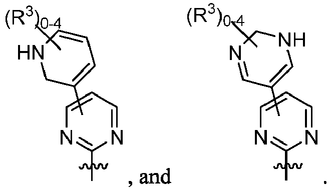

In some embodiments, structural unit 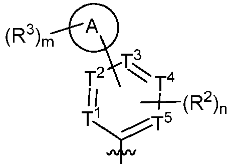 is selected from the group consisting of

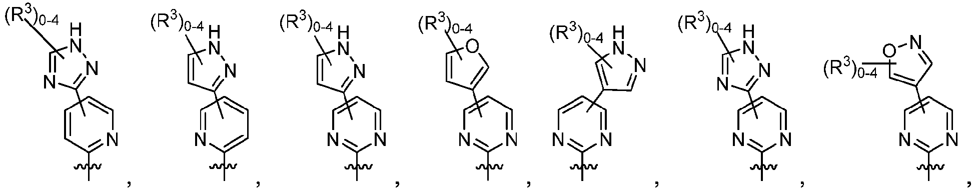

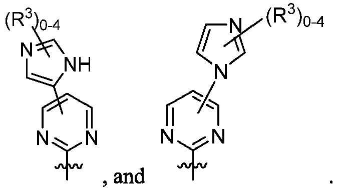

In some embodiments, structural unit 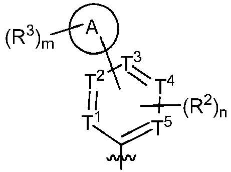 is selected from the group consisting of

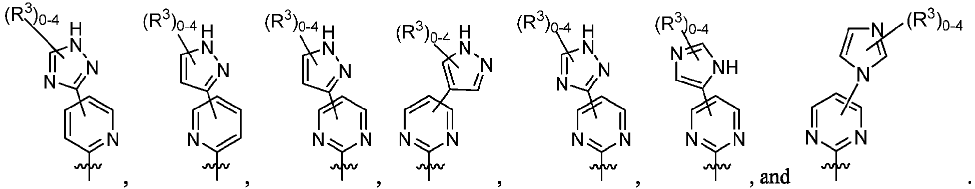

In some embodiments, structural unit 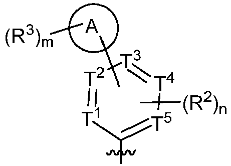 is selected from

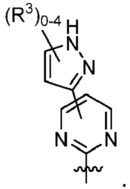

In some other embodiments, structural unit 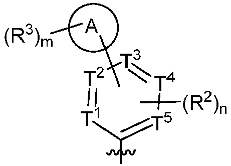 is selected from the group consisting of

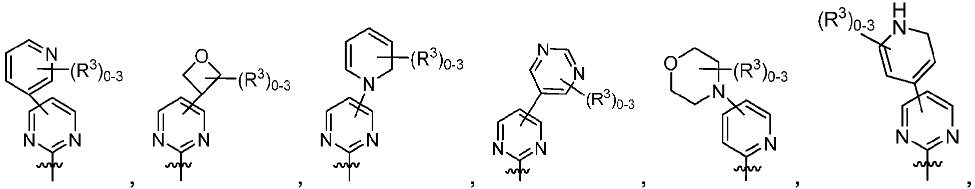

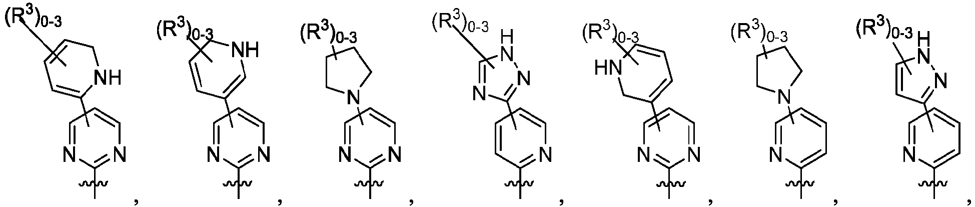

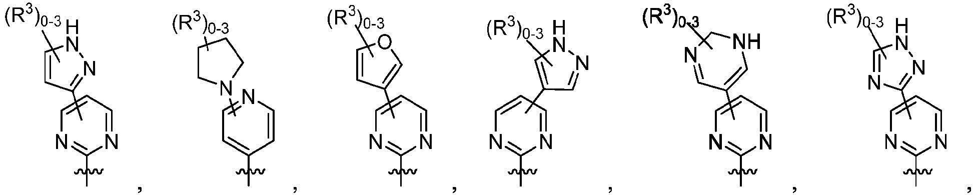

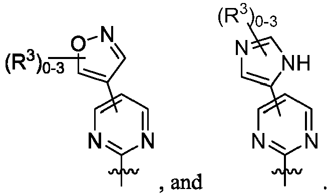

In some other embodiments, structural unit 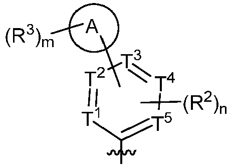 is selected from the group consisting of

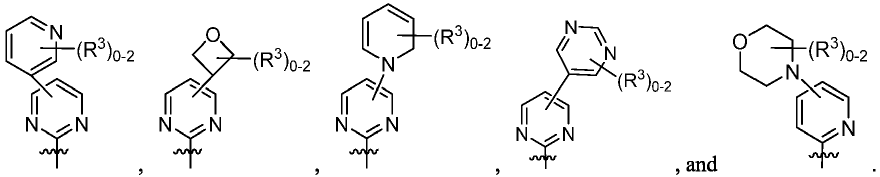

In some embodiments, structural unit 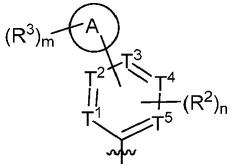 is selected from the group consisting of

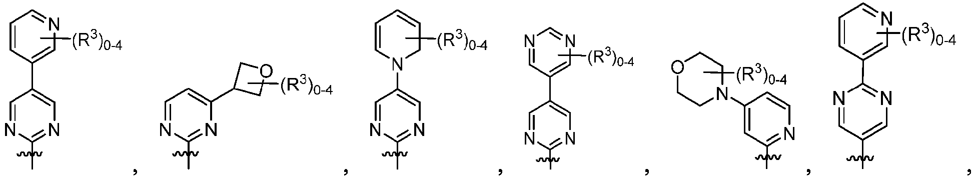

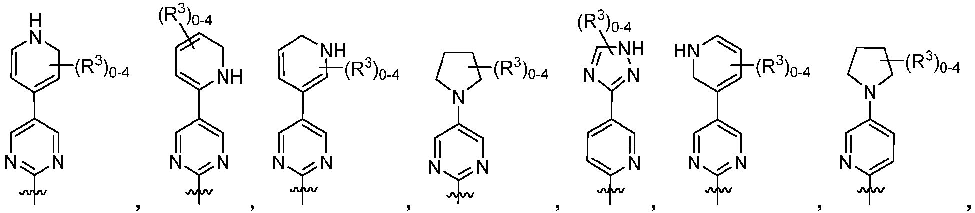

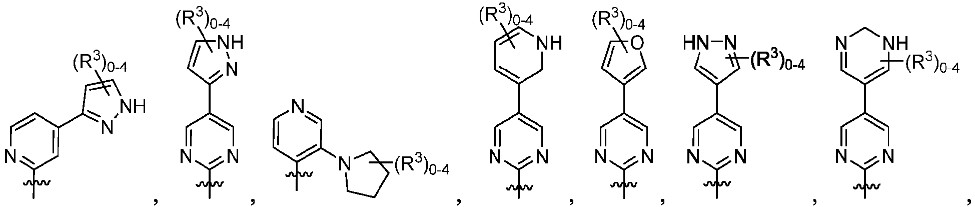

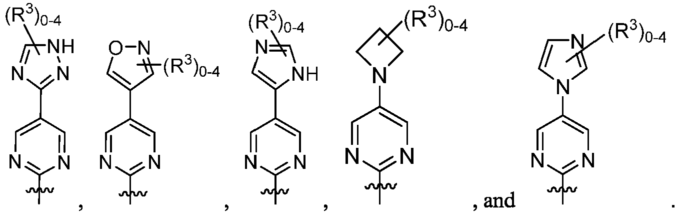

In some embodiments, structural unit 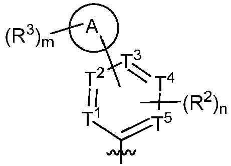 is selected from the group consisting of

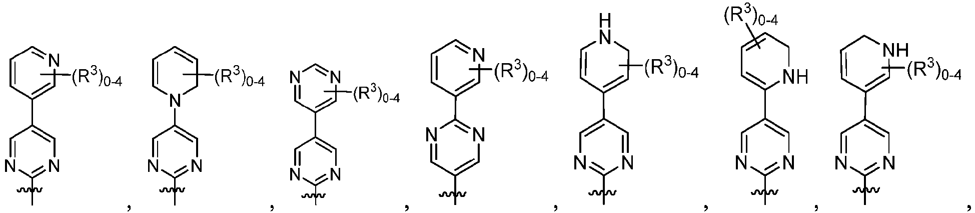

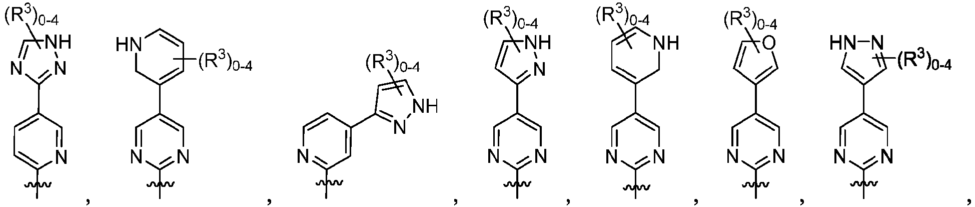

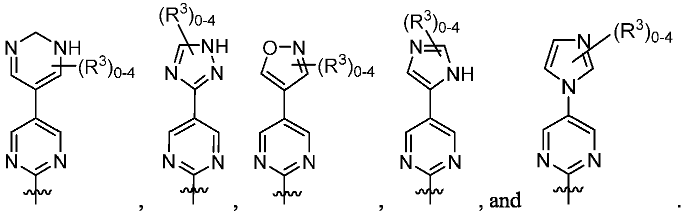

In some embodiments, structural unit 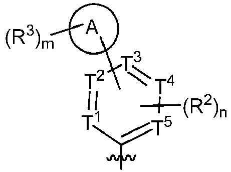 is selected from the group consisting of

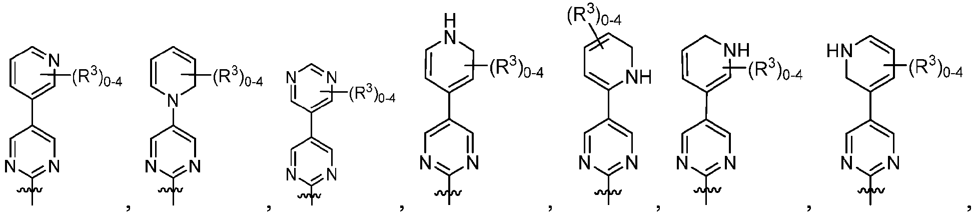

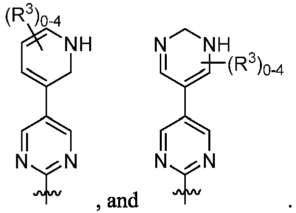

In some embodiments, structural unit 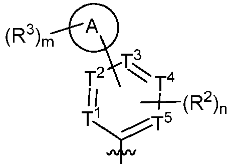 is selected from the group consisting of

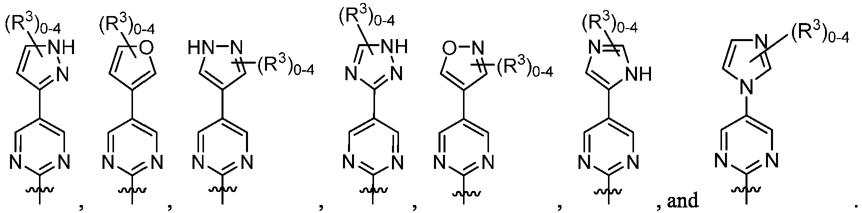

In some embodiments, structural unit 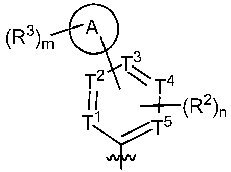 is selected from the group consisting of

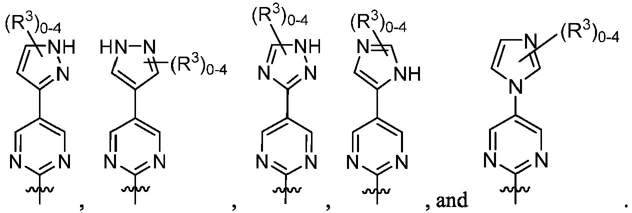

In some embodiments, structural unit 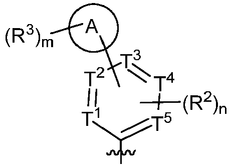 is selected from

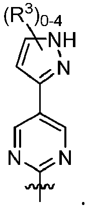

In some other embodiments, structural unit 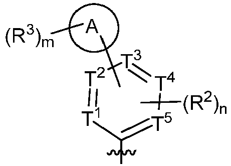 is selected from the group consisting of

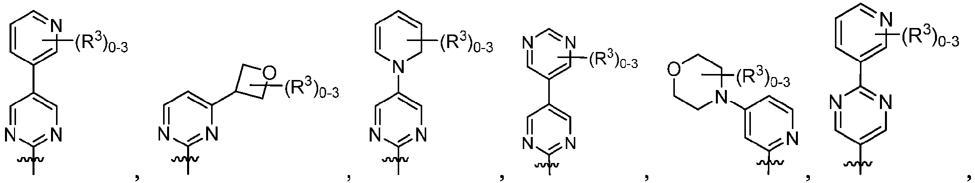

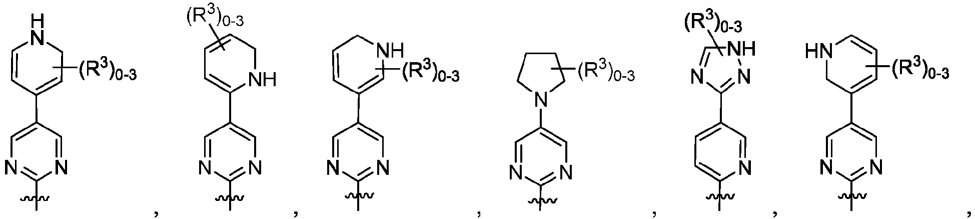

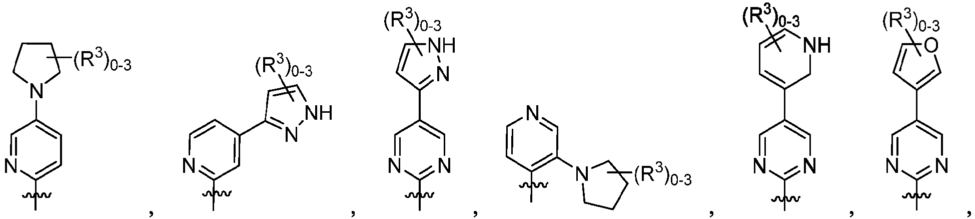

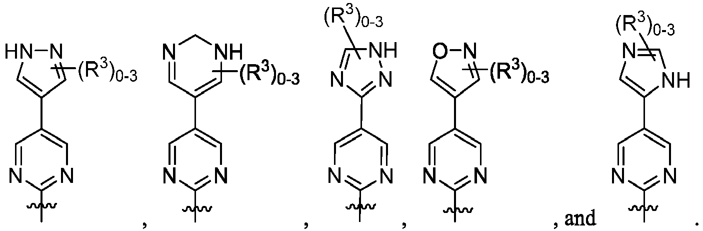

In some other embodiments, structural unit 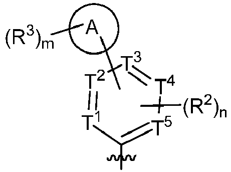 is selected from the group consisting of

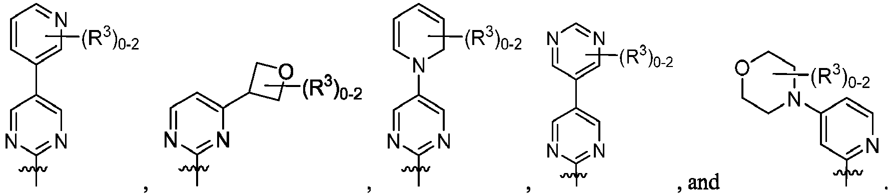

In some embodiments, structural unit 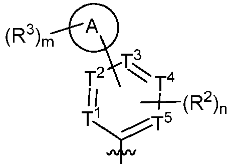 is selected from the group consisting of

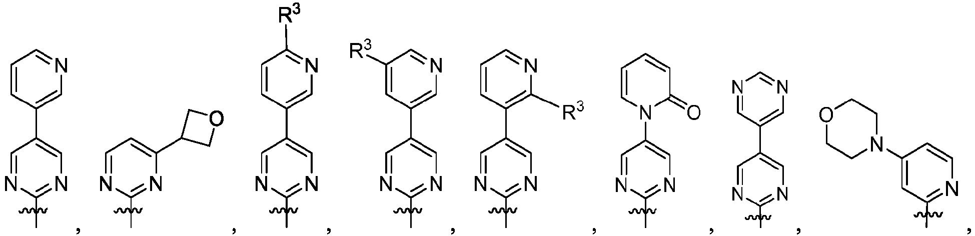

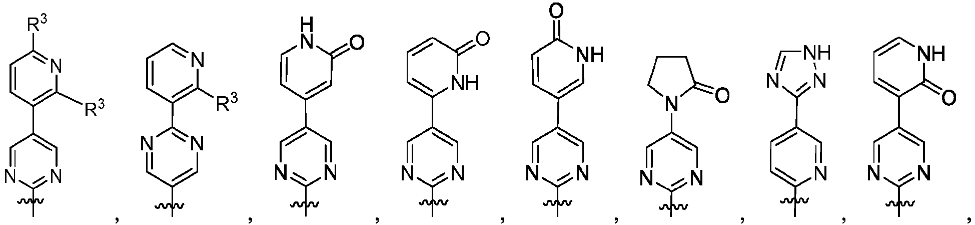

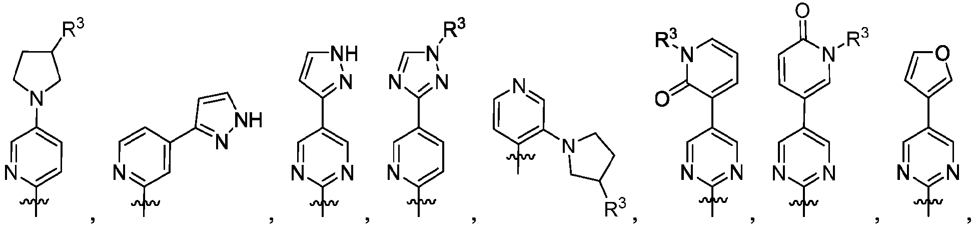

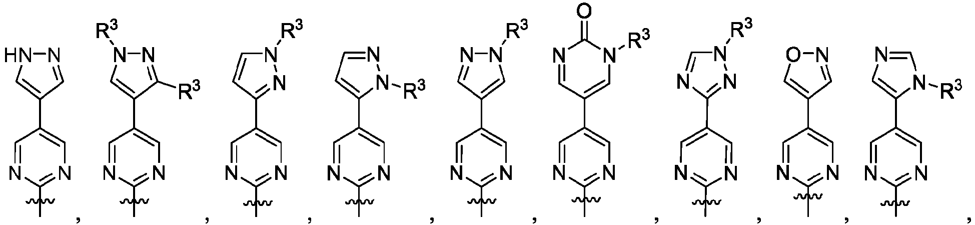

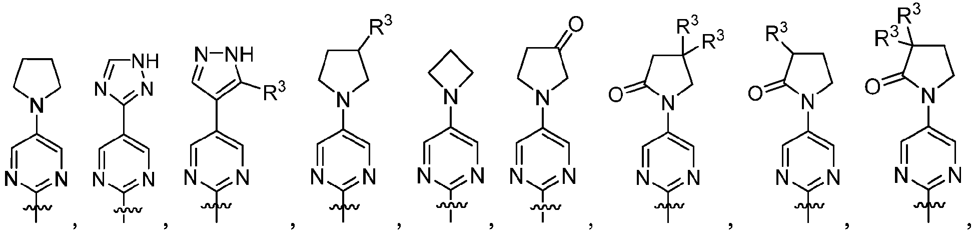

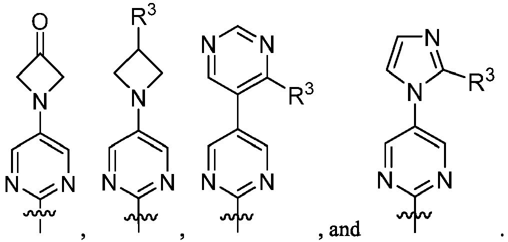

In some embodiments, structural unit 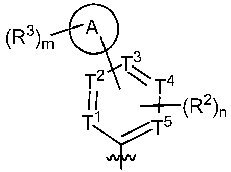 is selected from the group consisting of

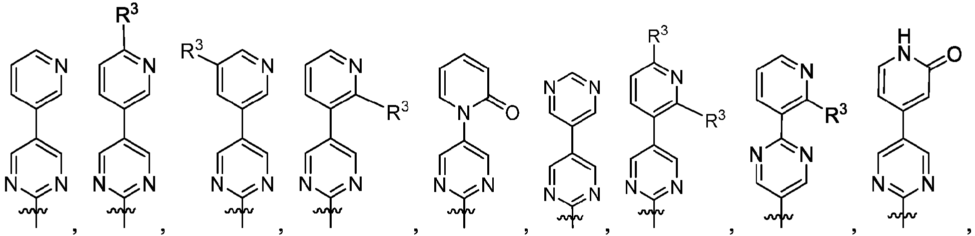

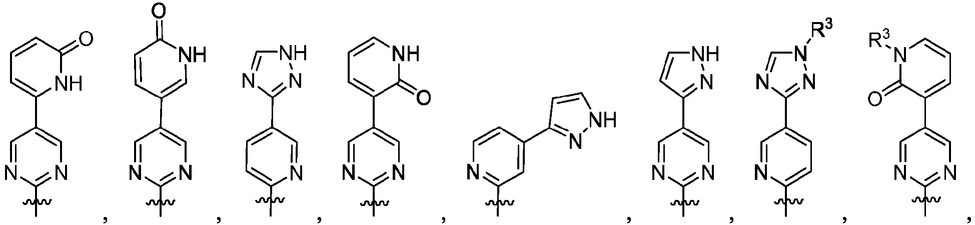

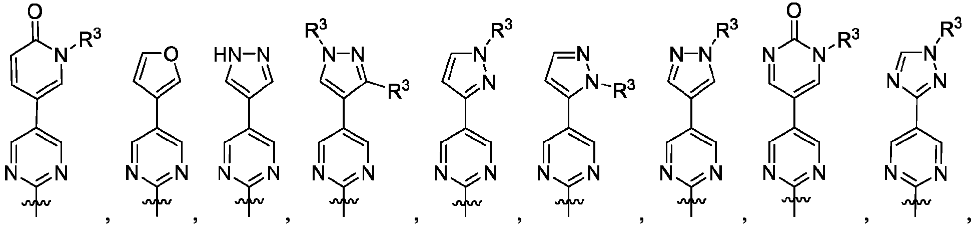

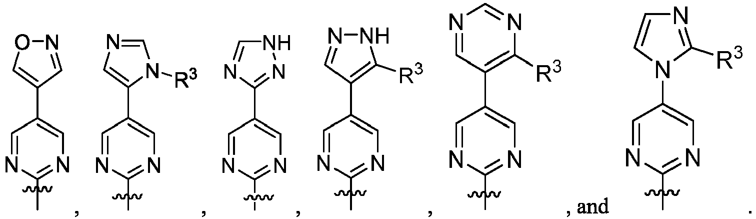

In some embodiments, structural unit 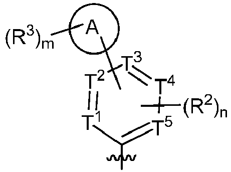 is selected from the group consisting of

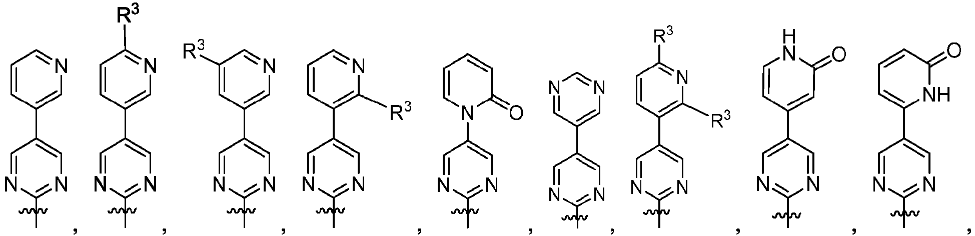

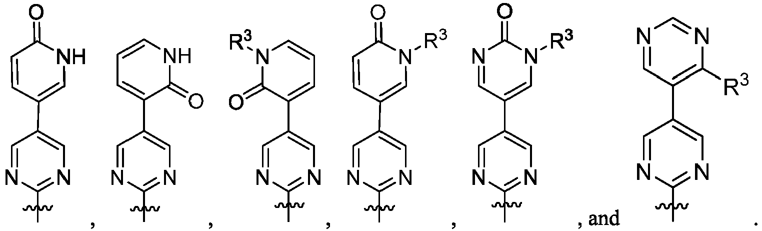

In some embodiments, structural unit 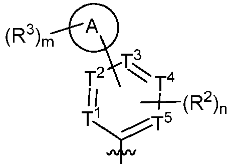 is selected from the group consisting of

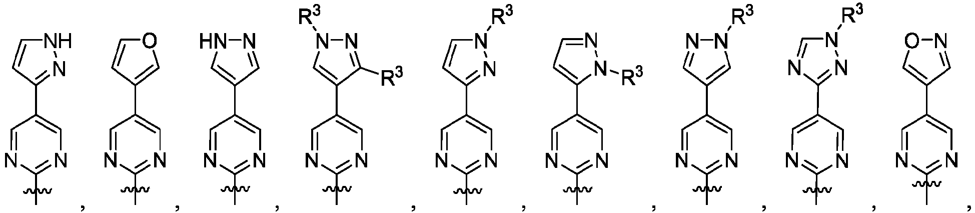

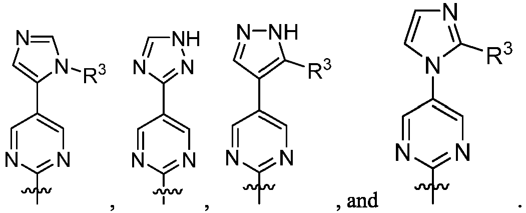

In some embodiments, structural unit 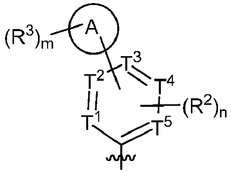 is selected from the group consisting of 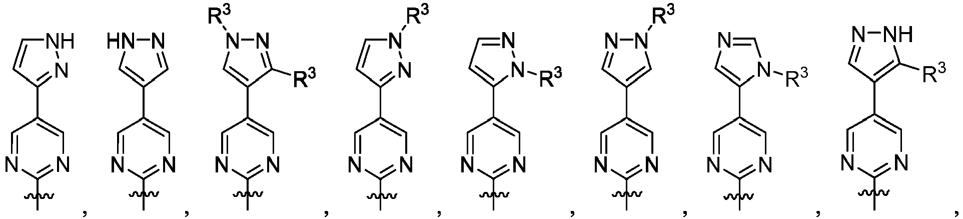 and

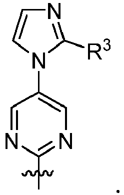

In some embodiments, structural unit 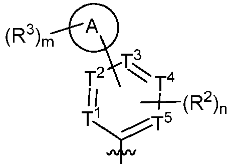 is selected from the group consisting of

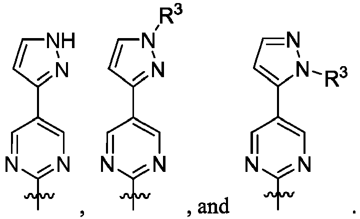

In some other embodiments, structural unit 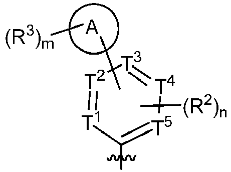 is selected from the group consisting of 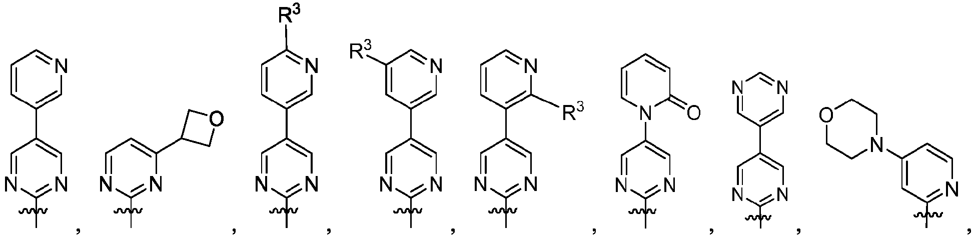 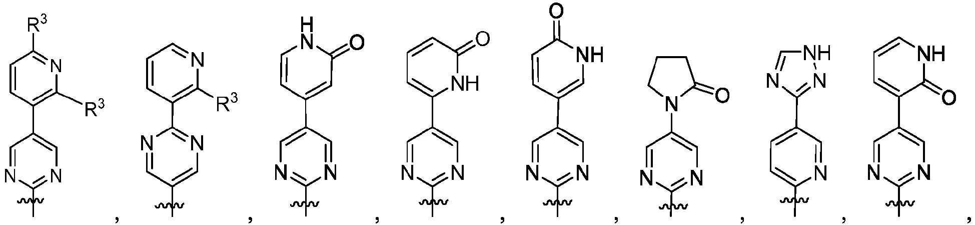 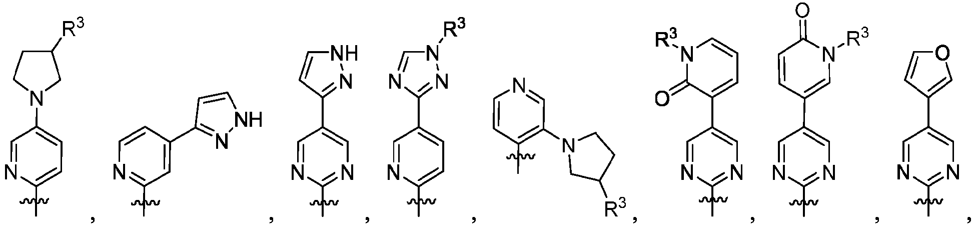 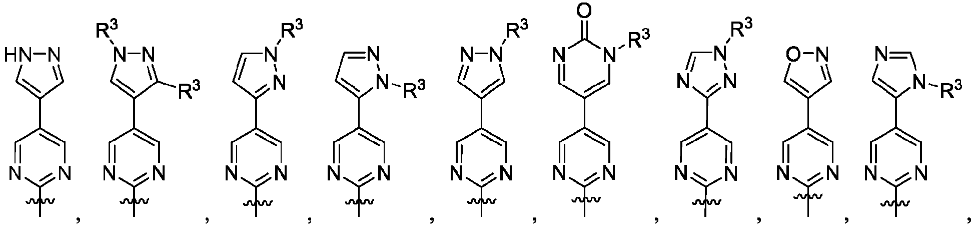 and

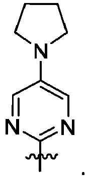

In some other embodiments, structural unit 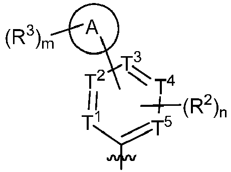 is selected from the group consisting of 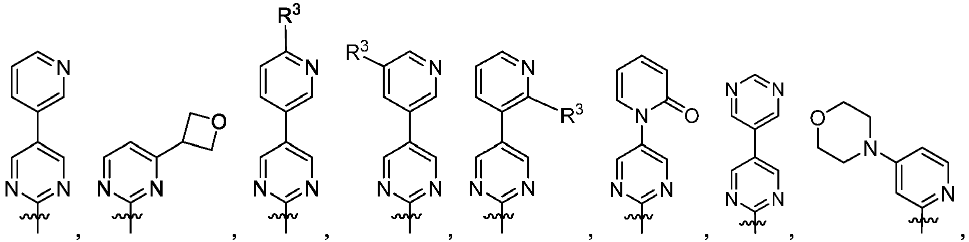 and

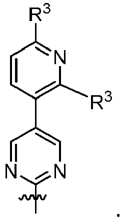

In some embodiments, structural unit 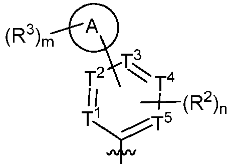 is selected from the group consisting of

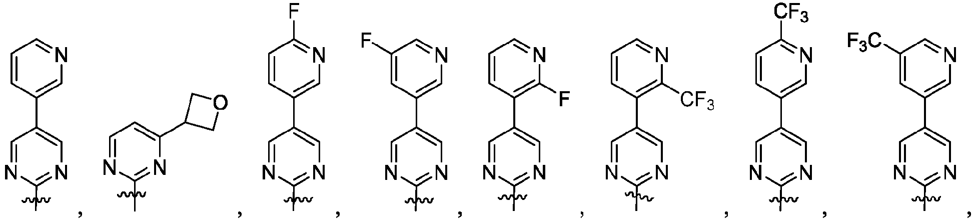

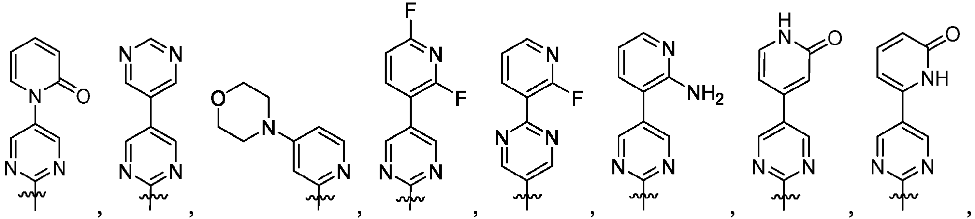

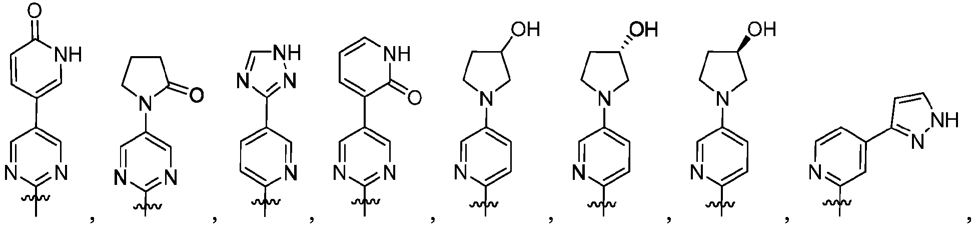

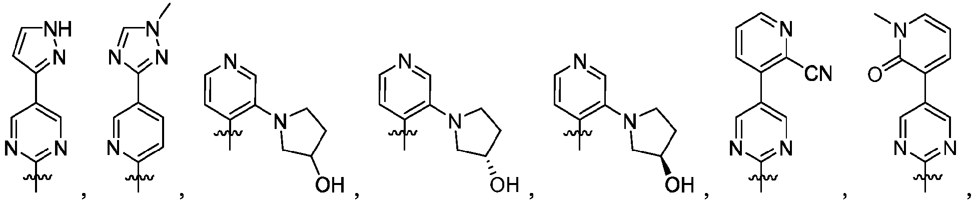

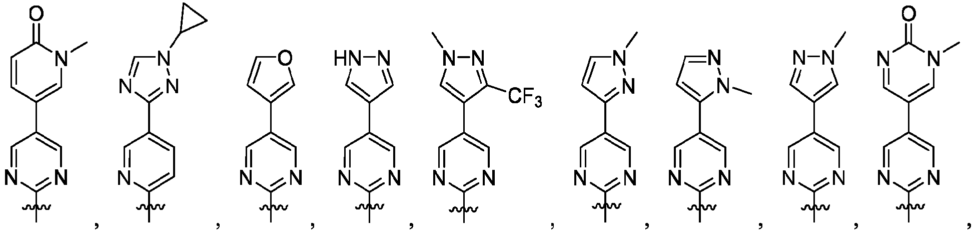

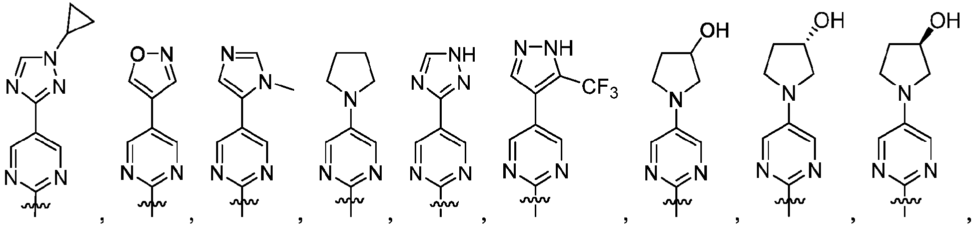

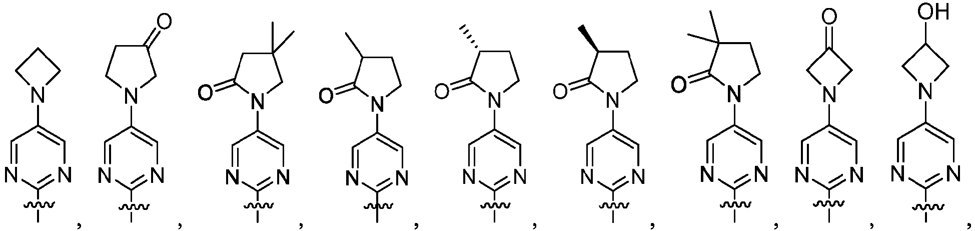

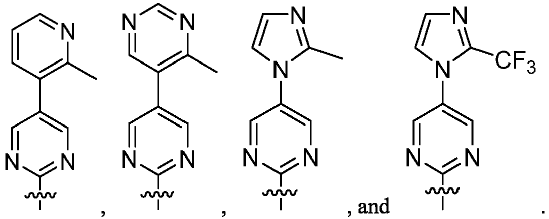

In some embodiments, structural unit 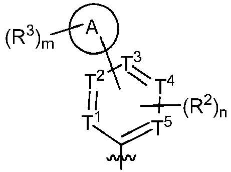 is selected from the group consisting of

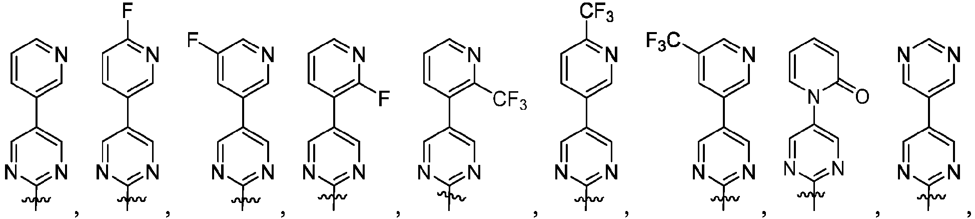

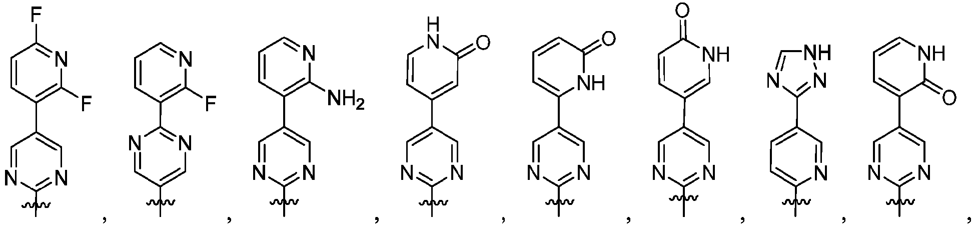

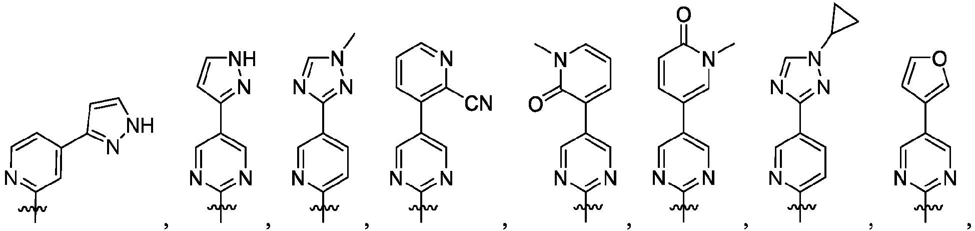

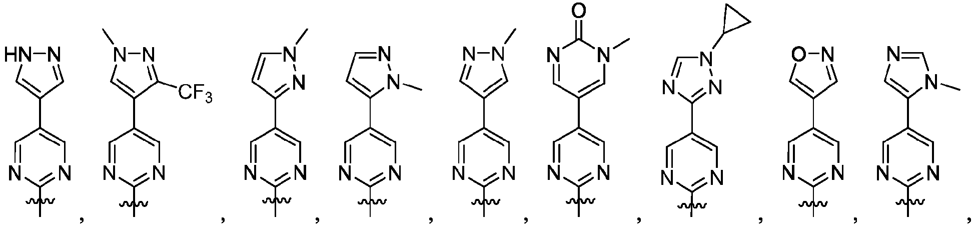

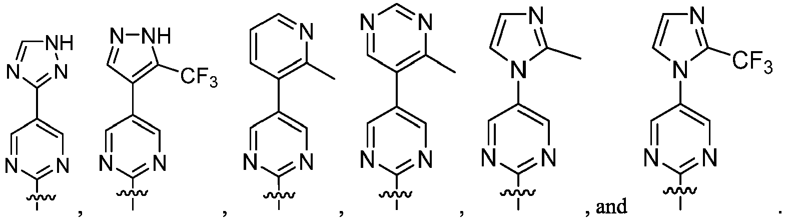

In some embodiments, structural unit 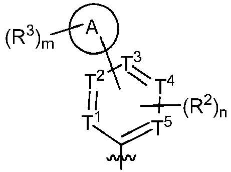 is selected from the group consisting of

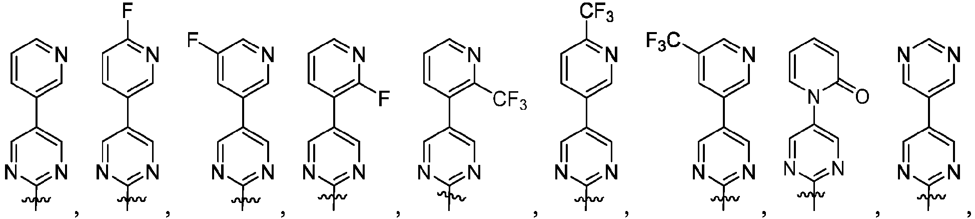

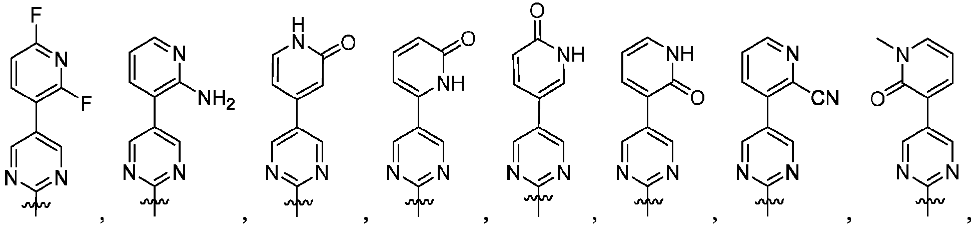

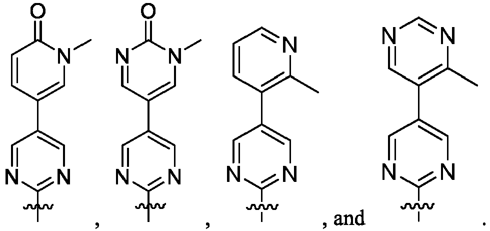

In some embodiments, structural unit 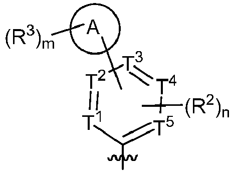 is selected from the group consisting of 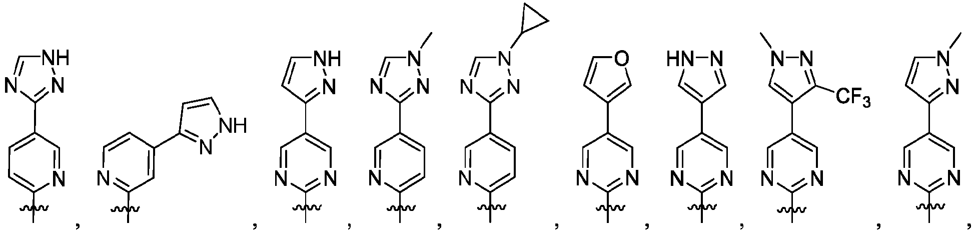 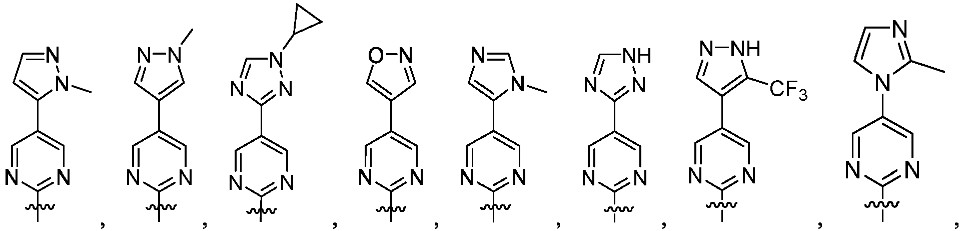 and

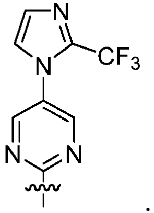

In some embodiments, structural unit 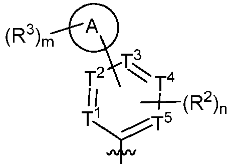 is selected from the group consisting of

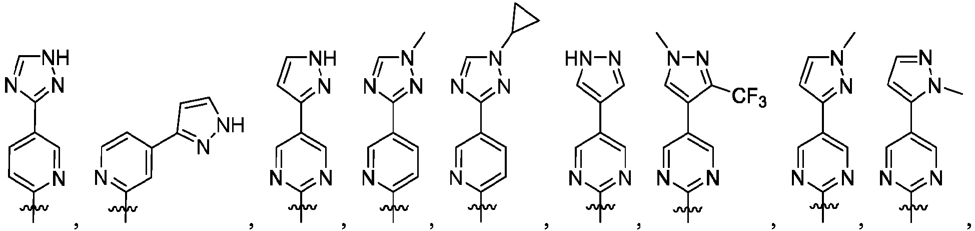

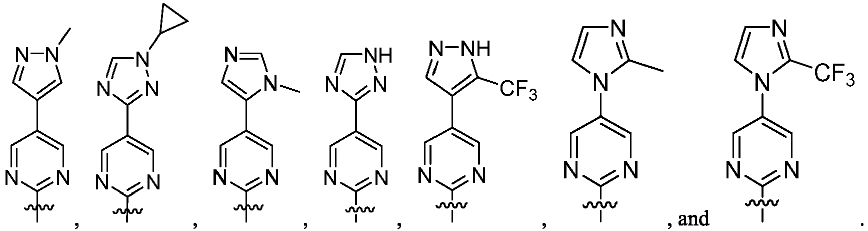

In some embodiments, structural unit 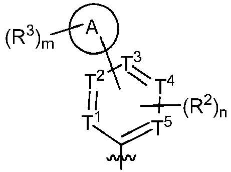 is selected from the group consisting of

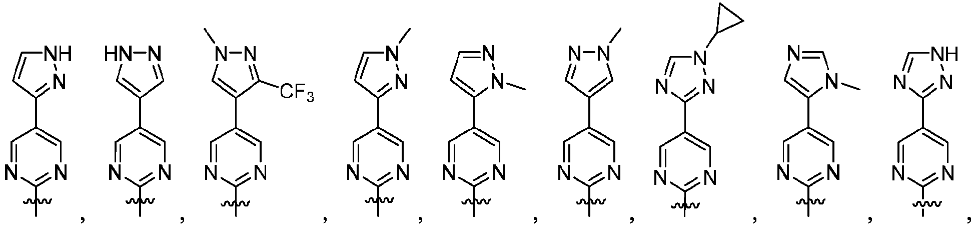

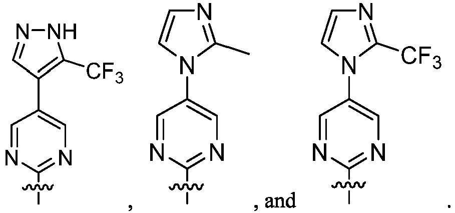

In some embodiments, structural unit 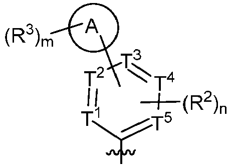 is selected from the group consisting of

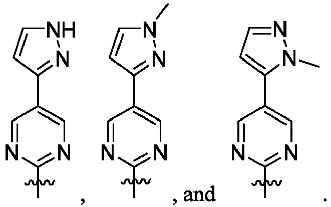

In some other embodiments, structural unit 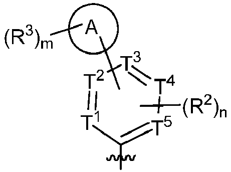 is selected from the group consisting of

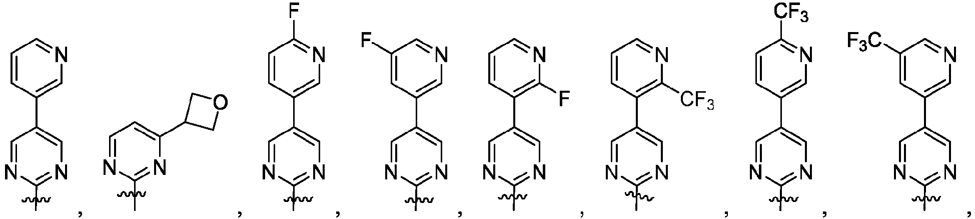

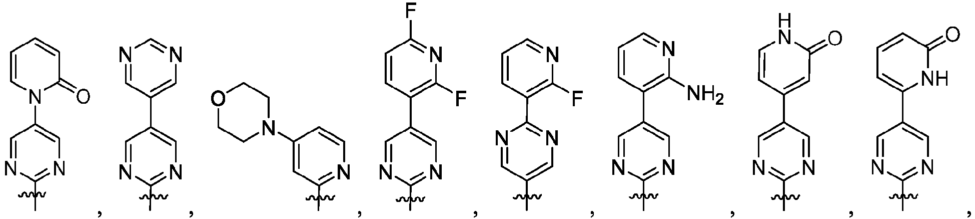

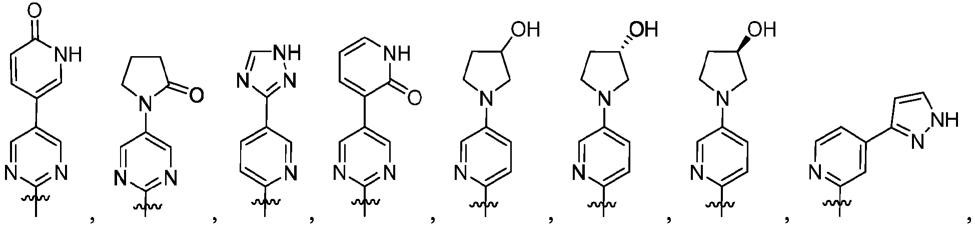

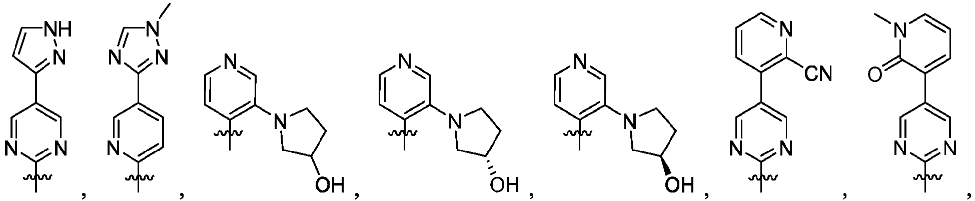

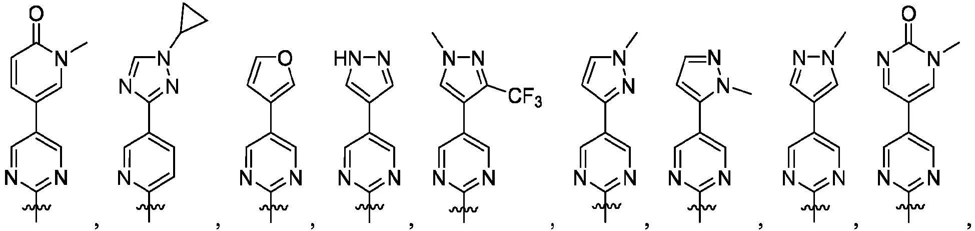

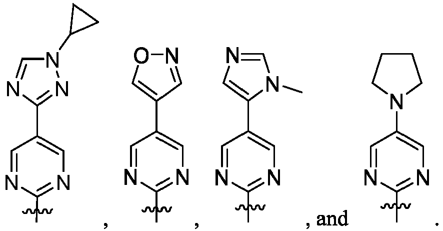

In some other embodiments, structural unit 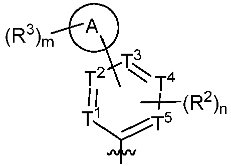 is selected from the group consisting of

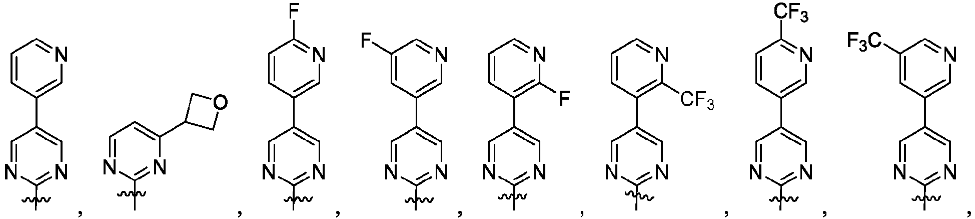

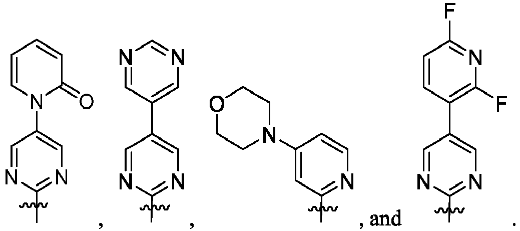

In a further aspect, the present disclosure provides a compound of formula I or a pharmaceutically acceptable salt thereof: 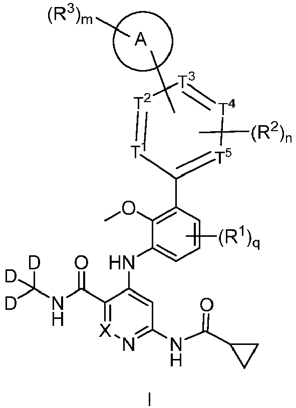 wherein,
X is selected from the group consisting of N and CH;
each R¹ is independently selected from halogen;
q is selected from the group consisting of 0, 1, and 2;
each R² is independently selected from the group consisting of halogen, hydroxy, amino, cyano, and nitro;
n is selected from the group consisting of 0, 1, and 2;
T¹, T², T³, T⁴, and T⁵ are each independently selected from the group consisting of CH and N, at least one of which is selected from CH, and T¹ and T⁴ are not both selected from N, and T² and T⁵ are not both selected from N;
ring A is selected from the group consisting of C₆₋₁₀ aryl, 3- to 8-membered heterocycloalkenyl, and 5- to 8-membered heteroaryl, wherein the 5- to 8-membered heteroaryl is selected from 5- to 8-membered heteroaryl containing 1 or 2 atoms selected from the group consisting of N and O;
each R³ is independently selected from the group consisting of =O, halogen, hydroxy, amino, cyano, nitro, C₁₋₈ alkyl, and C₁₋₈ alkoxy, wherein the C₁₋₈ alkyl or C₁₋₈ alkoxy is optionally substituted with one or more R^{a};
m is selected from the group consisting of 0, 1, 2, 3, and 4;
each R^{a} is independently selected from the group consisting of halogen, hydroxy, amino, and cyano.

In a further aspect, the present disclosure provides a compound of formula I or a pharmaceutically acceptable salt thereof: 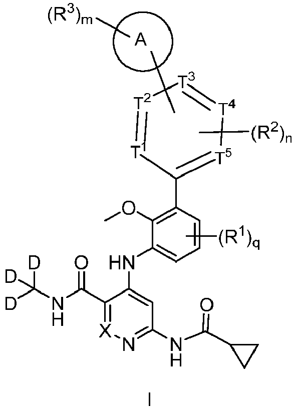 wherein,
X is selected from the group consisting of N and CH;
each R¹ is independently selected from halogen;
q is selected from 0;
each R² is independently selected from the group consisting of halogen, hydroxy, amino, cyano, and nitro;
n is selected from 0;
T¹, T², T³, T⁴, and T⁵ are each independently selected from the group consisting of CH and N, wherein T¹ and T⁵ are selected from N, and T², T³, and T⁴ are selected from CH;
ring A is selected from the group consisting of C₆₋₁₀ aryl, 5- to 6-membered heterocycloalkenyl, and 5- to 6-membered heteroaryl, wherein the 5- to 6-membered heterocycloalkenyl is selected from 5- to 6-membered heterocycloalkenyl containing 1 or 2 nitrogen atoms, and the 5- to 6-membered heteroaryl is selected from 5- to 6-membered heteroaryl containing 1 or 2 nitrogen atoms;
each R³ is independently selected from the group consisting of =O, halogen, hydroxy, amino, cyano, nitro, C₁₋₃ alkyl, and C₁₋₃ alkoxy, wherein the C₁₋₃ alkyl or C₁₋₃ alkoxy is optionally substituted with one or more R^{a};
m is selected from the group consisting of 0, 1, and 2;
each R^{a} is independently selected from the group consisting of halogen and hydroxy.

In a further aspect, the present disclosure provides a compound of formula I or a pharmaceutically acceptable salt thereof: 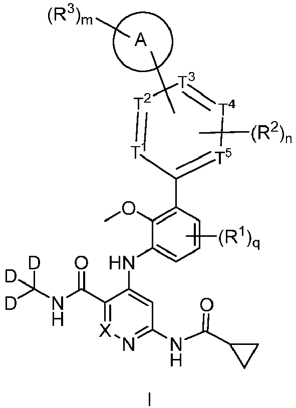 wherein,
X is selected from the group consisting of N and CH;
each R¹ is independently selected from halogen;
q is selected from 0;
each R² is independently selected from the group consisting of halogen, hydroxy, amino, cyano, and nitro;
n is selected from 0;
T¹, T², T³, T⁴, and T⁵ are each independently selected from the group consisting of CH and N, wherein T¹ and T⁵ are selected from N, and T², T³, and T⁴ are selected from CH;
ring A is selected from 5-membered heteroaryl, wherein the 5-membered heteroaryl is selected from 5-membered heteroaryl containing 1 or 2 nitrogen atoms;
each R³ is independently selected from the group consisting of =O, halogen, hydroxy, and C₁₋₃ alkyl, the C₁₋₃ alkyl being optionally substituted with one or more R^{a}; preferably, each R³ is independently selected from the group consisting of =O, fluorine, chlorine, bromine, and C₁₋₃ alkyl, the C₁₋₃ alkyl being optionally substituted with one or more R^{a}; more preferably, each R³ is independently selected from the group consisting of =O, fluorine, and methyl, the methyl being optionally substituted with one or more fluorine;
m is selected from the group consisting of 0, 1, and 2;
each R^{a} is independently selected from halogen.

In a further aspect, the present disclosure provides a compound of formula I or a pharmaceutically acceptable salt thereof: 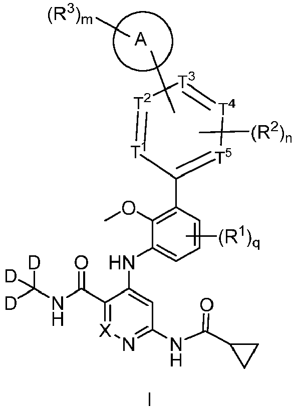 wherein,
X is selected from the group consisting of N and CH;
each R¹ is independently selected from halogen;
q is selected from 0;
each R² is independently selected from the group consisting of halogen, hydroxy, amino, cyano, and nitro;
n is selected from 0;
T¹, T², T³, T⁴, and T⁵ are each independently selected from the group consisting of CH and N, at least one of which is selected from CH, and T¹ and T⁴ are not both selected from N, and T² and T⁵ are not both selected from CH; preferably, T¹ and T⁵ are selected from N, and T², T³, and T⁴ are selected from CH, or T¹ or T⁵ is selected from N, and the remaining four are selected from CH;
ring A is selected from 5-membered heteroaryl, wherein the 5-membered heteroaryl is selected from 5-membered heteroaryl containing 1, 2, or 3 nitrogen atoms; preferably, the 5-membered heteroaryl is selected from 5-membered heteroaryl containing 3 nitrogen atoms;
each R³ is independently selected from C₃₋₆ cycloalkyl, the C₃₋₆ cycloalkyl being optionally substituted with one or more R^{a}; preferably, each R³ is independently selected from cyclopropyl optionally substituted by one or more R^{a};
m is selected from the group consisting of 1 and 2;
each R^{a} is independently selected from halogen.

In some embodiments, the compound of formula I or the pharmaceutically acceptable salt thereof is selected from the group consisting of a compound of formula I-1, a compound of formula I-2, a compound of formula I-3, a compound of formula I-4, a compound of formula I-5, a compound of formula I-6, a compound of formula I-7, and pharmaceutically acceptable salts thereof 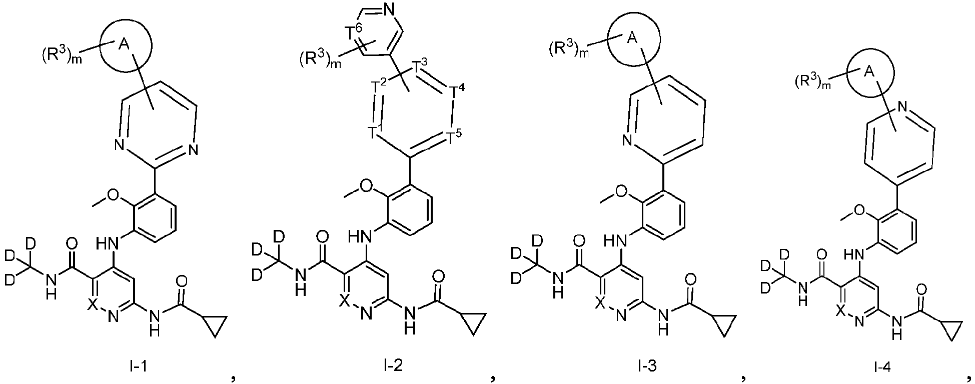 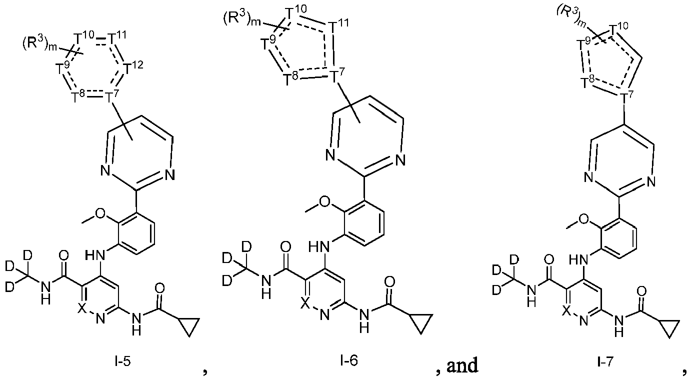
wherein, T⁶ is selected from the group consisting of C and N; T¹, T², T³, T⁴, T⁵, X, R³, m, and ring A are defined as above;
T⁷, T⁸, T⁹, T¹⁰, T¹¹, and T¹² are each independently selected from the group consisting of CH, C, NH, N, O, and a bond;
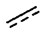 represents a single bond or double bond.

In some embodiments, T⁷, T⁸, T⁹, T¹⁰, T¹¹, and T¹² described above are each independently selected from the group consisting of CH, C, NH, N, O, and a bond, one or two of which are selected from the group consisting of NH and N. In some embodiments, T⁷, T⁸, T⁹, T¹⁰, T¹¹, and T¹² described above are each independently selected from the group consisting of CH, C, NH, N, O, and a bond, one or two of which are selected from the group consisting of NH and N and one of which is selected from a bond.

In some embodiments, T⁷, T⁸, T⁹, T¹⁰, and T¹¹ described above are each independently selected from the group consisting of CH, C, NH, N, and O, one or two of which are selected from the group consisting of NH and N.

In some embodiments, T⁷, T⁸, T⁹, and T¹⁰ described above are each independently selected from the group consisting of CH, C, NH, N, and O, one or two of which are selected from the group consisting of NH and N.

In some embodiments, T⁷, T⁸, T⁹, and T¹⁰ described above are each independently selected from the group consisting of CH, C, NH, and N, one or two of which are selected from the group consisting of NH and N.

In some embodiments, 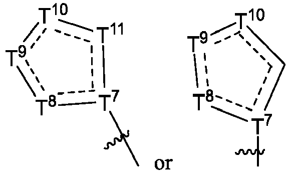 described above is a heteroaromatic ring system.

In some embodiments, structural unit 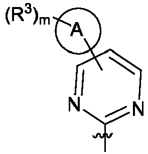 is selected from the group consisting of 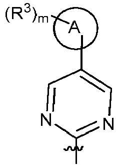 and

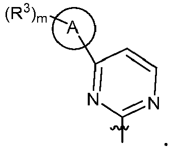

In some embodiments, structural unit 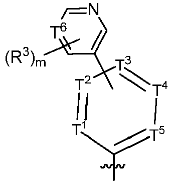 is selected from

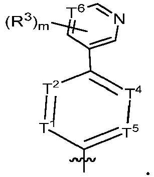

In some embodiments, structural unit 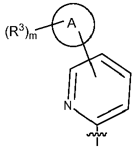 is selected from the group consisting of 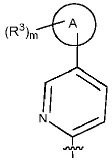 and

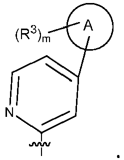

In some embodiments, structural unit 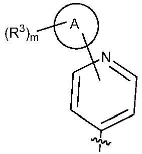 is selected from

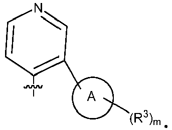

In some embodiments, structural unit 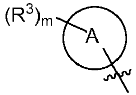 is defined as above.

In some embodiments, structural unit 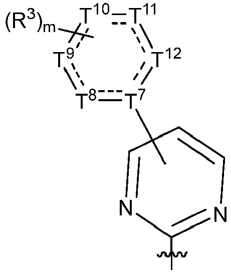 is selected from

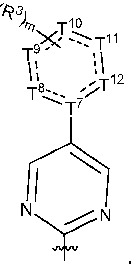

In some embodiments, structural unit 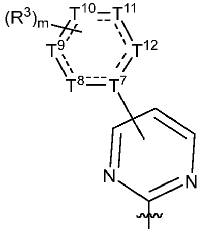 is selected from the group consisting of 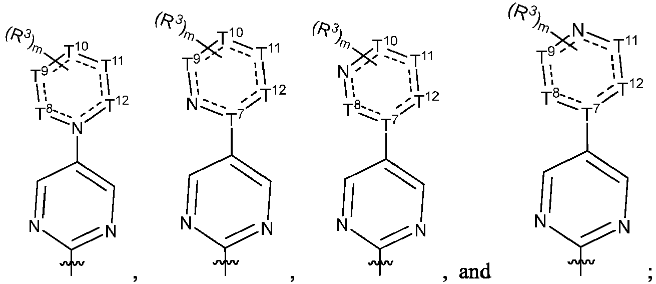 in some embodiments, structural unit 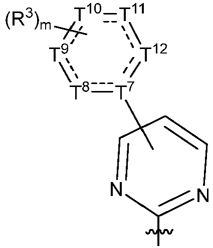 is selected from the group consisting of

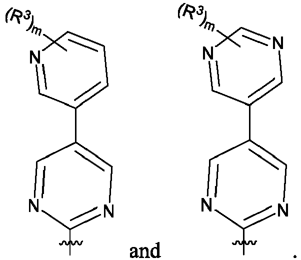

In some embodiments, structural uni 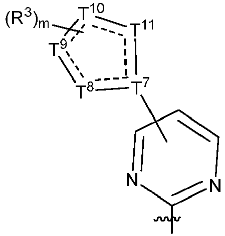 is selected from 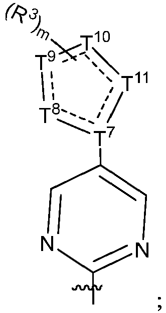 in some embodiments, structural unit 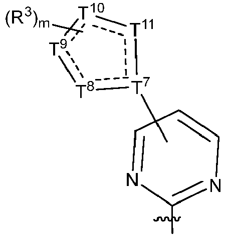 is selected from the group consisting of 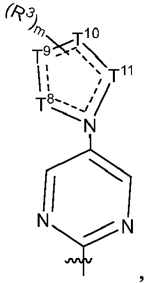 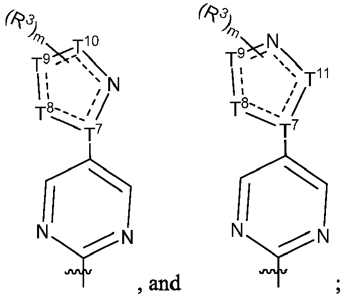 in some embodiments, structural unit 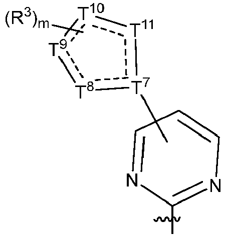 is selected from the group consisting of 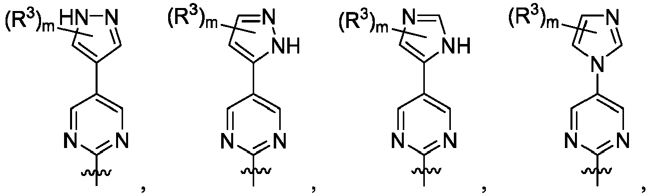 and 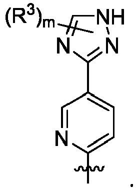 In some embodiments, structural unit 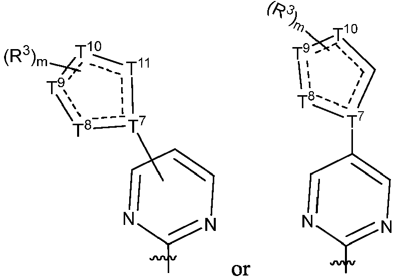 is selected from the group consisting of 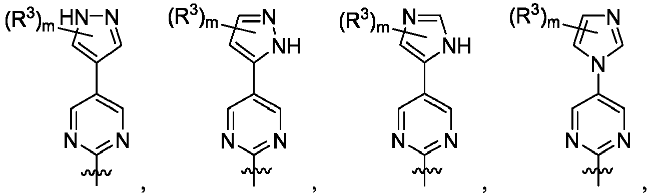 and 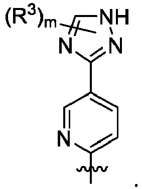 In some embodiments, structural unit 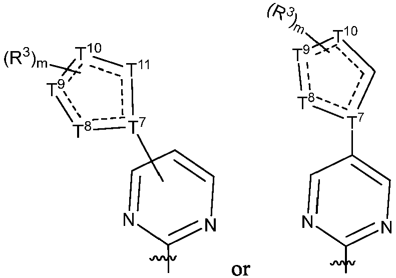 is selected from

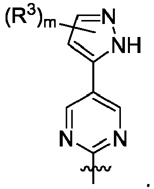

In some embodiments, the present disclosure encompasses the variables defined above and embodiments thereof, as well as any combination thereof.

The heteroatom in the heterocyclyl, heterocycloalkyl, heterocycloalkenyl, or heteroaryl described above is selected from the group consisting of nitrogen, oxygen, and sulfur, and the remaining ring atoms are selected from carbon. In some embodiments, the number of the heteroatom is selected from the group consisting of 1, 2, 3, and 4. In some embodiments, the number of the heteroatom is selected from the group consisting of 1, 2, and 3. In some embodiments, the number of the heteroatom is selected from the group consisting of 1 and 2.

"One or more" used herein refers to an integer ranging from one to ten. For example, "one or more" refers to one, two, three, four, five, six, seven, eight, nine, or ten; or "one or more" refers to one, two, three, four, five, or six; or "one or more" refers to one, two, or three.

In some embodiments, the compound disclosed herein is not a compound of a formula selected from the group consisting of the following formulas or a pharmaceutically acceptable salt thereof: 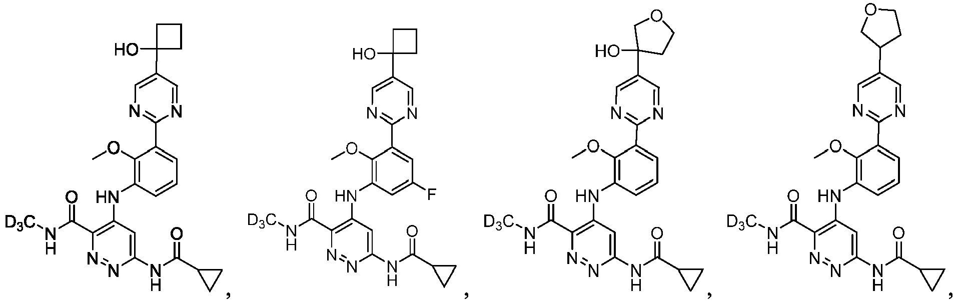 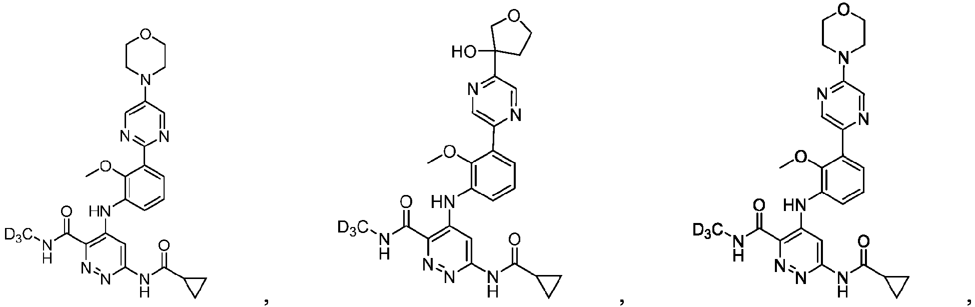 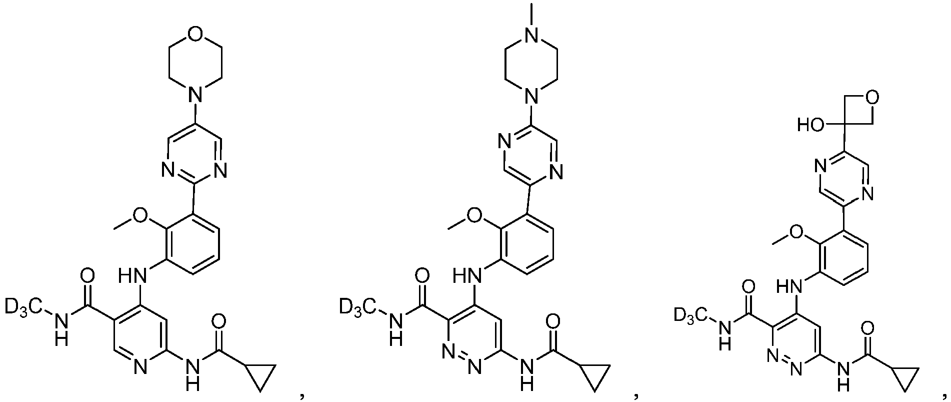 and

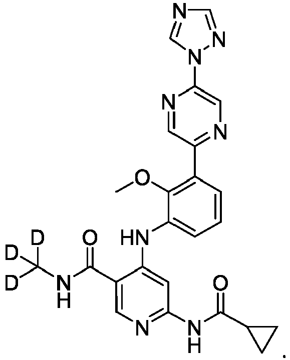

In some embodiments, the compound disclosed herein is not a compound of a formula selected from the group consisting of the following formulas or a pharmaceutically acceptable salt thereof:

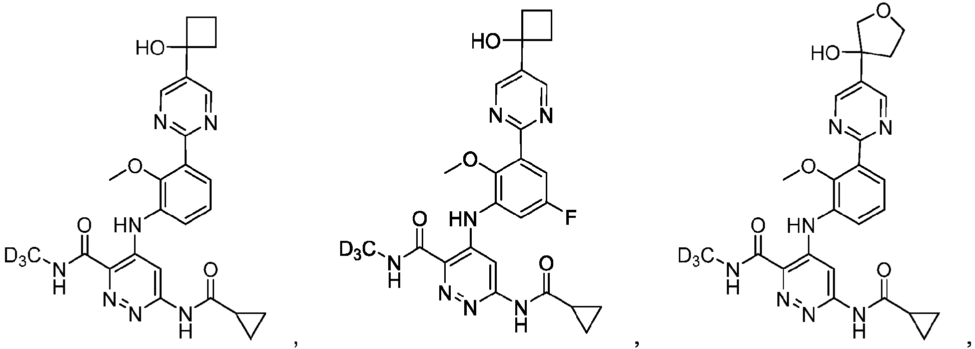

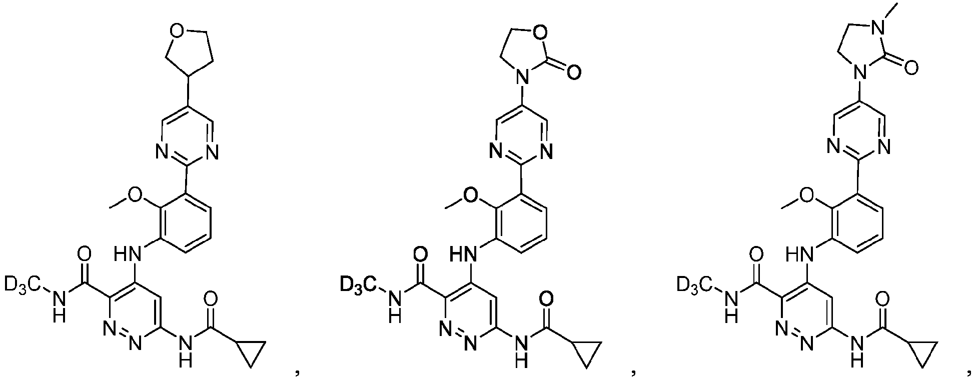

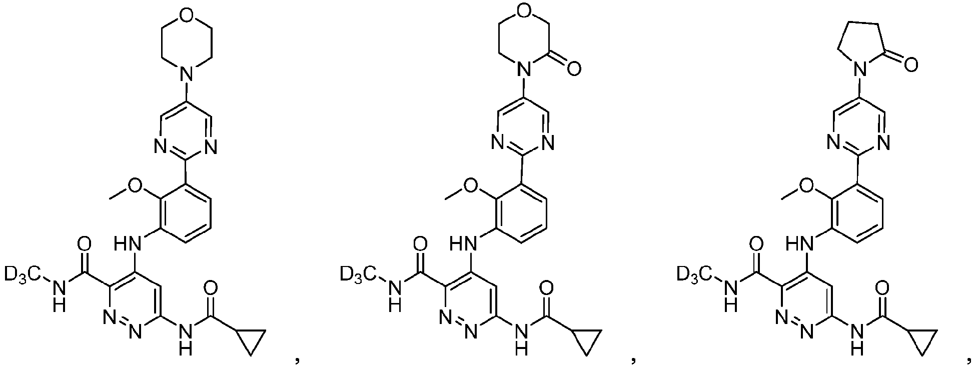

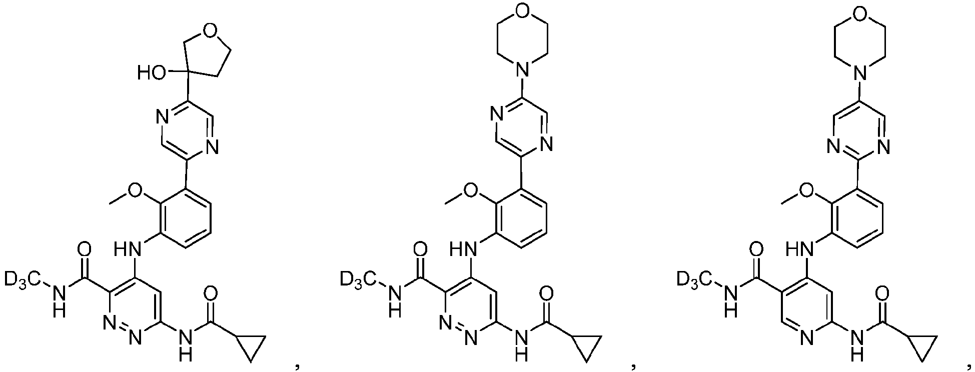

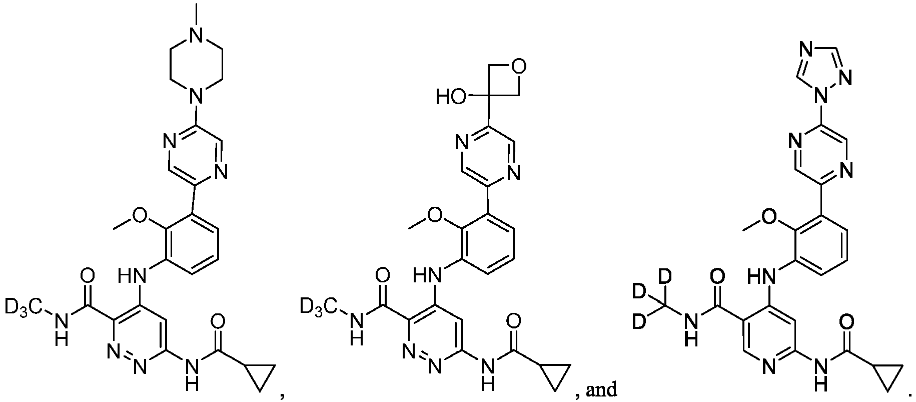

In another aspect, the present disclosure provides a compound of a formula selected from the group consisting of the following formulas or a pharmaceutically acceptable salt thereof:

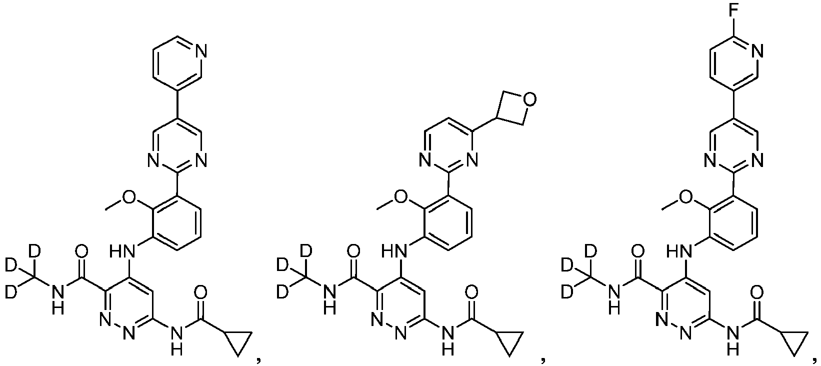

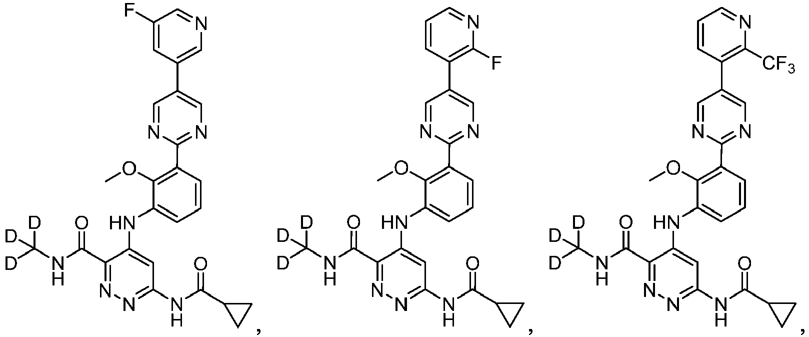

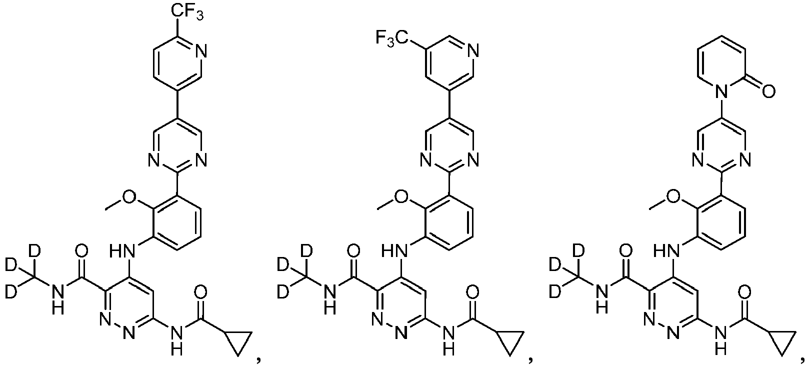

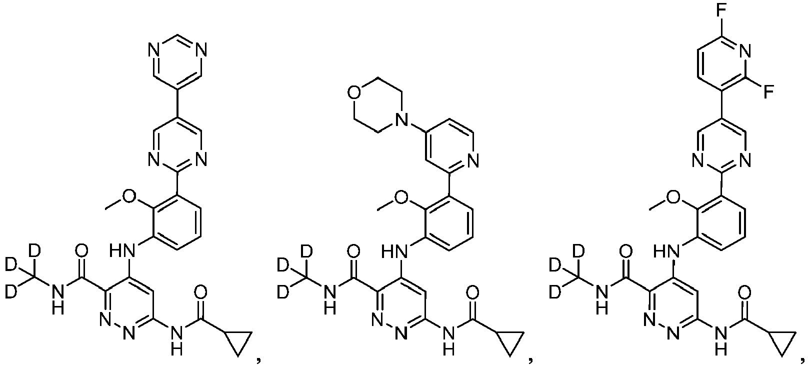

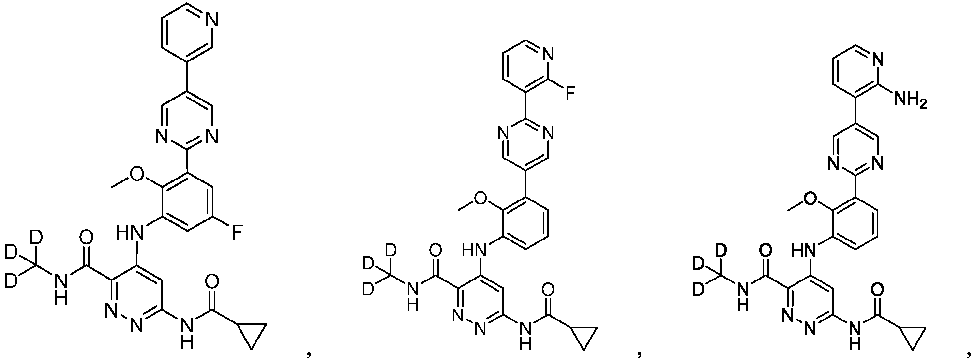

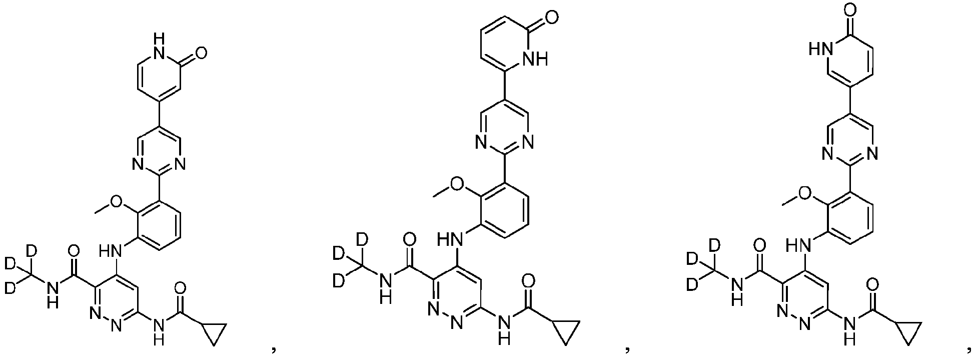

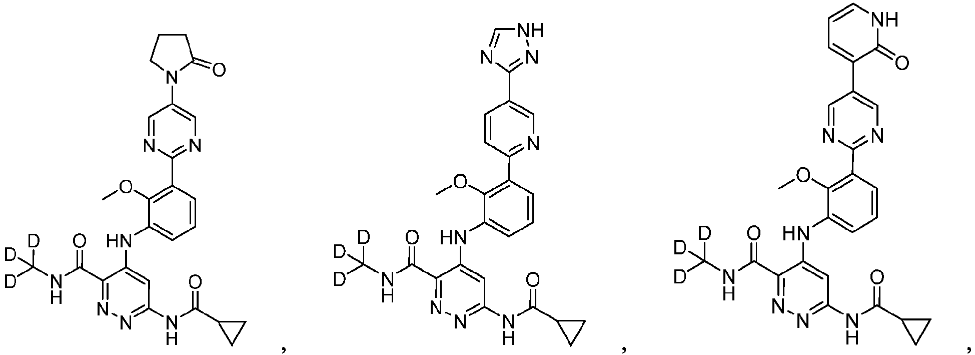

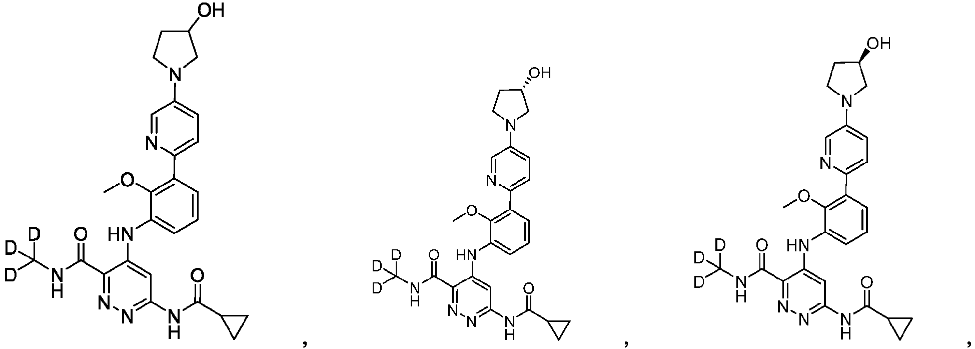

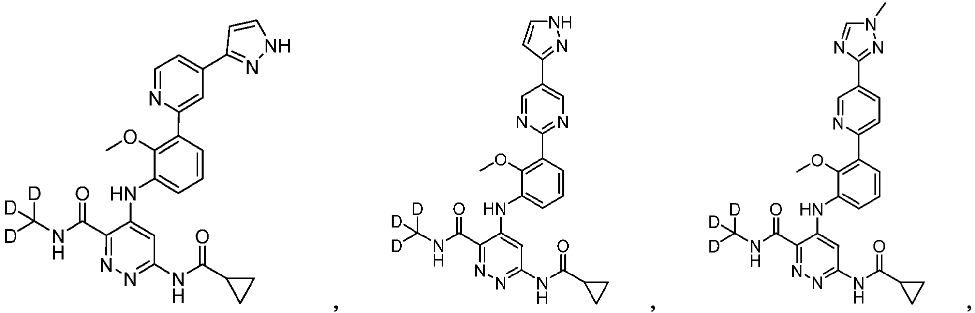

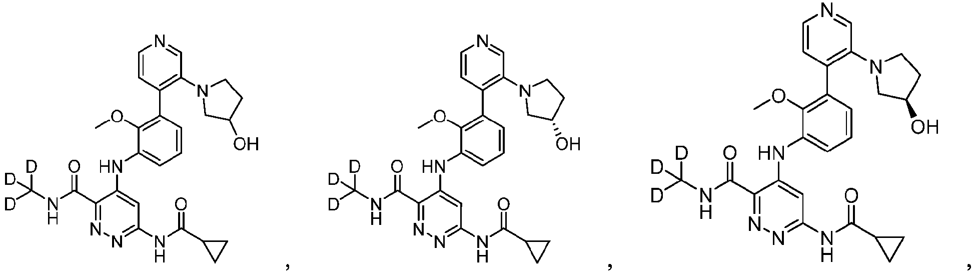

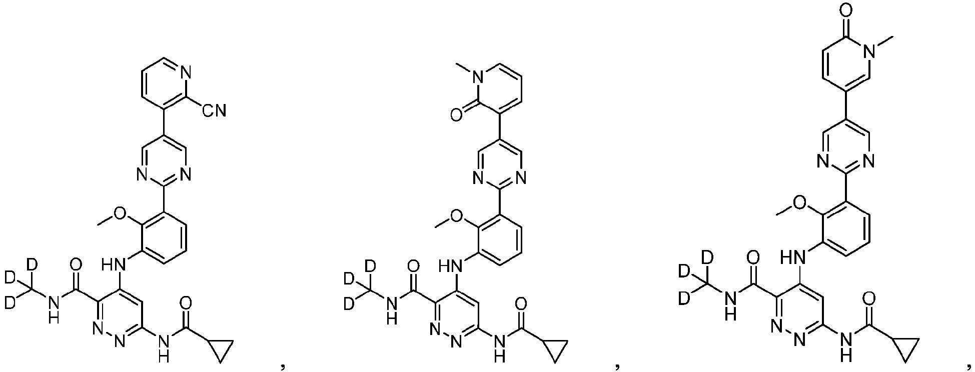

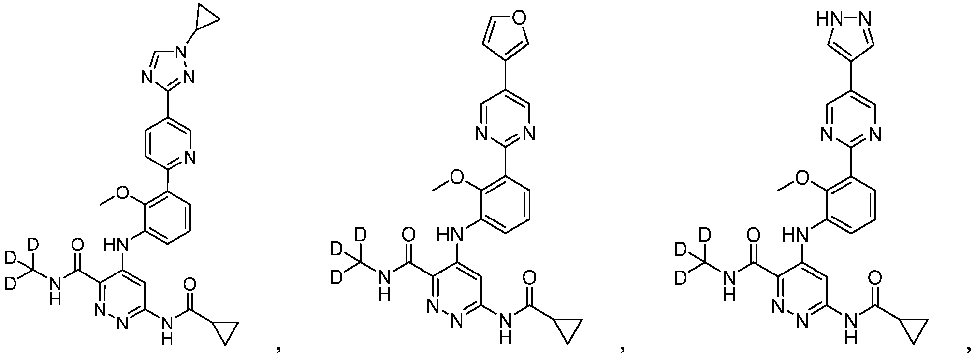

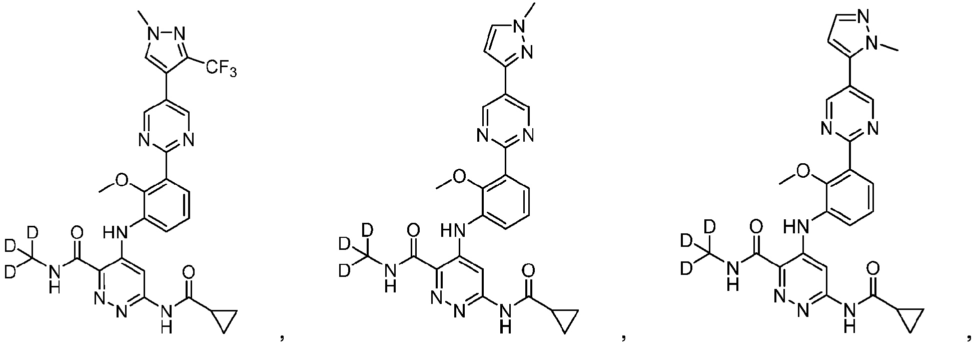

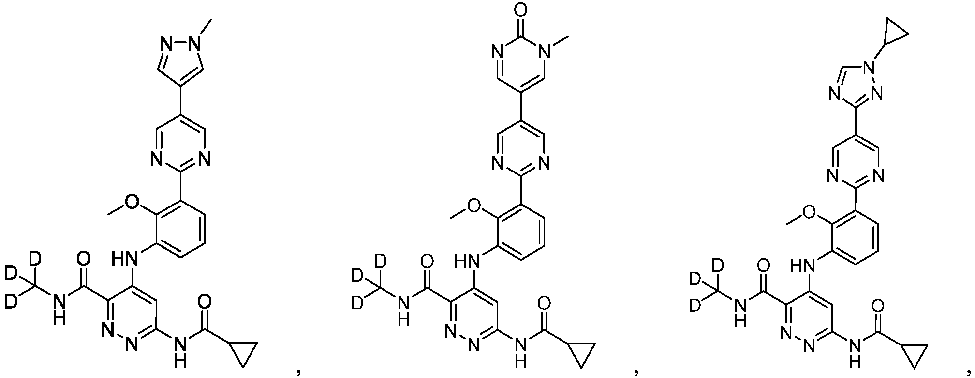

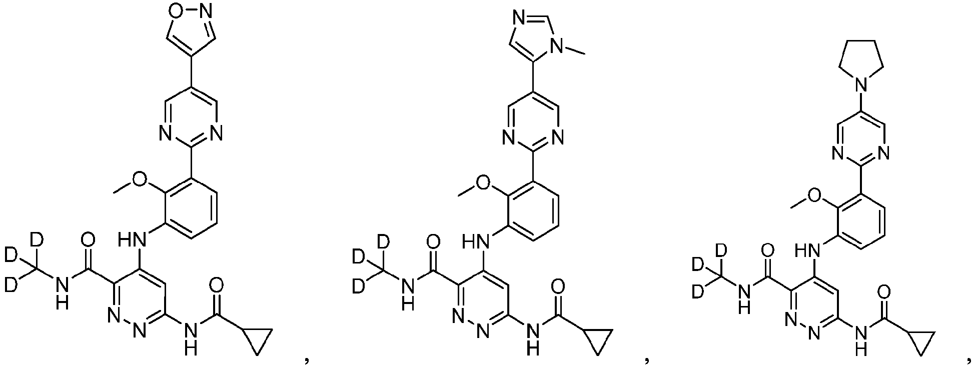

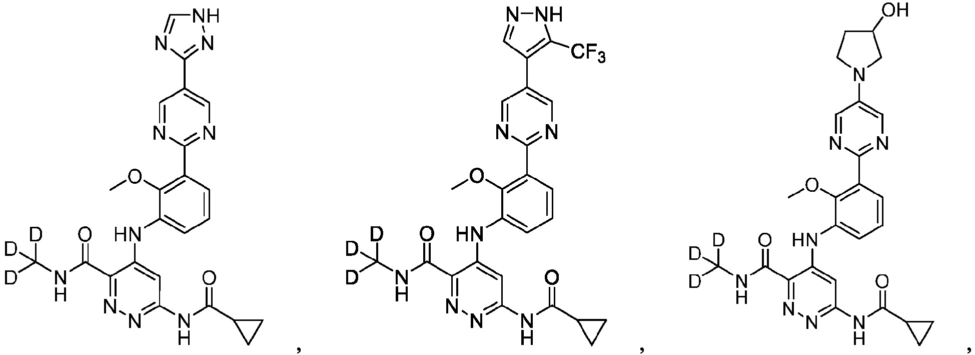

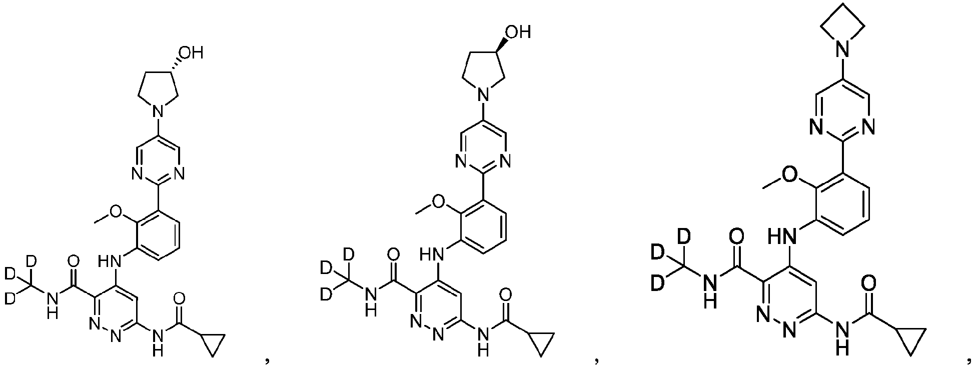

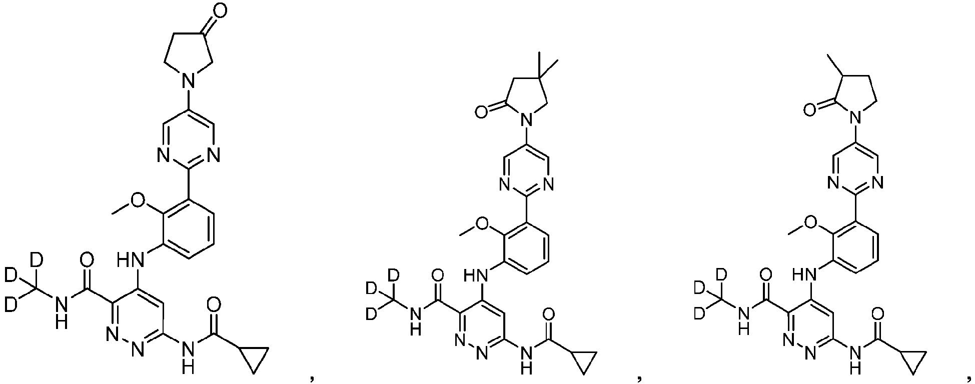

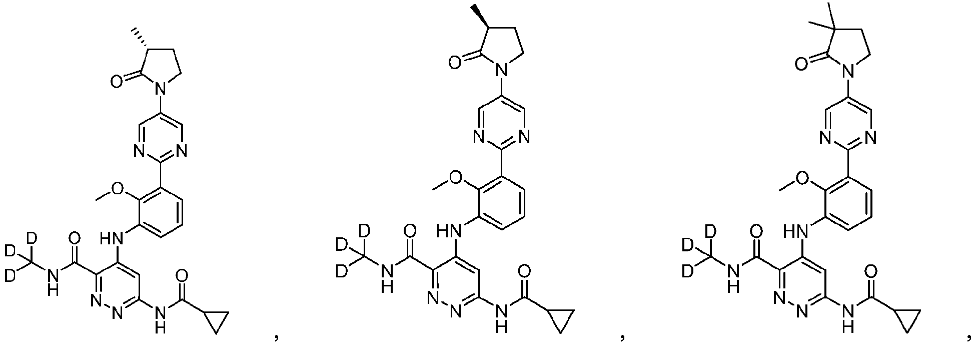

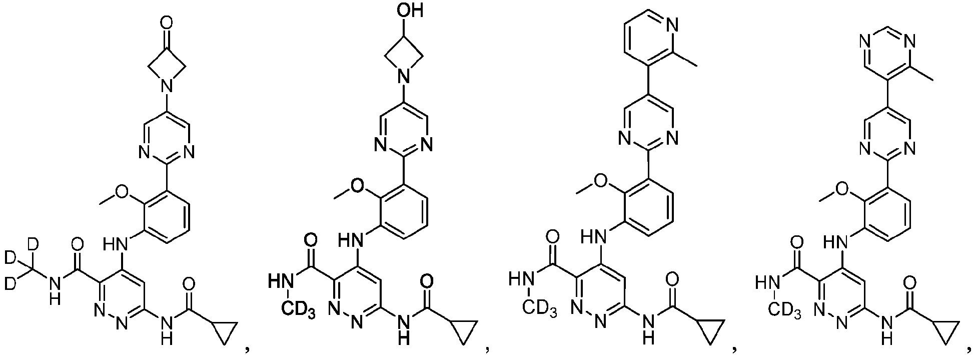

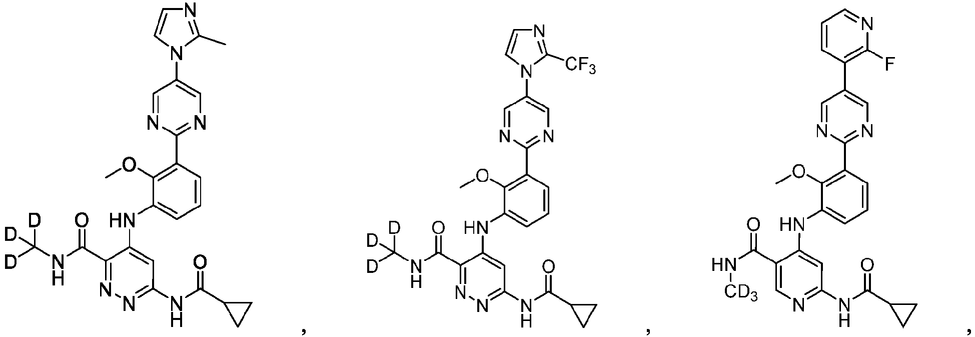

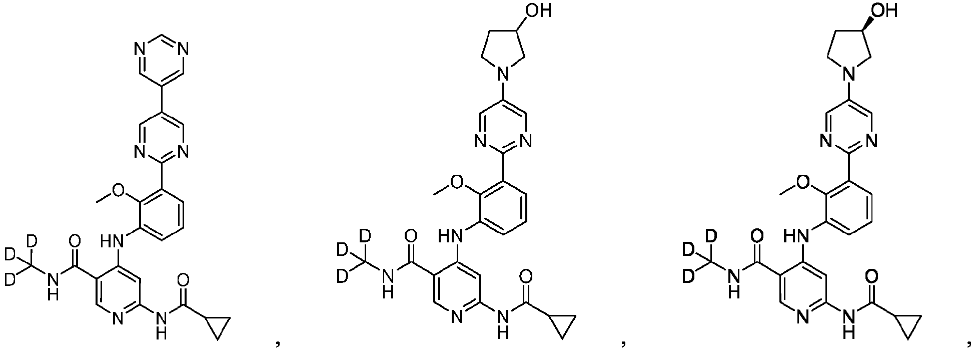

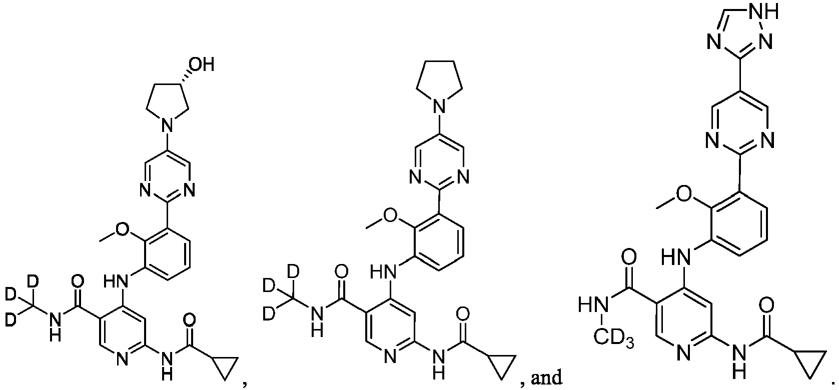

In another aspect, the present disclosure provides a compound of a formula selected from the group consisting of the following formulas or a pharmaceutically acceptable salt thereof: 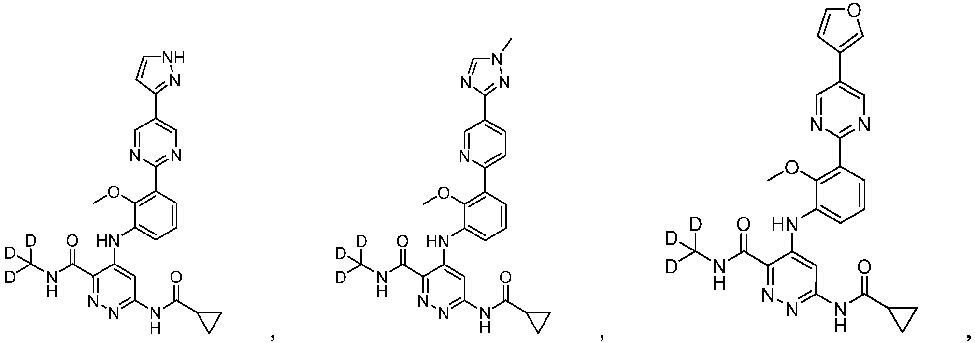 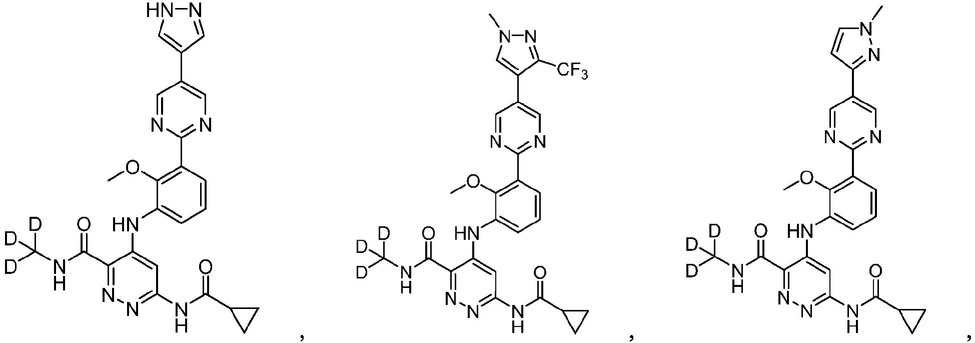 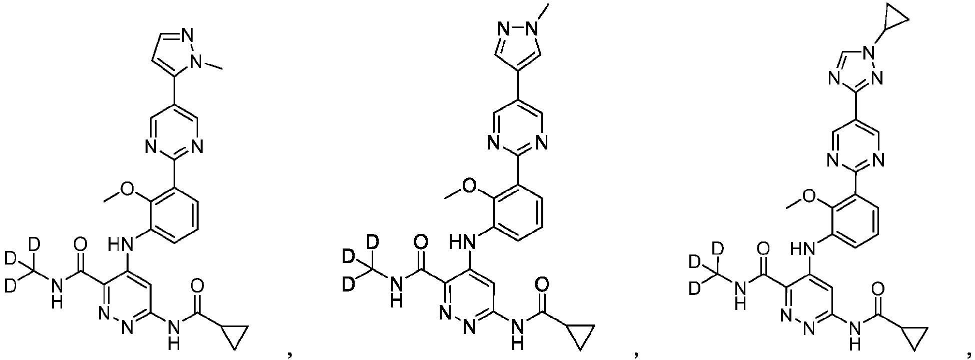 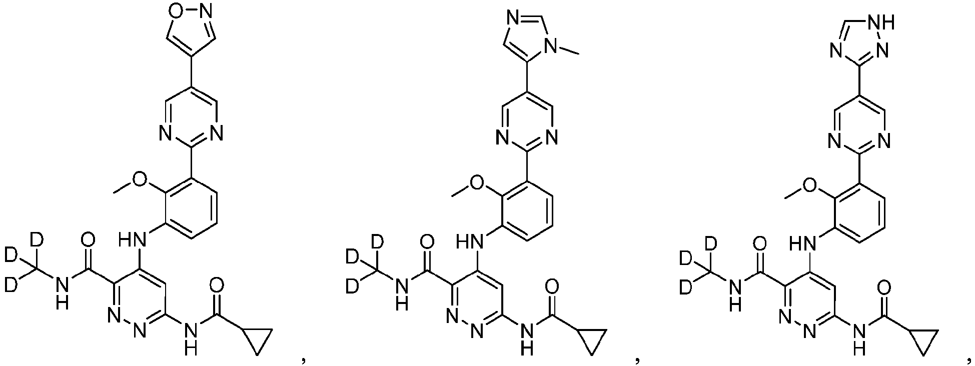 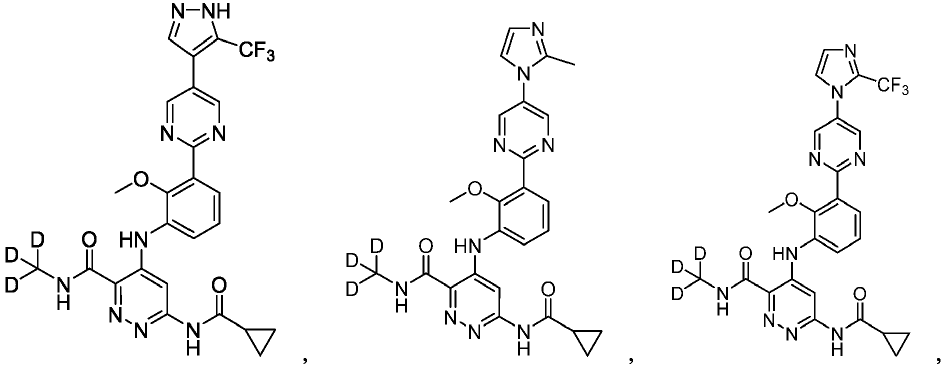 and

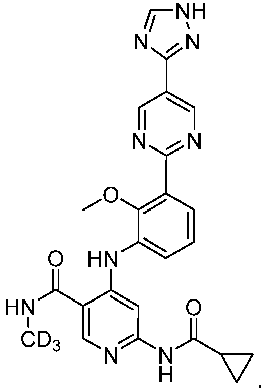

In another aspect, the present disclosure provides a compound of a formula selected from the group consisting of the following formulas or a pharmaceutically acceptable salt thereof:

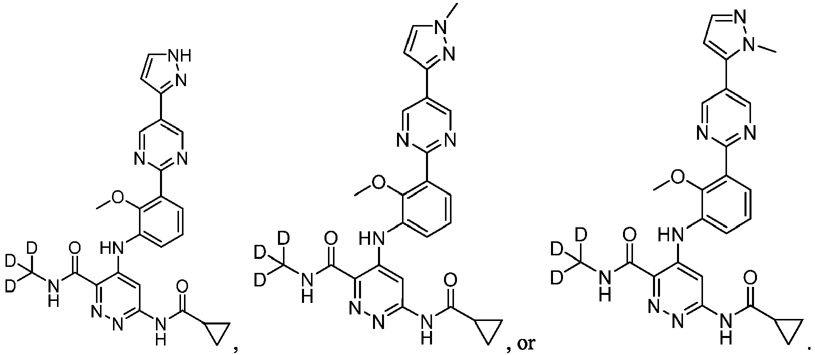

In another aspect, the present disclosure provides a compound of a formula selected from the group consisting of the following formulas or a pharmaceutically acceptable salt thereof:

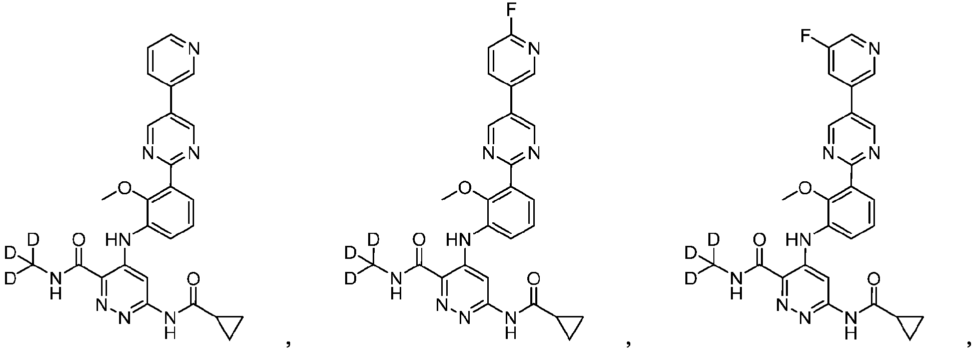

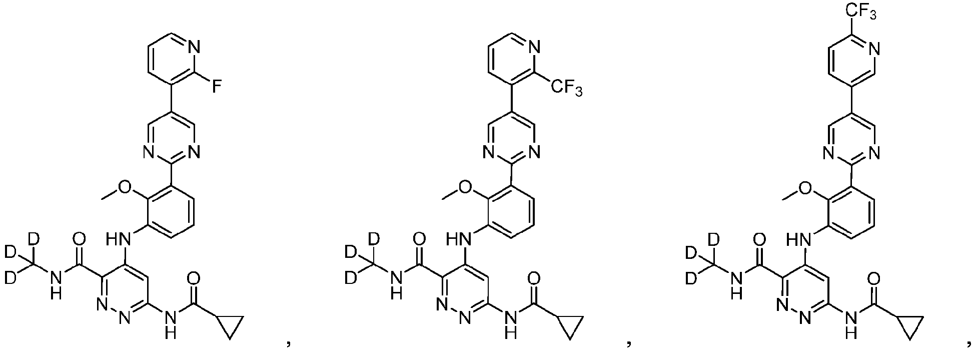

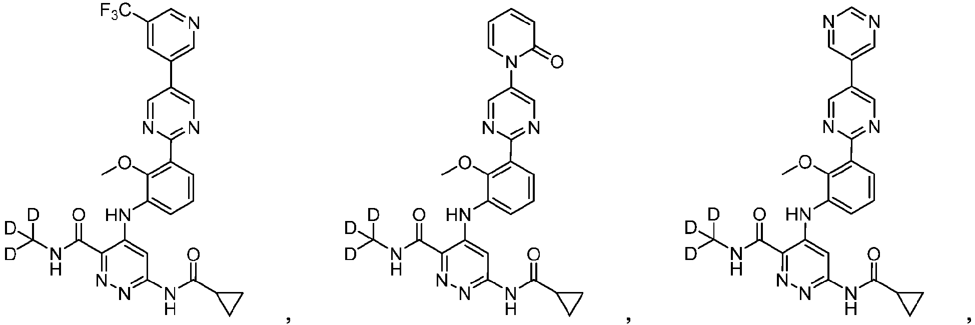

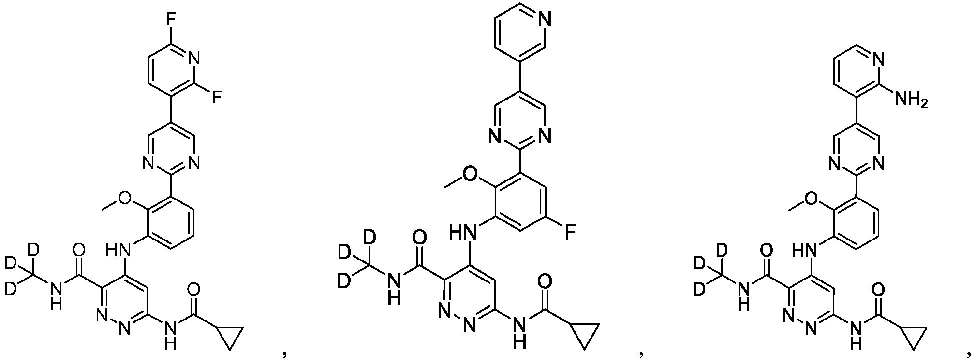

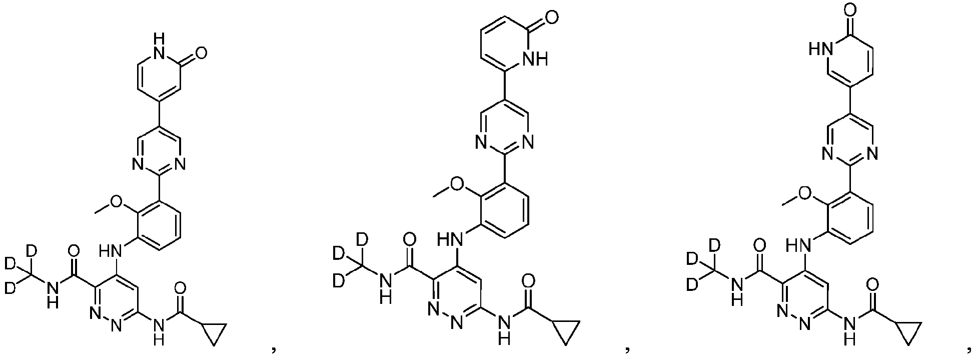

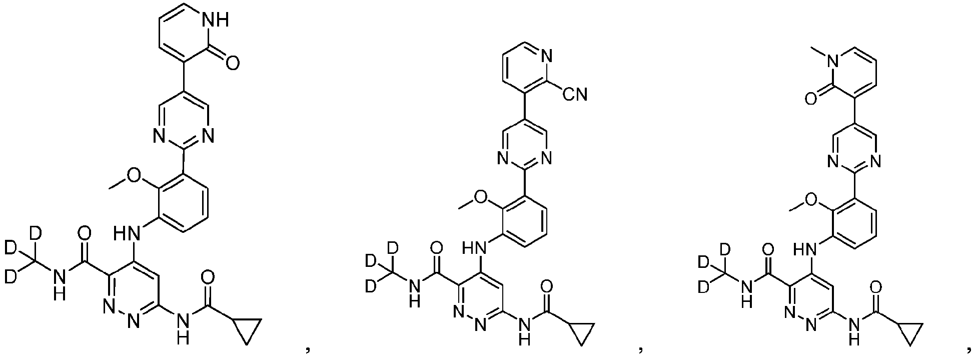

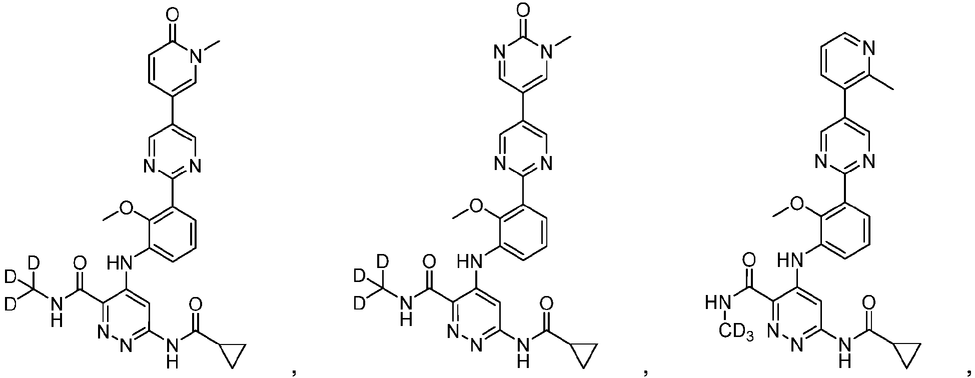

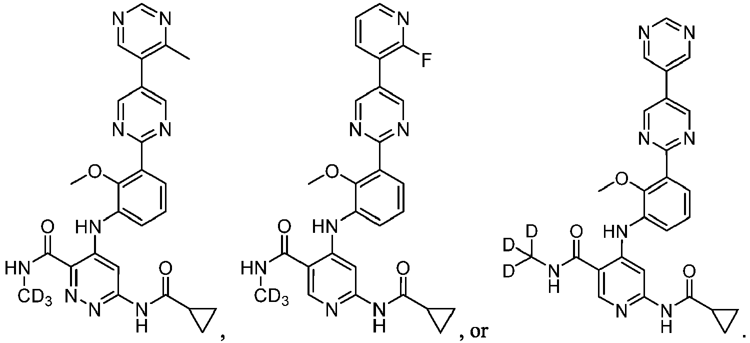

In another aspect, the present disclosure provides a compound of a formula selected from the group consisting of the following formulas or a pharmaceutically acceptable salt thereof:

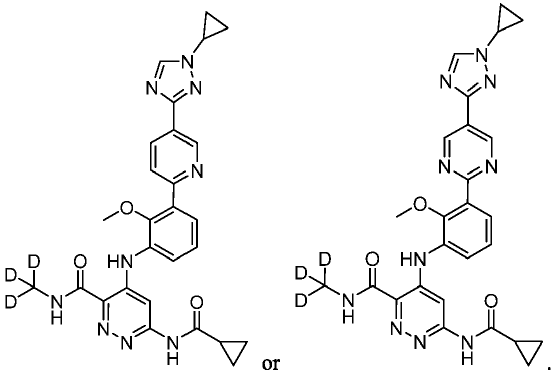

In another aspect, the present disclosure further provides a pharmaceutical composition comprising the compound or the pharmaceutically acceptable salt thereof described herein above. In some embodiments, the pharmaceutical composition disclosed herein further comprises a pharmaceutically acceptable excipient.

In another aspect, the present disclosure further provides a method for treating or preventing various TYK2-related diseases comprising administering to a mammal, preferably a human, in need of the treatment a therapeutically or prophylactically effective amount of the compound or the pharmaceutically acceptable salt thereof or the pharmaceutical composition thereof described herein above.

In another aspect, the present disclosure further provides use of the compound or the pharmaceutically acceptable salt thereof or the pharmaceutical composition thereof described herein above in preparing a medicament for use in treating or preventing various TYK2-related diseases.

In another aspect, the present disclosure further provides use of the compound or the pharmaceutically acceptable salt thereof or the pharmaceutical composition thereof described herein above in treating or preventing various TYK2-related diseases.

In another aspect, the present disclosure further provides the compound or the pharmaceutically acceptable salt thereof or the pharmaceutical composition thereof described herein above for use in treating or preventing various TYK2-related diseases.

In some embodiments, the various TYK2-related diseases are selected from inflammatory diseases and autoimmune diseases. In some embodiments, the various TYK2-related diseases are selected from colitis.

The compound disclosed herein has good *in vitro* enzymatic or cellular activity, metabolic stability such as hepatic microsomal metabolic stability, as well as pharmacokinetic properties and pharmacodynamic activities (e.g., in colitis models).

### Definitions

Unless otherwise stated, the following terms used in the present disclosure shall have the following meanings. A certain term, unless otherwise specifically defined, should not be considered uncertain or unclear, but construed according to its common meaning in the field. When referring to a trade name, it is intended to refer to its corresponding commercial product or its active ingredient.

When certain structural units or groups in the present disclosure have a covalent bond that is not connected to a specific atom, it means that the covalent bond can be connected to any atom within that structural unit or group, as long as it adheres to the rules of valence bonding.

The term "substituted" means that any one or more hydrogen atoms on a specific atom are substituted by substituents, as long as the valence of the specific atom is normal and the resulting compound is stable. When the substituent is oxo (namely =O), it means that two hydrogen atoms are substituted and oxo is not available on an aromatic group.

The terms "optional" or "optionally" means that the subsequently described event or circumstance may, but not necessarily, occur. The description includes instances where the event or circumstance occurs and instances where it does not. For example, ethyl being "optionally" substituted with halogen means that the ethyl may be unsubstituted (-CH₂CH₃), monosubstituted (for example, -CH₂CH₂F), polysubstituted (for example, -CHFCH₂F, -CH₂CHF₂, and the like), or fully substituted (-CF₂CF₃). It will be appreciated by those skilled in the art that for any groups comprising one or more substituents, any substitutions or substituting patterns which may not exist or can not be synthesized spatially are not introduced.

Cₘ₋ₙ used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms. For example, C₁₋₃ means that the group may have 1 carbon atom, 2 carbon atoms, or 3 carbon atoms.

When any variable (e.g., R) occurs more than once in the constitution or structure of a compound, the definition of the variable in each case is independent. Therefore, for example, if a group is substituted with 2 R, the definition of each R is independent.

When a connecting group has a number of 0, for example, -(CH₂)₀-, it means that the connecting group is a covalent bond.

When a substituent has a number of 0, such as -(R¹)₀, it means unsubstituted, for example, 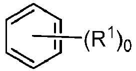 represents

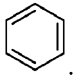

When one of the variables is selected from a covalent bond, it means that the two groups connected to it are directly linked. For example, in A-L'-Z, where L' represents a covalent bond, it means that the structure is actually A-Z.

When a bond of a substituent is crosslinked to two atoms on a ring, the substituent can be bonded to any atoms on the ring. For example, structural unit 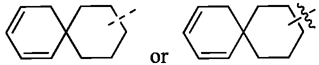 represents that substitution may occur at any one position of cyclohexyl or cyclohexadienyl.

The term "halo-" or "halogen" refers to fluorine, chlorine, bromine, and iodine.

The term "alkyl" refers to hydrocarbyl with a general formula of CₙH₂ₙ₊₁. The alkyl can be linear or branched. For example, the term "C₁₋₆ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). The alkyl moieties (namely alkyl) of alkoxy, alkylamino, dialkylamino, alkylsulfonyl, and alkylthio are similarly defined as above. As another example, the term "C₁₋₃ alkyl" refers to alkyl containing 1 to 3 carbon atoms (e.g., methyl, ethyl, propyl, and isopropyl).

The term "alkoxy" refers to -O-alkyl.

The term "cycloalkoxy" refers to -O-cycloalkyl.

The term "cycloalkyl" refers to a carbon ring that is fully saturated and may exist as a monocyclic, bridged cyclic, or spiro cyclic structure. Unless otherwise specified, the carbon ring is generally a 3- to 10-membered ring. Non-limiting examples of cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, dicyclo[1.1.1]pentan-1-yl, and the like. For example, C₃₋₄ cycloalkyl includes cyclopropyl and cyclobutyl.

The term "heterocyclyl" refers to a fully saturated or partially unsaturated (but not fully unsaturated heteroaromatic group) nonaromatic ring which may exist in the form of a monocyclic, bridged cyclic, or spiro cyclic structure, including, for example, heterocycloalkyl and heterocycloalkenyl. Unless otherwise specified, the heterocyclyl is usually a 3- to 10-membered or 3- to 7-membered ring containing 1 to 3 heteroatoms (preferably 1 or 2 heteroatoms) independently selected from the group consisting of sulfur, oxygen, and/or nitrogen. Non-limiting examples of heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, sulfomorpholinyl, tetrahydrothienyl, and the like.

The term "heterocycloalkyl" refers to a fully saturated cyclic group that may exist in the form of a monocyclic, bridged cyclic (including fused ring), or spiro cyclic structure. Unless otherwise specified, the heterocycloalkyl is usually a 3- to 10-membered, 3- to 7-membered, 3- to 6-membered, 4- to 6-membered, or 5- to 6-membered ring containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, thietanyl, oxetanyl, and azetidinyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiapyranyl, morpholinyl, piperazinyl, 1,4-oxathianyl, 1,4-dioxanyl, sulfomorpholinyl, 1,3-dithianyl, and 1,4-dithianyl. Examples of 7-membered heterocycloalkyl include, but are not limited to, azacycloheptanyl, oxacycloheptanyl, and thiocycloheptanyl. Preferably, the heterocycloalkyl is a monocyclic heterocycloalkyl having 5 or 6 ring atoms. The term "heterocycloalkenyl" refers to a nonaromatic cyclic group that is not fully saturated (but not fully unsaturated heteroaromatic group) and may exist in the form of a monocyclic, bridged cyclic, or spiro cyclic structure. Unless otherwise specified, the heterocyclyl generally contains 1 to 3 heteroatoms (preferably 1 or 2 heteroatoms) independently selected from the group consisting of sulfur, oxygen, and/or nitrogen. Unless otherwise indicated, the heterocycloalkenyl is generally a 3- to 10-membered ring or 4- to 8-membered ring. A specific example of the heterocycloalkenyl is

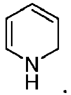

The term "aryl" refers to an aromatic monocyclic or fused polycyclic group of carbon atoms with the conjugated pi-electron system. For example, aryl may have 6-20 carbon atoms, 6-14 carbon atoms, or 6-12 carbon atoms. Non-limiting examples of aryl include, but are not limited to, phenyl, naphthyl, anthryl, 1,2,3,4-tetrahydronaphthalene, and the like.

The term "heteroaryl" refers to a monocyclic or fused polycyclic system which comprises at least one ring atom selected from the group consisting of N, O, and S, with the remaining ring atoms being C, and which has at least one aromatic ring. Preferably, heteroaryl has a single 5- to 8-membered or 5- to 6-membered ring, or is a plurality of fused rings comprising 6 to 14 ring atoms, in particular 6 to 10 ring atoms. Non-limiting examples of heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The compounds disclosed herein may exist in the form of a specific geometric isomer or stereoisomer. All such compounds are contemplated herein, including *cis* and *trans* isomers, (-)- and (+)-enantiomers, (R)- and (S)-enantiomers, diastereoisomers, (D)-isomers, (L)-isomers, and racemic mixtures and other mixtures thereof, such as an enantiomer or diastereomer enriched mixture, all of which are encompassed within the scope of the present disclosure. The substituents such as alkyl may have an additional asymmetric carbon atom. All these isomers and mixtures thereof are encompassed within the scope of the present disclosure.

Unless otherwise stated, the absolute configuration of a stereogenic center is represented by a wedged solid bond ( 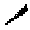 ) and a wedged dashed bond ( 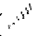 ), and the relative configuration of a stereogenic center is represented by a straight solid bond ( 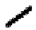 ) and a straight dashed bond ( 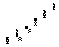 ). A wavy line ( 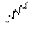 ) represents a wedged solid bond ( 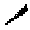 ) or a wedged dashed bond ( 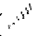 ), or a wavy line ( 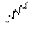 ) represents a straight solid bond ( 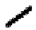 ) and a straight dashed bond ( 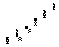 ).

Unless otherwise stated, when a double bond structure such as a carbon-carbon double bond, a carbon-nitrogen double bond, and a nitrogen-nitrogen double bond is present in the compound, and each atom on the double bond is linked to two different substituents (in the double bond including a nitrogen atom, a lone pair of electrons on the nitrogen atom is regarded as a substituent to which the nitrogen atom is linked), if the atom on the double bond of the compound and its substituents are linked using a wavy line ( 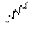 ), it means that the compound exists in the form of a (Z)-type isomer, an (E)-type isomer, or a mixture of the two isomers.

The term "treat", "treating", or "treatment" refers to administering the compound or the pharmaceutically acceptable salt thereof or the pharmaceutical composition thereof or formulation described herein to ameliorate or eliminate a disease or one or more symptoms associated with the disease, and includes:
(i) inhibiting a disease or disease state, i.e., arresting its development; and
(ii) alleviating a disease or disease state, i.e., causing its regression.

The term "prevent", "preventing", or "prevention" refers to administering the compound or the pharmaceutically acceptable salt thereof or the pharmaceutical composition thereof or formulation described herein so as to prevent a disease or one or more symptoms associated with the disease, and includes: preventing the occurrence of the disease or disease state in a mammal, particularly when such a mammal is predisposed to the disease state but has not yet been diagnosed as having it.

The term "therapeutically or prophylactically effective amount" refers to an amount of the compound disclosed herein for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound disclosed herein composing the "therapeutically effective amount" varies dependently on the compound, the condition and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the pharmaceutically acceptable salts thereof disclosed herein and a pharmaceutically acceptable excipient. The pharmaceutical composition is intended to facilitate the administration of the compound disclosed herein to an organism.

The term "pharmaceutically acceptable excipient" refers to those which do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The word "comprise" and variations thereof such as "comprises" or "comprising" will be understood in an open, non-exclusive sense, i.e., "including but not limited to".

The compounds and intermediates disclosed herein may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerism. A specific example of a proton tautomer is an imidazole moiety where a proton can transfer between two ring nitrogens. A valence tautomer includes the interconversion via recombination of some bonding electrons.

The present disclosure also comprises isotopically labeled compounds which are identical to those recited herein but have one or more atoms replaced by an atom having an atomic mass or mass number different from the atomic mass or mass number usually found in nature. Examples of isotopes that can be incorporated into the compounds disclosed herein include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds disclosed herein (e.g., those labeled with ³H and ¹⁴C) can be used to analyze compounds and/or substrate tissue distribution. Tritium (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F can be used in positron emission tomography (PET) studies to determine substrate occupancy. Isotopically labeled compounds disclosed herein can generally be prepared by following procedures analogous to those disclosed in the schemes and/or examples below while substituting a non-isotopically labeled reagent with an isotopically-labeled reagent.

Furthermore, substitution with heavier isotopes such as deuterium (i.e., ²H) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium, and all such forms of the compounds are included within the scope of the present disclosure.

The compounds disclosed herein can be asymmetrical, for example, has one or more stereoisomers. Unless otherwise stated, all stereoisomers are included, for example, enantiomers and diastereoisomers. The compounds with asymmetric carbon atoms disclosed herein can be isolated in an optically pure form or in a racemic form. The optically pure form can be isolated from a racemic mixture or can be synthesized using a chiral starting material or a chiral reagent.

The pharmaceutical composition disclosed herein can be prepared by combining the compound or the pharmaceutically acceptable salt thereof disclosed herein with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof disclosed herein include, but are not limited to, oral, rectal, topical, inhalational, parenteral, sublingual, intravaginal, intranasal, intraocular, intraperitoneal, intramuscular, subcutaneous, and intravenous administration.

The pharmaceutical composition disclosed herein can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound or the pharmaceutically acceptable salt thereof disclosed herein to be formulated into tablets, pills, pastilles, dragees, capsules, liquids, gels, slurries, suspensions, and the like for oral administration to a patient.

A solid oral composition can be prepared by conventional mixing, filling, or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents, and the like.

The pharmaceutical composition may also be suitable for parenteral administration, such as a sterile solution, a suspension, or a lyophilized product in a suitable unit dosage form.

Therapeutic dosages of the compound disclosed herein may be determined by, for example, the specific use of a treatment, the route of administration of the compound, the health and condition of a patient, and the judgment of a prescribing physician. The proportion or concentration of the compound disclosed herein in a pharmaceutical composition may not be constant and depends on a variety of factors including dosages, chemical properties (e.g., hydrophobicity), and routes of administration. For example, the compound disclosed herein may be provided for parenteral administration by a physiological buffered aqueous solution containing about 0.1-10% w/v of the compound. Certain typical dosages range from about 1 µg/kg body weight/day to about 1 g/kg body weight/day. In certain embodiments, the dosage ranges from about 0.01 mg/kg body weight/day to about 100 mg/kg body weight/day. The dosage is likely to depend on such variables as the type and degree of progression of the disease or disorder, the general health condition of a particular patient, the relative biological potency of the compound selected, the excipient formulation, and its route of administration. Effective doses can be extrapolated from dose-response curves derived from *in vitro* or animal model test systems.

The compounds disclosed herein can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

The chemical reactions of the embodiments disclosed herein are conducted in a proper solvent that must be suitable for the chemical changes in the present disclosure and the reagents and materials required. In order to acquire the compounds disclosed herein, it is sometimes necessary for those skilled in the art to modify or select a synthesis procedure or a reaction process based on the existing embodiments.

An important consideration in synthesis route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

In some embodiments, the compounds disclosed herein can be prepared by those skilled in the art of organic synthesis by reference to the following routes: 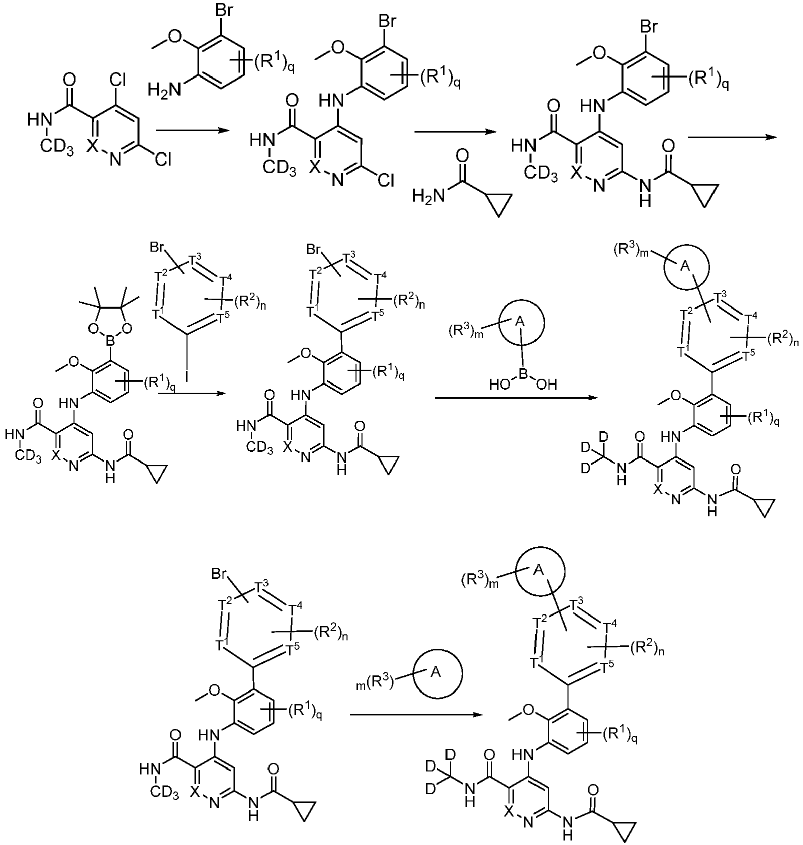 wherein X, R¹, q, R², n, T¹, T², T³, T⁴, T⁵, ring A, m, or R³ is defined as above.

The following abbreviations are used in the present disclosure:
DMF represents N,N-dimethylformamide; DIPEA represents diisopropylethylamine; LiHMDS represents lithium bis(trimethylsilyl)amide; Pd(dba)₂ represents bis(dibenzylideneacetone)palladium(0); Pd₂(dba)₃ represents tris(dibenzylideneacetone)dipalladium(0); Xantphos represents 4,5-bis(diphenylphosphino)-9,9-dimethylxanthene; Pd(dppf)Cl₂ represents 1,1'-bis(diphenylphosphino)ferrocenepalladium dichloride; DMSO represents dimethyl sulfoxide.

For clarity, the present disclosure is further described with the following examples, which are, however, not intended to limit the scope of the present disclosure. All the reagents used in the present disclosure are commercially available and can be used without further purification.

All patents, patent applications, and other identified publications are explicitly incorporated herein by reference for the purpose of description and disclosure. Any reference to these publications herein shall not be construed as an admission that the publications form part of the commonly recognized knowledge in the art.

### DETAILED DESCRIPTION

### Example 1: Compound 1-j

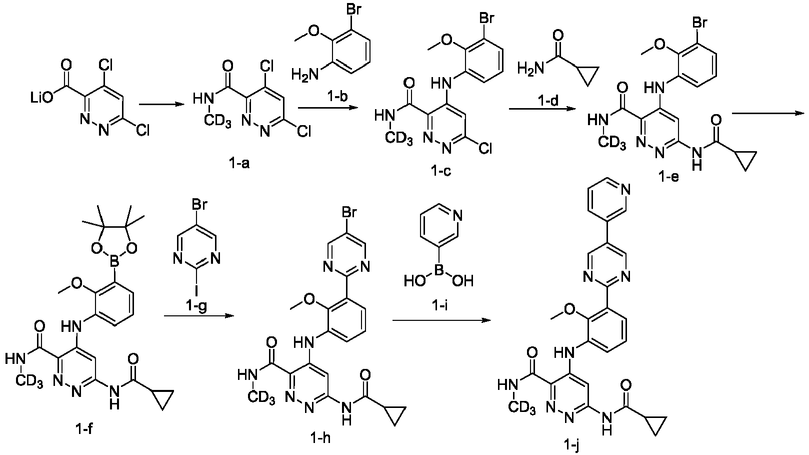

### 1) Preparation method for compound 1-a:

Lithium 4,6-dichloropyridazine-3-carboxylate (50 g) was dissolved in dichloromethane (500 mL), and after a catalytic amount of DMF was added at 0 °C, oxalyl chloride (96 g) was slowly added dropwise into the solution. After the completion of the dropwise addition, the mixture was stirred and reacted at room temperature for another 2 h to complete the reaction. The reaction solution was concentrated. Methyl-d3-amine hydrochloride (18.6 g) and DIPEA (162 g) were dissolved in dichloromethane (200 mL) and stirred for complete dissolution at -25 °C. The concentrate was dissolved in 300 mL of dichloromethane and slowly added dropwise into the above solution. After the completion of the dropwise addition, the mixture was stirred for another 2 h. The reaction was quenched by addition of water (750 mL), and the mixture was extracted twice with dichloromethane (2 × 500 mL). The organic phases were combined, washed with saturated aqueous sodium chloride solution, dried over anhydrous Na₂SO₄, and filtered. The filtrate was concentrated to give compound 1-a (52 g). ESI-MS: m/z = 209.2 [M+H]⁺.

### 2) Preparation method for compound 1-c:

Compound 1-a (2 g) and compound 1-b (2.3 g) were dissolved in tetrahydrofuran (40 mL), and a 1 M LiHMDS tetrahydrofuran solution (48 mL) was added dropwise at -10 °C. After the completion of the dropwise addition, the mixture was stirred overnight at room temperature. Purified water and ethyl acetate were added. The resulting mixture was stirred and the phases were separated. The organic phases were washed with brine, dried, filtered, and subjected to rotary evaporation to give compound 1-c (3.5 g). ESI-MS: m/z = 373.90 [M+H]⁺.

### 3) Preparation method for compound 1-e:

Compound 1-c (1.8 g), compound 1-d (0.41 g), Pd₂(dba)₃ (0.88 g), Xantphos (0.56 g), and cesium carbonate (3.13 g) were dissolved successively in 50 mL of dioxane, and the mixture was heated to 105 °C and reacted for 24 h under nitrogen atmosphere. The reaction solution was concentrated and purified by column chromatography to give compound 1-e (1.6 g). ESI-MS: m/z = 422.97 [M+H]⁺.

### 4) Preparation method for compound 1-f:

Compound 1-e (1.6 g), bis(pinacolato)diboron (4.8 g), Pd(dppf)Cl₂ (1.38 g), and potassium acetate (1.12 g) were dissolved successively in dioxane (50 mL), and the mixture was heated to 100 °C and reacted for 5 h under nitrogen atmosphere. The reaction solution was filtered and the filtrate was concentrated into an oil. Methanol and n-heptane were added. The resulting mixture was stirred and the phases were separated. The methanol phase was subjected to rotary evaporation to give compound 1-f (1.3 g). ESI-MS: m/z = 471.09 [M+H]⁺.

### 5) Preparation method for compound 1-h:

Compound 1-f (1 g), compound 1-g (0.67 g), Pd(dppf)Cl₂ (0.31 g), and potassium carbonate (0.59 g) were dissolved successively in dioxane (50 mL) and water (10 mL), and the mixture was heated to 80 °C and reacted for 8 h under nitrogen atmosphere. The reaction solution was concentrated and purified by column chromatography to give compound **1-h** (0.3 g). ESI-MS: m/z = 501.08 [M+H]⁺.

### 6) Preparation method for compound 1-j:

Compound 1-h (70 mg), compound 1-i (17 mg), Pd(dppf)Cl₂ (10 mg), and potassium carbonate (38 mg) were dissolved successively in dioxane (6 mL) and 1mL of water, and the mixture was heated to 110 °C and reacted for 8 h under nitrogen atmosphere. The reaction solution was concentrated and subjected to purification and separation to give compound 1-j (0.3 g). ESI-MS: m/z = 500.14 [M+H]⁺.

### Example 2: Compound 2-h

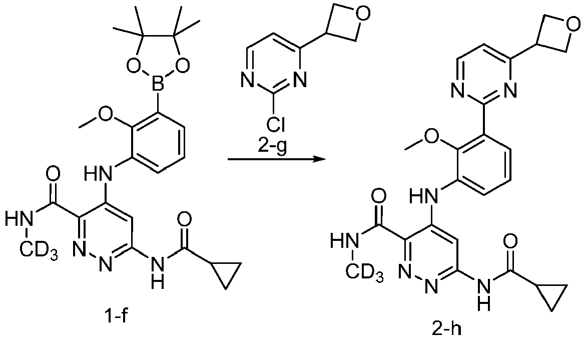

Compound **2-h** was obtained by reference to the preparation method for compound **1-h** in step 5) of Example 1, with compound 1-g being replaced by compound 2-g. ESI-MS: m/z = 479.10 [M+H]⁺.

### Example 3: Compound 3-j

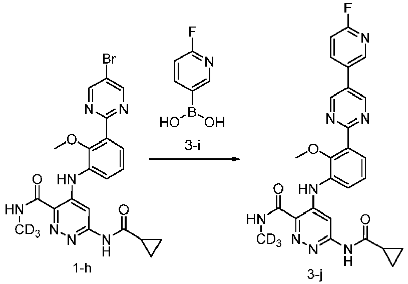

Compound **3-j** was obtained by reference to the preparation method for compound **1-j** in step 6) of Example 1, with compound 1-i being replaced by compound 3-i. ESI-MS: m/z = 518.10 [M+H]⁺.

### Example 4: Compound 4-j

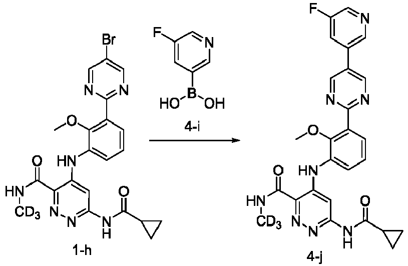

Compound **4-j** was obtained by reference to the preparation method for compound **1-j** in step 6) of Example 1, with compound 1-i being replaced by compound 4-i. ESI-MS: m/z = 518.09 [M+H]⁺.

### Example 5: Compound 5-j

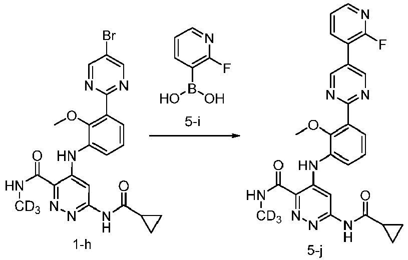

Compound **5-j** was obtained by reference to the preparation method for compound **1-j** in step 6) of Example 1, with compound 1-i being replaced by compound 5-i. ESI-MS: m/z = 518.10 [M+H]⁺.

### Example 6: Compound 6-j

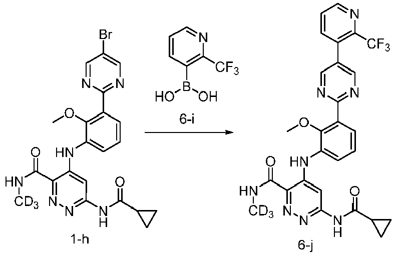

Compound **6-j** was obtained by reference to the preparation method for compound **1-j** in step 6) of Example 1, with compound 1-i being replaced by compound 6-i. ESI-MS: m/z = 568.09 [M+H]⁺.

### Example 7: Compound 7-j

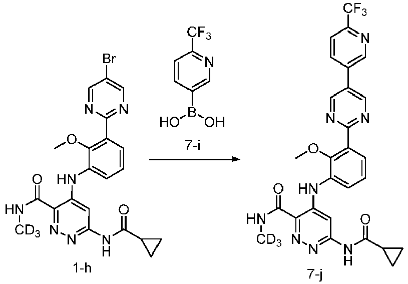

Compound **7-j** was obtained by reference to the preparation method for compound **1-j** in step 6) of Example 1, with compound 1-i being replaced by compound 7-i. ESI-MS: m/z = 568.11 [M+H]⁺.

### Example 8: Compound 8-j

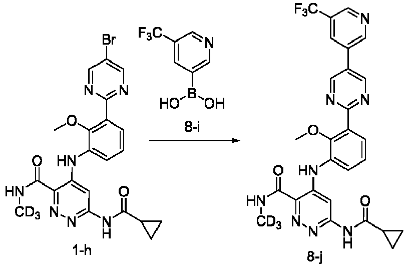

Compound **8-j** was obtained by reference to the preparation method for compound **1-j** in step 6) of Example 1, with compound 1-i being replaced by compound 8-i. ESI-MS: m/z = 568.10 [M+H]⁺.

### Example 9: Compound 9-j

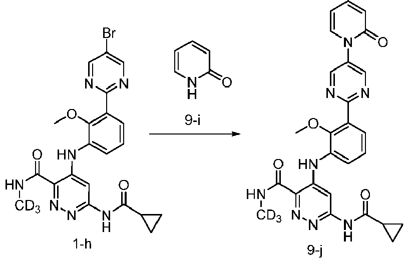

Compound **9-j** was obtained by reference to the preparation method for compound **1-j** in step 6) of Example 1, with compound 1-i being replaced by compound 9-i. ESI-MS: m/z = 516.06 [M+H]⁺.

### Example 10: Compound 10-j

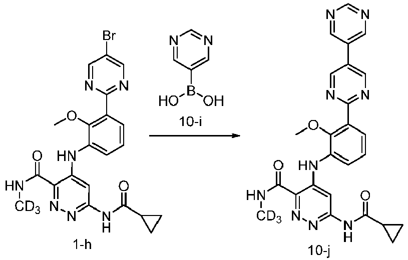

Compound **10-j** was obtained by reference to the preparation method for compound **1-j** in step 6) of Example 1, with compound 1-i being replaced by compound 10-i. ESI-MS: m/z = 501.13 [M+H]⁺.

### Example 11: Compound 11-h

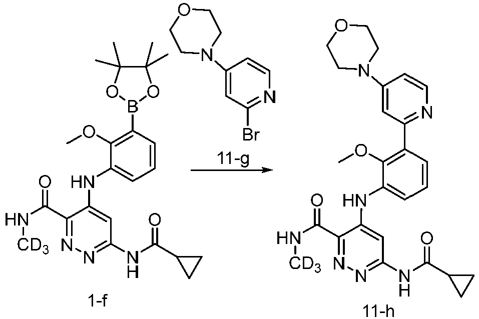

Compound **11-h** was obtained by reference to the preparation method for compound **1-h** in step 5) of Example 1, with compound 1-g being replaced by compound 11-g. ESI-MS: m/z = 507.08 [M+H]⁺.

### Example 12: Compound 12-j

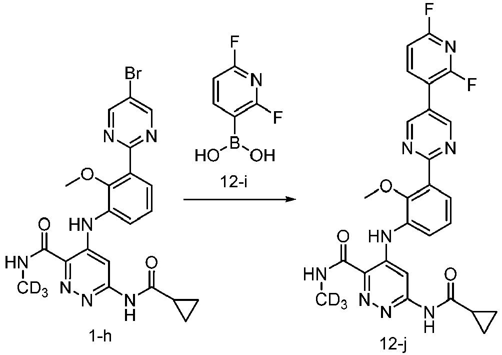

Compound **12-j** was obtained by reference to the preparation method for compound **1-j** in step 6) of Example 1, with compound 1-i being replaced by compound 12-i. ESI-MS: m/z = 536.04 [M+H]⁺.

**Examples 13-41:** Target compounds were obtained by reference to the preparation method for compound **2-h** of Example 2, with compound 2-g being replaced by compounds of the starting materials shown in the table below.

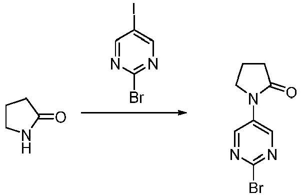

2-Pyrrolidone (1.0 g), 5-iodo-2-bromopyrimidine (1.7 g), Pd₂(dba)₃ (1.88 g), Xantphos (1.1 g), and cesium carbonate (11 g) were dissolved successively in 50 mL of dioxane, and the mixture was heated to 105 °C and reacted for 0.5 h under nitrogen atmosphere. The reaction solution was concentrated and subjected to separation and purification to give a compound (60 mg). ESI-MS: m/z = 242.08 [M+H]⁺.

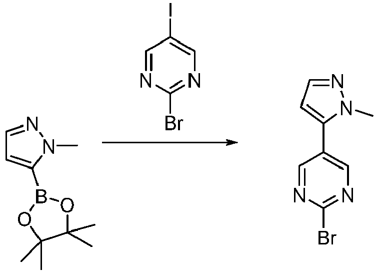

1-Methyl-1*H*-pyrazole-5-boronic acid pinacol ester (2.0g), 5-iodo-2-bromopyrimidine (2.8 g), Pd(dppf)Cl₂ (1.4 g), and potassium carbonate (4.1 g) were dissolved successively in dioxane (30 mL), and the mixture was heated to 100 °C and reacted for 3 h under nitrogen atmosphere. The reaction solution was filtered, and the filtrate was concentrated into an oil. The oil was subjected to column chromatography to give a product (0.6 g). ESI-MS: m/z = 239.11 [M+H]⁺.

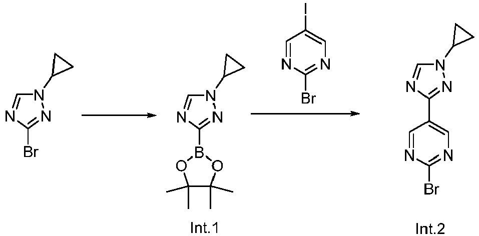

### Step 1

3-Bromo-1-cyclopropyl-1*H*-1,2,4-triazole (5.0 g), bis(pinacolato)diboron (10.2 g), Pd(dppf)Cl₂ (1.9 g), and potassium acetate (7.8 g) were dissolved successively in dioxane (50 mL), and the mixture was heated to 80 °C and reacted for 3 h under nitrogen atmosphere. The reaction solution was filtered, and the filtrate was concentrated into an oil. The oil was subjected to column chromatography to give Int.1 (1.2 g).

### Step 2

Int.1 (1.2 g), 5-iodo-2-bromopyrimidine (0.7 g), Pd(dppf)Cl₂ (0.7 g), and potassium carbonate (2.1 g) were dissolved successively in dioxane (20 mL), and the mixture was heated to 100 °C and reacted for 3 h under nitrogen atmosphere. The reaction solution was filtered, and the filtrate was concentrated into an oil. The oil was subjected to column chromatography to give Int.2 (0.2 g). ESI-MS: m/z = 266.23 [M+H]⁺.

| **Example** | **Starting material** | **Compound** | **ESI-MS: m/z [M+1]** | **Example** | **Starting material** | **Compound** | **ESI-MS: m/z [M+1]** |
|---|---|---|---|---|---|---|---|
| **13** | 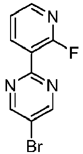 | 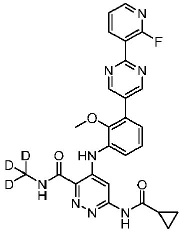 **13-h** | 518.36 | **14** | 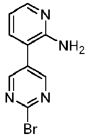 | 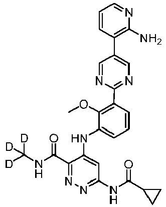 **14-h** | 515.36 |
| **15** | 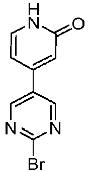 | 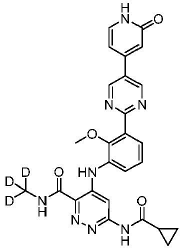 **15-h** | 516.30 | **16** | 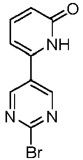 | 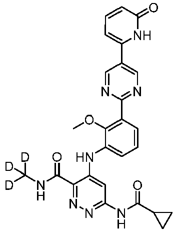 **16-h** | 516.32 |
| **17** | 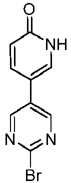 | 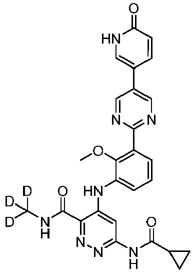 **17-h** | 516.30 | **18** | 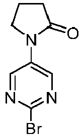 | 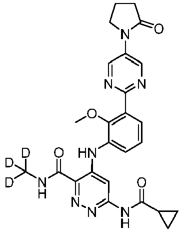 **18-h** | 506.31 |
| **19** | 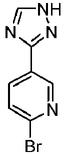 | 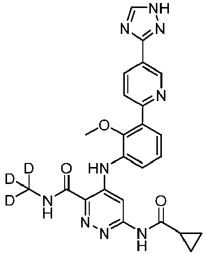 **19-h** | 489.40 | **20** | 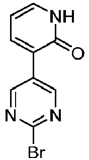 | 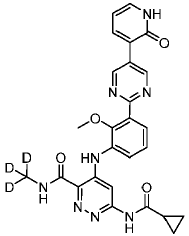 **20-h** | |
| **21** | 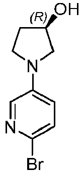 | 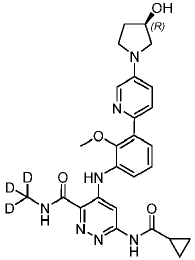 **21-h** | 507.34 | **22** | 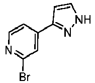 | 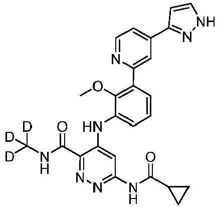 **22-h** | 488.39 |
| **23** | 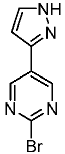 | 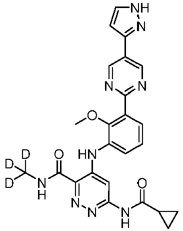 **23-h** | 489.37 | **24** | 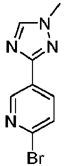 | 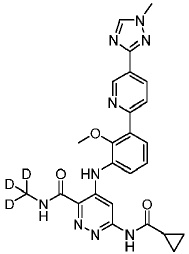 **24-h** | 503.38 |
| **25** | 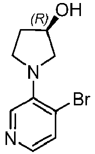 | 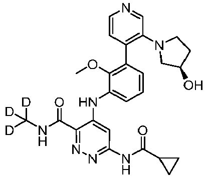 **25-h** | 507.56 | **26** | 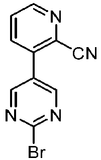 | 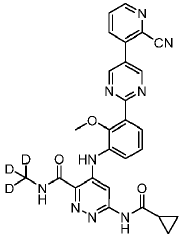 **26-h** | 525.22 |
| **27** | 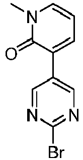 | 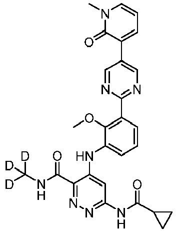 **27-h** | 530.24 | **28** | 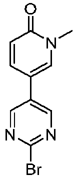 | 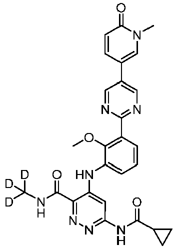 **28-h** | 530.41 |
| **29** | 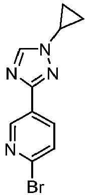 | 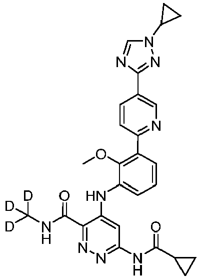 **29-h** | 529.42 | **30** | 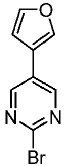 | 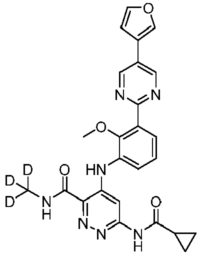 **30-h** | 489.41 |
| **31** | 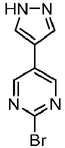 | 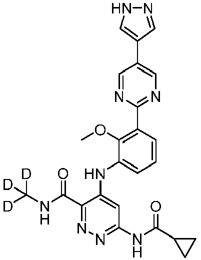 **31-h** | 489.35 | **32** | 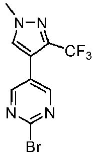 | 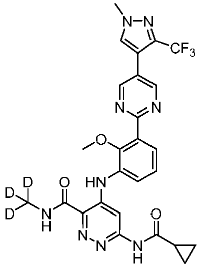 **32-h** | 571.38 |
| **33** | 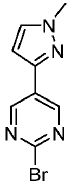 | 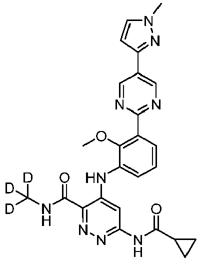 **33-h** | 503.34 | **34** | 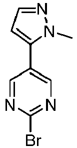 | 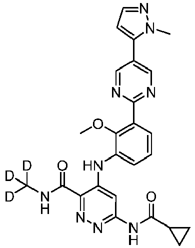 **34-h** | 503.39 |
| **35** | 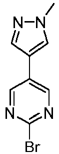 | 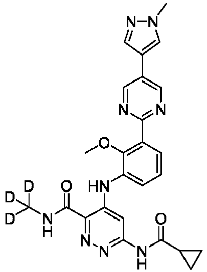 **35-h** | 503.34 | **36** | 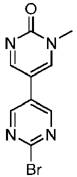 | 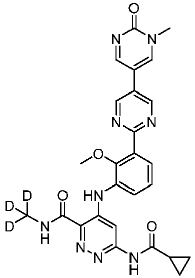 **36-h** | 531.31 |
| **37** | 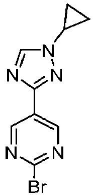 | 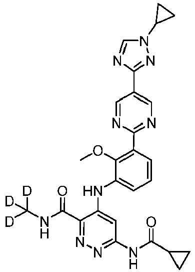 **37-h** | 530.49 | **38** | 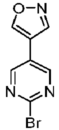 | 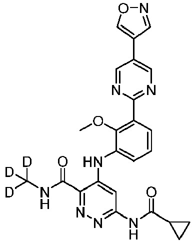 **38-h** | 490.33 |
| **39** | 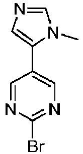 | 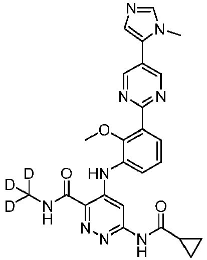 **39-h** | 503.48 | **40** | 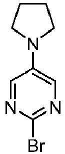 | 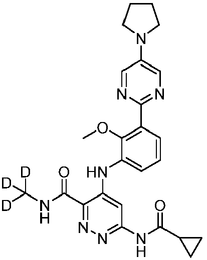 **40-h** | 492.28 |
| **41** | 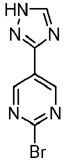 | 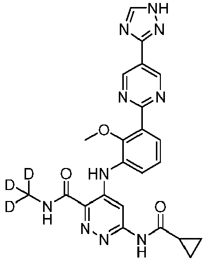 **41-h** | 490.40 | | | | |

**Examples 42-55:** Target compounds were obtained by reference to the preparation method for compound **1-j** of Example 1, with compound 1-i being replaced by compounds of the starting materials shown in the table below.

| **Example** | **Starting material** | **Compound** | **ESI-M S: m/z [M+1]** | **Example** | **Starting material** | **Compound** | **ESI-MS: m/z [M+1]** |
|---|---|---|---|---|---|---|---|
| **42** | 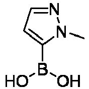 | 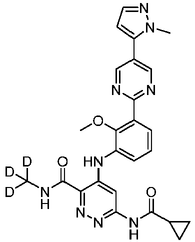 **34-h** | 503.39 | **43** | 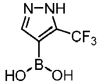 | 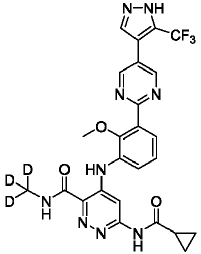 **43-j** | 557.34 |
| **44** | 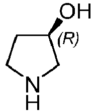 | 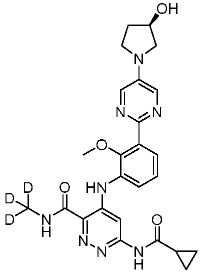 **44-j** | 508.40 | **45** | 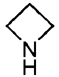 | 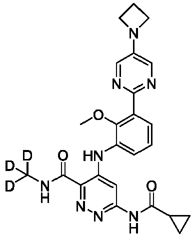 **45-j** | 478.40 |
| **46** | 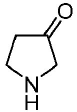 | 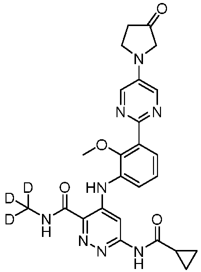 **46-j** | 506.36 | **47** | 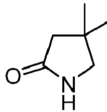 | 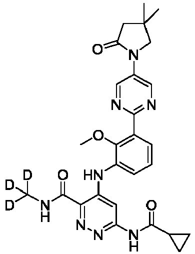 **47-j** | 534.46 |
| **48** | 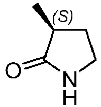 | 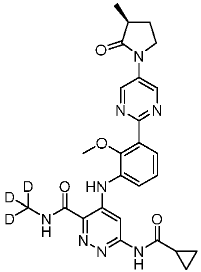 **48-j** | 520.43 | **49** | 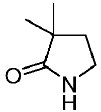 | 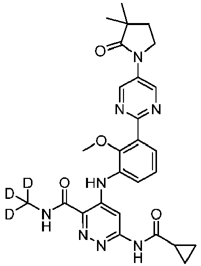 **49-j** | 534.46 |
| **50** | 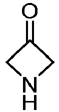 | 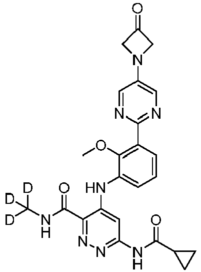 **50-j** | 492.40 | **51** | 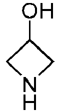 | 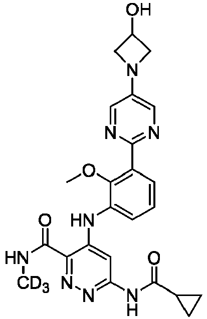 **51-j** | 494.41 |
| **52** | 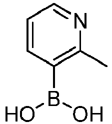 | 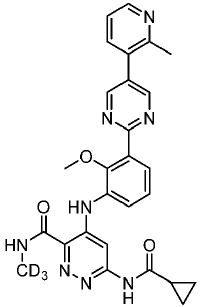 **52-j** | | **53** | 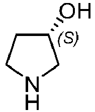 | 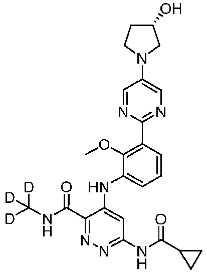 **53-j** | 508.40 |
| **54** | 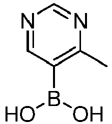 | 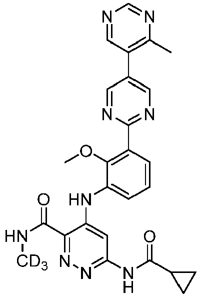 **54-j** | 515.43 | **55** | 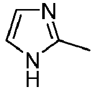 | 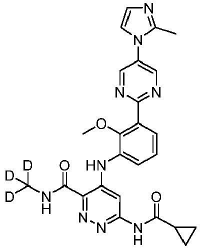 **55-j** | 503.68 |
| **56** | 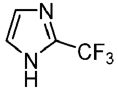 | 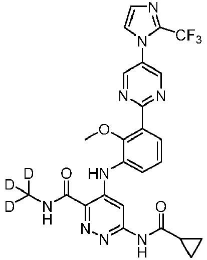 **56-j** | 557.38 | | | | |

### Example 57: Compound 57-j

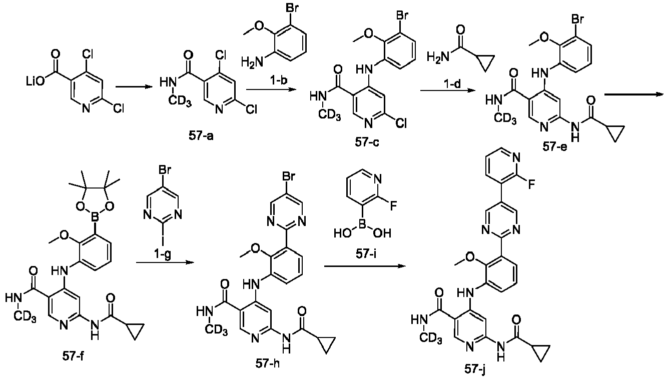

### 1) Preparation method for compound 57-a:

Compound **57-a** was obtained by reference to the preparation method for compound 1-a in step 1) of Example 1, with lithium 4,6-dichloropyridazine-3-carboxylate being replaced by lithium 4,6-dichloropyridine-3-carboxylate.

### 2) Preparation method for compound 57-c:

Compound 57-c was obtained by reference to the preparation method for compound 1-c in step 2) of Example 1, with compound 1-a being replaced by compound 57-a.

### 3) Preparation method for compound 57-e:

Compound 57-e was obtained by reference to the preparation method for compound 1-e in step 3) of Example 1, with compound 1-c being replaced by compound 57-c.

### 4) Preparation method for compound 57-f:

Compound 57-f was obtained by reference to the preparation method for compound 1-f in step 4) of Example 1, with compound 1-e being replaced by compound 57-e.

### 5) Preparation method for compound 57-h:

Compound **57-h** was obtained by reference to the preparation method for compound **1-h** in step 5) of Example 1, with compound 1-e being replaced by compound 57-f.

### 6) Preparation method for compound 57-j:

Compound **57-j** was obtained by reference to the preparation method for compound **1-j** in step 6) of Example 1, with compound 1-h being replaced by compound 57-h and compound 1-i being replaced by compound 57-i. ESI-MS: m/z = 517.46 [M+H]⁺.

**Examples 58-60:** The following compounds were prepared by reference to the preparation method in Example 57, with compound 57-i being replaced by compounds of the starting materials.

| **Example** | **Starting material** | **Compound** | **ESI-MS: m/z [M+1]** | **Example** | **Starting material** | **Compound** | **ESI-MS: m/z [M+1]** |
|---|---|---|---|---|---|---|---|
| **58** | 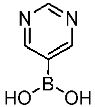 | 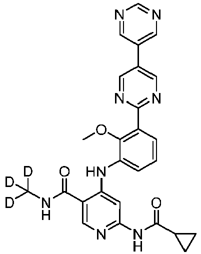 **58-j** | 500.45 | **59** | 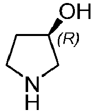 | 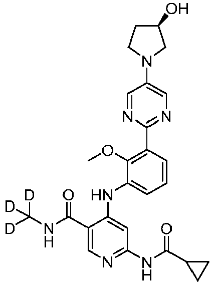 **59-j** | 507.35 |
| **60** | 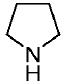 | 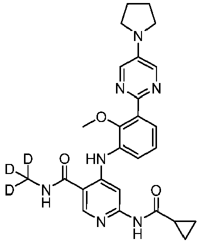 **60-j** | 491.36 | | | | |

### Example 61: Compound 61-h

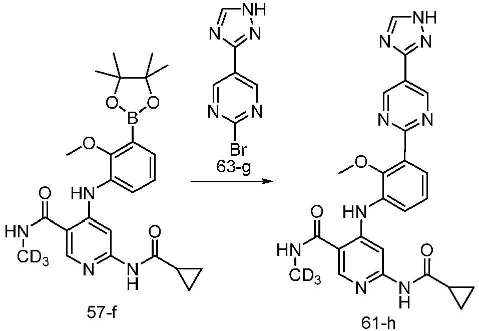

Compound **61-h** was obtained by reference to the preparation method for compound **1-h** in step 5) of Example 1, with compound 1-g being replaced by compound 63-g and compound 1-f being replaced by compound 57-f. ESI-MS: m/z = 488.21 [M+H]⁺.

| **Compound** | **Proton/Carbon NMR data** |
|---|---|
| **1-j** | ¹H NMR (500 MHz, DMSO-*d*₆) δ 11.35 (s, 1H), 10.99 (s, 1H), 9.37 (s, 2H), 9.14 (d, *J* = 17.8 Hz, 2H), 8.70 (d, *J* = 4.8 Hz, 1H), 8.34 (d, *J* = 8.0 Hz, 1H), 8.19 (s, 1H), 7.74 - 7.50 (m, 3H), 7.35 (t, *J =* 7.8 Hz, 1H), 3.75 (s, 3H), 2.10 (p, *J* = 6.1 Hz, 1H), 0.84 (d, *J* = 6.2 Hz, 4H). |
| **2-h** | ¹H NMR (500 MHz, DMSO-*d*₆) δ 11.34 (s, 1H), 10.97 (s, 1H), 9.15 (s, 1H), 8.89 (d, *J* = 5.2 Hz, 1H), 8.17 (s, 1H), 7.60 (d, *J* = 7.9 Hz, 2H), 7.46 (d, *J* = 5.1 Hz, 1H), 7.33 (t, *J* = 7.9 Hz, 1H), 4.94 (dd*, J* = 8.5, 5.7 Hz, 2H), 4.88 - 4.79 (m, 2H), 4.48 (tt, *J* = 8.5, 6.7 Hz, 1H), 3.74 (s, 3H), 2.10 (m,1H), 0.90 - 0.76 (m, 4H). |
| **3-j** | ¹H NMR (500 MHz, DMSO-*d*₆) δ 11.34 (s, 1H), 10.98 (s, 1H), 9.36 (s, 2H), 9.15 (s, 1H), 8.81 (d, *J* = 2.6 Hz, 1H), 8.55 (td, *J* = 8.2, 2.7 Hz, 1H), 8.18 (s, 1H), 7.71 - 7.54 (m, 2H), 7.41 (dd, *J* = 8.5, 2.8 Hz, 1H), 7.35 (t, *J* = 7.9 Hz, 1H), 3.75 (s, 3H), 2.10 (dd, *J* = 12.4, 6.3 Hz, 1H), 0.83 (d, *J* = 6.2 Hz, 4H). |
| **4-j** | ¹H NMR (500 MHz, DMSO-*d*₆) δ 11.34 (s, 1H), 10.97 (s, 1H), 9.41 (s, 2H), 9.15 (s, 1H), 9.03 (s, 1H), 8.71 (s, 1H), 8.38 (d, *J* = 7.8 Hz, 1H), 8.17 (s, 1H), 7.70 - 7.57 (m, 2H), 7.34 (d, *J* = 7.8 Hz, 1H), 3.74 (s, 3H), 2.15 - 2.03 (m, 1H), 0.85 - 0.80 (m, 4H). |
| **5-j** | ¹H NMR (500 MHz, DMSO-*d*₆) δ 11.34 (s, 1H), 10.98 (s, 1H), 9.24 (s, 2H), 9.15 (s, 1H), 8.47 - 8.32 (m, 2H), 8.17 (s, 1H), 7.71 - 7.52 (m, 3H), 7.36 (d, *J* = 7.9 Hz, 1H), 3.75 (s, 3H), 2.10 (m, 1H), 0.87 (s, 4H). |
| **6-j** | ¹H-NMR(DMSO-*d₆*)*δ*: 11.34(1H, s), 10.99(1H, s), 9.15(1H, s), 9.03(2H, s),8.89(1H, d, *J*=4.0), 8.21(1H, m), 8.18(1H, s),7.91(1H, m), 7.66(2H, m), 7.36(1H, m), 3.73(3H, s), 2.10(1H, m), 0.84(4H, m). |
| **7-j** | ¹H NMR (500 MHz, DMSO-*d*₆) δ 11.34 (s, 1H), 10.98 (s, 1H), 9.45 (s, 2H), 9.32 (d, *J* = 2.2 Hz, 1H), 9.16 (s, 1H), 8.63 (dd, *J* = 8.1, 2.3 Hz, 1H), 8.18 (s, 1H), 8.11 (d, *J* = 8.2 Hz, 1H), 7.64 (td, *J* = 8.1, 1.6 Hz, 2H), 7.36 (t, *J* = 7.9 Hz, 1H), 3.76 (s, 3H), 2.14 - 2.01 (m, 1H), 0.93 - 0.73 (m, 4H). |
| **8-j** | ¹H NMR (500 MHz, DMSO-*d*₆) δ 11.34 (s, 1H), 10.98 (s, 1H), 9.47 (s, 2H), 9.43 (d, *J* = 2.1 Hz, 1H), 9.18 (s, 1H), 9.10 (s, 1H), 8.80 (s, 1H), 8.17 (s, 1H), 7.62 (d*, J* = 5.8 Hz, 2H), 7.36 (t, *J* = 7.8 Hz, 1H), 3.75 (s, 3H), 2.14 - 2.04 (m, 1H), 0.82 (m, 4H). |
| **9-j** | ¹H NMR (500 MHz, DMSO-*d*₆) δ 11.34 (s, 1H), 10.97 (s, 1H), 9.15 (s, 1H), 9.11 (s, 2H), 8.17 (s, 1H), 7.91 (dd, *J* = 6.9, 2.1 Hz, 1H), 7.61 (dddd, *J* = 13.3, 9.0, 6.4, 1.9 Hz, 3H), 7.35 (t, *J* = 7.9 Hz, 1H), 6.58 (d, *J* = 9.1 Hz, 1H), 6.44 (td, *J* = 6.7, 1.3 Hz, 1H), 3.75 (s, 3H), 2.09 (p, *J* = 6.3 Hz, 1H), 0.86 - 0.78 (m, 4H). |
| **10-j** | ¹H NMR (500 MHz, DMSO-*d*₆) δ 11.35 (s, 1H), 10.98 (s, 1H), 9.44 (s, 2H), 9.37 (s, 2H), 9.30 (s, 1H), 9.16 (s, 1H), 8.18 (s, 1H), 7.63 (dd, *J* = 7.8, 3.1 Hz, 2H), 7.35 (t, *J* = 7.9 Hz, 1H), 3.75 (s, 3H), 2.10 (q, *J* = 6.2 Hz, 1H), 0.95 - 0.74 (m, 4H). |
| **11-h** | ¹H NMR (500 MHz, DMSO-*d*₆) δ11.42 (s, 1H), 11.10 (s, 1H), 9.20 (s, 1H), 8.34 (d, *J* = 7.4 Hz, 1H), 8.21 (s, 1H), 7.72 (dq, *J* = 8.8, 4.3, 3.8 Hz, 1H), 7.45 - 7.41 (m, 2H), 7.38 (d, *J* = 2.9 Hz, 1H), 7.26 (dd, *J* = 7.5, 2.8 Hz, 1H), 3.75 (m, 7H), 3.54 (s, 4H), 2.10 (m, 1H), 0.91 - 0.74 (m, 4H). |
| **12-j** | ¹H NMR (500 MHz, DMSO-*d*₆) δ 11.44 (s, 1H), 11.06 (s, 1H), 9.23 (s, 2H), 9.16 (s, 1H), 8.62 (dt, *J* = 9.8, 7.9 Hz, 1H), 8.12 (s, 1H), 7.65 (ddd, *J* = 12.0, 7.9, 1.6 Hz, 2H), 7.43 (dd, *J* = 8.2, 2.5 Hz, 1H), 7.36 (t, *J* = 7.9 Hz, 1H), 3.77 (s, 3H), 2.10 (q, *J* = 6.2 Hz, 1H), 0.82 (m, 4H). |
| **13-h** | ¹H NMR (500 MHz, DMSO-*d*₆) δ 11.30 (s, 1H), 10.97 (s, 1H), 9.19 (s, 2H), 9.10 (s, 1H), 8.76 - 8.62 (m, 1H), 8.42 (dd, *J* = 4.8, 2.0 Hz, 1H), 8.18 (s, 1H), 7.58 (ddd, *J* = 6.2, 4.7, 1.6 Hz, 2H), 7.49 - 7.26 (m, 2H), 3.78 (s, 3H), 2.10 (q, *J* = 6.2 Hz, 1H), 0.95 - 0.74 (m, 4H). |
| **14-h** | ¹H NMR (500 MHz, DMSO-*d*₆) δ 11.34 (s, 1H), 10.97 (s, 1H), 9.15 (s, 1H), 8.99 (s, 2H), 8.17 (s, 1H), 8.04 (dd*, J* = 4.9, 1.8 Hz, 1H), 7.62 (ddd, *J* = 18.2, 8.0, 1.6 Hz, 2H), 7.51 (dd, *J* = 7.4, 1.9 Hz, 1H), 7.33 (t, *J* = 7.9 Hz, 1H), 6.71 (dd, *J* = 7.3, 4.9 Hz, 1H), 6.02 (s, 2H), 3.76 (s, 3H), 2.09 (tt, *J* = 7.0, 5.5 Hz, 1H), 0.88 - 0.80 (m, 4H). |
| **15-h** | ¹H NMR (500 MHz, DMSO-*d*₆) δ 11.80 (s, 1H), 11.34 (s, 1H), 10.97 (s, 1H), 9.31 (s, 2H), 9.15 (s, 1H), 8.16 (s, 1H), 7.62 (d, *J* = 7.9 Hz, 2H), 7.56 (d, *J* = 6.8 Hz, 1H), 7.34 (t, *J* = 7.9 Hz, 1H), 6.90 (d, *J* = 1.8 Hz, 1H), 6.82 - 6.63 (m, 1H), 3.79 (s, 3H), 2.08 (p, *J* = 6.4 Hz, 1H), 0.92 - 0.69 (m, 4H). |
| **16-h** | ¹H NMR (500 MHz, DMSO-*d*₆) δ 11.34 (s, 1H), 10.98 (s, 1H), 9.38 (s, 2H), 9.15 (s, 1H), 8.16 (s, 1H), 7.69 (t, *J* = 7.9 Hz, 1H), 7.66 - 7.52 (m, 2H), 7.34 (t, *J* = 7.9 Hz, 1H), 7.16 (d, *J* = 29.2 Hz, 1H), 6.59 (d, *J* = 8.9 Hz, 1H), 3.72 (s, 3H), 2.19 - 2.03 (m, 1H), 0.97 - 0.74 (m, 4H). |
| **17-h** | ¹H NMR (500 MHz, DMSO-*d*₆) δ 12.05 (s, 1H), 11.36 (s, 1H), 10.99 (s, 1H), 9.20 (s, 2H), 9.16 (s, 1H), 8.20 (s, 1H), 8.13 - 8.07 (m, 1H), 8.05 (dd, *J* = 9.5, 2.7 Hz, 1H), 7.66 - 7.53 (m, 2H), 7.33 (t, *J* = 7.9 Hz, 1H), 6.54 (d, *J* = 9.5 Hz, 1H), 3.74 (s, 3H), 2.11 (p, *J* = 6.3 Hz, 1H), 0.85 (d, *J* = 6.2 Hz, 4H). |
| **18-h** | ¹H NMR (500 MHz, DMSO-*d*₆) δ 11.36 (s, 1H), 10.98 (d*, J* = 4.2 Hz, 1H), 9.26 (d*, J* = 4.2 Hz, 2H), 9.14 (s, 1H), 8.13 (d, *J=* 4.4 Hz, 1H), 7.65 - 7.49 (m, 2H), 7.31 (t, *J* = 7.8 Hz, 1H), 3.96 (t, *J* = 7.0 Hz, 2H), 3.69 (d, *J* = 4.2 Hz, 3H), 2.56 (t, *J* = 8.0 Hz, 2H), 2.15 (p, *J* = 7.5 Hz, 2H), 2.07 (q, *J* = 6.2 Hz, 1H), 0.83 (d, *J* = 4.9 Hz, 4H). |
| **19-h** | ¹H NMR (500 MHz, DMSO-*d*₆) δ 14.22 (s,1H), 11.34 (s, 1H), 11.02 (s, 1H), 9.31 (d, *J* = 2.2 Hz, 1H), 9.15 (s, 1H), 8.59 (s, 1H), 8.42 (dd, *J* = 8.3, 2.3 Hz, 1H), 8.19 (s, 1H), 8.01 (d, *J* = 8.3 Hz, 1H), 7.63 (dd, *J* = 7.9, 1.6 Hz, 1H), 7.54 (dd*, J* = 8.0, 1.6 Hz, 1H), 7.33 (t, *J* = 7.9 Hz, 1H), 3.51 (s, 3H), 2.10 (p, *J* = 6.2 Hz, 1H), 0.84 (d, *J* = 6.2 Hz, 4H). |
| **20-h** | ¹H NMR (500 MHz, DMSO-*d*₆) δ 12.09 (s, 1H), 11.33 (s, 1H), 10.96 (s, 1H), 9.31 (d, *J* = 2.0 Hz, 2H), 9.14 (s, 1H), 8.16 (d, *J* = 2.0 Hz, 1H), 8.01 (dd, *J* = 7.0, 2.1 Hz, 1H), 7.68 - 7.55 (m, 2H), 7.53 (d, *J* = 6.5 Hz, 1H), 7.32 (t, *J* = 7.9 Hz, 1H), 6.40 (t, *J* = 6.7 Hz, 1H), 3.72 (d, *J* = 2.2 Hz, 2H), 2.08 (p, *J* = 6.3 Hz, 1H), 0.92 - 0.75 (m, 4H). |
| **21-h** | ¹H NMR (500 MHz, DMSO-*d*₆) δ 11.32 (s, 1H), 10.98 (s, 1H), 9.14 (s, 1H), 8.32 - 8.10 (m, 2H), 7.47 (dd, *J* = 14.4, 7.8 Hz, 2H), 7.25 (t, *J* = 7.9 Hz, 1H), 6.92 (d, *J* = 2.4 Hz, 1H), 6.47 (dd, *J* = 5.9, 2.4 Hz, 1H), 4.49 - 4.36 (m, 1H), 3.50 (s, 3H), 3.48 - 3.33 (m, 4H), 3.18 (m, 2H), 2.57 (m, 1H), 2.09 (m, 1H), 0.83 (d, *J* = 6.2 Hz, 4H). |
| **22-h** | ¹H NMR (500 MHz, DMSO-*d*₆) δ 13.26 (s, 1H), 11.36 (s, 1H), 11.05 (s, 1H), 9.17 (s, 1H), 8.73 (d, *J* = 5.3 Hz, 1H), 8.38 - 8.14 (m, 2H), 8.00 - 7.76 (m, 2H), 7.56 (t, *J* = 7.5 Hz, 2H), 7.34 (t, *J* = 7.7 Hz, 1H), 6.98 (d, *J* = 2.8 Hz, 1H), 3.55 (s, 3H), 2.12 (m, 1H), 0.86 (d, *J* = 8.6 Hz, 4H). |
| **23-h** | ¹H NMR (500 MHz, DMSO-*d*₆) δ 11.38 (s, 1H), 11.01 (s, 1H), 9.35 (s, 2H), 9.15 (s, 1H), 8.14 (s, 1H), 7.90 (d, *J* = 2.3 Hz, 1H), 7.61 (ddd, *J* = 10.8, 7.9, 1.6 Hz, 2H), 7.33 (t, *J* = 7.9 Hz, 1H), 7.02 (d, *J* = 2.3 Hz, 1H), 3.72 (s, 3H), 2.08 (p, *J* = 6.3 Hz, 1H), 0.84 (d, *J* = 6.2 Hz, 4H). |
| **24-h** | ¹H NMR (500 MHz, DMSO-*d*₆) δ 11.34 (s, 1H), 11.01 (s, 1H), 9.27 (d, *J* = 2.2 Hz, 1H), 9.15 (s, 1H), 8.63 (s, 1H), 8.39 (dd, *J* = 8.3, 2.2 Hz, 1H), 8.19 (s, 1H), 8.00 (d, *J* = 8.2 Hz, 1H), 7.62 (dd, *J* = 7.8, 1.6 Hz, 1H), 7.54 (dd, *J* = 7.9, 1.7 Hz, 1H), 7.33 (t, *J* =7.9 Hz, 1H), 3.97 (s, 3H), 3.51 (s, 3H), 2.09 (p, *J* = 6.2 Hz, 1H), 0.88 - 0.81 (m, 4H). |
| **25-h** | ¹H NMR (500 MHz, DMSO-*d*₆) δ 11.33 (s, 1H), 10.93 (d, *J* = 14.6 Hz, 1H), 9.13 (s, 1H), 8.20 - 8.09 (m, 2H), 7.50 - 7.31 (m, 2H), 7.21 (p, *J* = 7.8 Hz, 1H), 7.16 - 6.98 (m, 1H), 6.67 (t, *J* = 6.0 Hz, 1H), 4.14 (p, *J* = 3.7 Hz, 1H), 3.55 - 3.02 (m, 7H), 2.87 (m, 1H), 2.10 (p, *J* = 6.2 Hz, 1H), 1.85 - 1.59 (m, 2H), 0.84 (d, *J* = 6.2 Hz, 4H). |
| **26-h** | ¹H NMR (500 MHz, DMSO-*d*₆) δ 11.34 (s, 1H), 10.99 (s, 1H), 9.30 (s, 2H), 9.16 (s, 1H), 8.96 - 8.79 (m, 1H), 8.45 - 8.31 (m, 1H), 8.18 (s, 1H), 7.94 (dd, *J* = 8.0, 4.7 Hz, 1H), 7.74 - 7.59 (m, 2H), 7.37 (t, *J* = 7.9 Hz, 1H), 3.76 (s, 3H), 2.09 (p, *J* = 6.3 Hz, 1H), 0.83 (d, *J* = 6.2 Hz, 4H). |
| **27-h** | ¹H NMR (500 MHz, DMSO-*d*₆) δ 11.35 (s, 1H), 10.99 (s, 1H), 9.30 (s, 2H), 9.14 (s, 1H), 8.15 (s, 1H), 7.98 (dd, *J* = 7.0, 2.0 Hz, 1H), 7.88 (dd, *J* = 6.7, 2.0 Hz, 1H), 7.60 (dq, *J* = 8.0, 1.7 Hz, 2H), 7.33 (t, *J* = 7.9 Hz, 1H), 6.43 (t, *J* = 6.8 Hz, 1H), 3.73 (s, 3H), 3.56 (s, 3H), 2.08 (p, *J* = 6.3 Hz, 1H), 0.83 (d, *J* = 6.2 Hz, 4H). |
| **28-h** | ¹H NMR (500 MHz, DMSO-*d*₆) δ 11.34 (s, 1H), 10.96 (s, 1H), 9.20 (s, 2H), 9.15 (s, 1H), 8.46 (d, *J* = 2.7 Hz, 1H), 8.17 (s, 1H), 8.04 (dd, *J* = 9.5, 2.7 Hz, 1H), 7.59 (d, *J* = 7.9 Hz, 2H), 7.33 (t, *J* = 7.9 Hz, 1H), 6.56 (d, *J* = 9.5 Hz, 1H), 3.71 (s, 3H), 3.54 (s, 3H), 2.09 (p, *J* = 6.3 Hz, 1H), 0.88 - 0.80 (m, 4H). |
| **29-h** | ¹H NMR (500 MHz, DMSO-*d*₆) δ 11.34 (s, 1H), 11.02 (s, 1H), 9.27 (d, *J* = 2.2 Hz, 1H), 9.15 (s, 1H), 8.75 (s, 1H), 8.38 (dd, *J=* 8.3, 2.3 Hz, 1H), 8.19 (s, 1H), 8.04 - 7.97 (m, 1H), 7.62 (dd, *J* = 7.8, 1.6 Hz, 1H), 7.54 (dd, *J* = 7.9, 1.6 Hz, 1H), 7.33 (t, *J* = 7.9 Hz, 1H), 3.51 (s, 3H), 2.10 (p, *J* = 6.2 Hz, 1H), 1.23 (dd, *J* = 8.6, 5.6 Hz, 1H), 1.22 - 1.13 (m, 2H), 1.15 - 1.04 (m, 2H), 0.84 (d, *J* = 6.2 Hz, 4H). |
| **30-h** | ¹H NMR (500 MHz, DMSO-*d*₆) δ 11.34 (s, 1H), 10.97 (s, 1H), 9.25 (s, 2H), 9.15 (s, 1H), 8.52 - 8.48 (m, 1H), 8.17 (s, 1H), 7.88 (t, *J* = 1.7 Hz, 1H), 7.66 - 7.52 (m, 2H), 7.33 (t, *J* = 7.9 Hz, 1H), 7.22 (d, *J* = 1.8 Hz, 1H), 3.71 (s, 3H), 2.09 (p, *J* = 6.3 Hz, 1H), 0.83 (d, *J* = 6.2 Hz, 4H). |
| **31-h** | ¹H NMR (500 MHz, DMSO-*d*₆) δ 13.30 (s, 1H), 11.34 (s, 1H), 10.96 (s, 1H), 9.23 (s, 2H), 9.14 (s, 1H), 8.45 (s, 1H), 8.17 (s, 1H), 7.61 - 7.55 (m, 2H), 7.31 (t, *J* = 7.9 Hz, 1H), 3.71 (s, 3H), 2.08 (q, *J* = 6.2 Hz, 1H), 1.26 - 1.21 (m, 1H), 0.83 (d, *J* = 6.2 Hz, 4H). |
| **32-h** | ¹H NMR (500 MHz, DMSO-*d*₆) δ 11.34 (s, 1H), 10.97 (s, 1H), 9.15 (s, 1H), 8.98 (s, 2H), 8.45 (s, 1H), 8.16 (s, 1H), 7.66 - 7.58 (m, 2H), 7.34 (t, *J* = 7.9 Hz, 1H), 4.02 (s, 3H), 3.72 (s, 3H), 2.09 (p, *J* = 6.3 Hz, 1H), 0.87 - 0.80 (m, 4H). |
| **33-h** | ¹H NMR (500 MHz, DMSO-*d*₆) δ 11.34 (s, 1H), 10.98 (s, 1H), 9.31 (s, 2H), 9.15 (s, 1H), 8.18 (s, 1H), 7.87 (d, *J* = 2.4 Hz, 1H), 7.60 (dd, *J* = 7.9, 4.2 Hz, 2H), 7.32 (t, *J* = 7.9 Hz, 1H), 6.99 (d, *J* = 2.3 Hz, 1H), 3.96 (s, 3H), 3.71 (s, 3H), 2.10 (p, *J* = 6.2 Hz, 1H), 0.83 (d, *J*= 6.1 Hz, 4H). |
| **34-h** | ¹H-NMR(DMSO-*d₆*)*δ*_{:} 11.33(1H, s), 10.98(1H, s), 9.31(2H, s), 9.15(1H, s), 7.17(1H, s), 7.87(1H, d, *J*=2.0), 7.61(2H, m), 7.34(1H, t, *J*=8.0), 6.99(1H, d, *J*=2.0), 3.96(3H, m), 3.33(3H, s), 2.09(1H, m), 0.84(4H, m). ¹³C-NMR(DMSO-*d₆*)*δ*_{:} 174.2, 167.1, 163.2, 156.3, 153.8, 151.8, 145.3, 144.8, 135.5, 134.1, 133.5, 132.7, 127.9, 125.4, 124.5, 124.3, 104.2, 97.2, 62.2, 14.9, 8.6, 0.57. |
| **35-h** | ¹H NMR (500 MHz, DMSO-*d*₆) δ 11.34 (s, 1H), 10.96 (s, 1H), 9.19 (s, 2H), 9.15 (s, 1H), 8.42 (s, 1H), 8.15 (d, *J* = 17.6 Hz, 2H), 7.58 (d, *J* = 7.9 Hz, 2H), 7.31 (t, *J* = 7.9 Hz, 1H), 3.92 (s, 3H), 3.71 (s, 3H), 2.09 (p, *J* = 6.3 Hz, 1H), 0.83 (d, *J* = 6.2 Hz, 4H). |
| **36-h** | ¹H NMR (500 MHz, DMSO-*d*₆) δ 11.35 (s, 1H), 10.97 (s, 1H), 9.25 (s, 2H), 9.15 (s, 2H), 8.93 (s, 1H), 8.16 (s, 1H), 7.60 (d, *J* = 7.8 Hz, 2H), 7.33 (t, *J* = 7.9 Hz, 1H), 6.07 (s, 2H), 3.72 (s, 3H), 2.08 (t, *J* = 6.4 Hz, 1H), 1.24 (s, 1H), 0.83 (d, *J* = 6.7 Hz, 4H). |
| **37-h** | ¹H NMR (500 MHz, DMSO-*d*₆) δ 11.34 (s, 1H), 10.99 (s, 1H), 9.41 (s, 2H), 9.15 (s, 1H), 8.83 (s, 1H), 8.17 (s, 1H), 7.65 - 7.59 (m, 2H), 7.34 (t, *J* = 7.9 Hz, 1H), 3.90 (tt, *J* = 7.5, 3.8 Hz, 1H), 3.71 (s, 3H), 2.09 (p, *J* = 6.3 Hz, 1H), 1.21 (td, *J* = 4.8, 4.4, 3.2 Hz, 2H), 1.16 - 1.07 (m, 2H), 0.83 (d, *J =* 6.2 Hz, 4H). |
| **38-h** | ¹H NMR (500 MHz, DMSO-*d*₆) δ 11.32 (d, *J =* 13.8 Hz, 1H), 10.94 (d, *J* = 8.5 Hz, 1H), 9.13 (d, *J* = 13.6 Hz, 2H), 8.93 (d, *J =* 13.7 Hz, 1H), 8.62 (s, 1H), 8.15 (s, 1H), 7.62 - 7.52 (m, 1H), 7.55 - 7.47 (m, 1H), 7.38 - 7.22 (m, 1H), 7.19 (d, *J* = 11.4 Hz, 1H), 3.67 (d, *J* = 13.3 Hz, 3H), 2.11 - 2.05 (m, 1H), 0.88 - 0.80 (m, 4H). |
| **39-h** | ¹H NMR (500 MHz, DMSO-*d*₆) δ 11.35 (s, 1H), 10.96 (s, 1H), 9.15 (s, 3H), 8.16 (s, 1H), 7.86 (s, 1H), 7.62 (dd, *J* = 10.9, 7.8 Hz, 2H), 7.41 (s, 1H), 7.34 (t, *J* = 7.9 Hz, 1H), 3.84 (s, 3H), 3.74 (s, 3H), 2.09 (p, *J* = 6.2 Hz, 1H), 0.88 - 0.80 (m, 4H). |
| **40-h** | ¹H NMR (500 MHz, DMSO-*d*₆) δ 11.31 (s, 1H), 10.92 (s, 1H), 9.13 (s, 1H), 8.40 (s, 2H), 8.16 (s, 1H), 7.55 - 7.40 (m, 2H), 7.26 (t, *J* = 7.9 Hz, 1H), 3.85 (s, 2H), 3.78 (t, *J* = 7.5 Hz, 2H), 3.67 (s, 3H), 2.73 (t, *J* = 7.5 Hz, 2H), 2.54 (s, 2H), 2.13 - 2.02 (m, 1H), 0.90 - 0.73 (m, 4H). |
| **41-h** | ¹H NMR (500 MHz, DMSO-*d*₆) δ 14.39 (s, 1H), 11.34 (s, 1H), 10.98 (s, 1H), 9.46 (s, 2H), 9.15 (s, 1H), 8.72 (s, 1H), 8.17 (s, 1H), 7.62 (t, J = 7.6 Hz, 2H), 7.34 (t, J = 7.9 Hz, 1H), 3.72 (s, 3H), 2.13 - 2.06 (m, 1H), 0.83 (d, J = 6.1 Hz, 4H). |
| **43-j** | ¹H NMR (500 MHz, DMSO-*d*₆) δ 13.74 (s, 1H), 11.34 (s, 1H), 10.97 (s, 1H), 9.15 (s, 1H), 9.01 (s, 2H), 8.51 (s, 1H), 8.17 (s, 1H), 7.66 - 7.58 (m, 2H), 7.34 (t, *J=* 7.9 Hz, 1H), 3.73 (s, 3H), 2.09 (p, *J=* 6.3 Hz, 1H), 0.96 - 0.76 (m, 4H). |
| **44-j** | ¹H NMR (500 MHz, DMSO-*d*₆) δ 11.31 (s, 1H), 10.92 (s, 1H), 9.13 (s, 1H), 8.40 (s, 2H), 8.16 (s, 1H), 7.53 - 7.47 (m, 2H), 7.26 (t, *J* = 7.9 Hz, 1H), 4.35 (m, 1H), 3.84 (d, *J=* 5.3 Hz, 1H), 3.74 -3.36 (m, 7H), 2.11 (m, 1H), 1.97-1.86 (m, 2H), 1.03 - 0.88 (m, 4H). |
| **45-j** | ¹H NMR (500 MHz, DMSO-*d*₆) δ 11.30 (s, 1H), 10.91 (s, 1H), 9.11 (s, 1H), 8.14 (d, *J* = 3.3 Hz, 3H), 7.47 (dd, *J* = 7.9, 4.5 Hz, 2H), 7.24 (t, *J* = 7.8 Hz, 1H), 4.01 (t, *J* = 7.2 Hz, 4H), 3.64 (s, 3H), 2.42 (t, *J* = 7.4 Hz, 2H), 2.08 (p, *J* = 6.4 Hz, 1H), 0.82 (d, *J=* 6.1 Hz, 4H). |
| **46-j** | ¹H NMR (500 MHz, DMSO-*d*₆) δ 11.31 (s, 1H), 10.92 (s, 1H), 9.13 (s, 1H), 8.40 (s, 2H), 8.16 (s, 1H), 7.53 - 7.47 (m, 2H), 7.26 (t, *J* = 7.9 Hz, 1H), 3.85 (s, 2H), 3.78 (t, *J* = 7.5 Hz, 2H), 3.67 (s, 3H), 2.75 (s, 2H), 2.13 - 2.04 (m, 1H), 0.89 - 0.76 (m, 4H). |
| **47-j** | ¹H-NMR(DMSO-*d₆*)*δ*_{:} 11.32(1H, s), 10.94(1H, s), 9.23(2H, s), 9.13(1H, d), 8.16(1H, s), 7.5(2H, m), 7.30(1H, t), 3.70(5H, m), 2.50(1H, t), 2.43(1H, m), 2.08(1H, m), 1.23(6H, s), 0.82(4H, d). |
| **48-j** | ¹H NMR (500 MHz, DMSO-*d*₆) δ 11.33 (s, 1H), 10.95 (s, 1H), 9.27 (s, 2H), 9.14 (s, 1H), 8.16 (s, 1H), 7.63 - 7.50 (m, 2H), 7.30 (t, *J* = 7.9 Hz, 1H), 3.93 (td, *J* = 9.2, 2.6 Hz, 1H), 3.86 (td, *J* = 9.3, 7.2 Hz, 1H), 3.68 (s, 3H), 2.71 (td, *J* = 9.1, 6.9 Hz, 1H), 2.44 - 2.35 (m, 1H), 2.08 (p, *J* = 6.5 Hz, 1H), 1.78 (dq, *J* = 12.1, 9.0 Hz, 1H), 1.20 (d, *J* = 7.1 Hz, 3H), 0.88 - 0.76 (m, 4H). |
| **49-j** | ¹H NMR (500 MHz, DMSO-*d*₆) δ 11.33 (s, 1H), 10.95 (s, 1H), 9.28 (s, 2H), 9.14 (s, 1H), 8.16 (s, 1H), 7.55 (ddd, *J* = 14.8, 7.9, 1.6 Hz, 2H), 7.30 (t, *J* = 7.9 Hz, 1H), 3.92 (t, *J* = 6.9 Hz, 2H), 3.69 (s, 3H), 2.13 - 1.95 (m, 3H), 1.19 (s, 6H), 0.89 - 0.72 (m, 4H). |
| **50-j** | ¹H NMR (500 MHz, DMSO-*d*₆) δ 11.32 (s, 1H), 10.93 (s, 1H), 9.13 (s, 1H), 8.36 (s, 2H), 8.16 (s, 1H), 7.50 (ddd, *J* = 7.9, 6.2, 1.7 Hz, 2H), 7.27 (t, *J* = 7.9 Hz, 1H), 4.93 (s, 4H), 3.67 (s, 3H), 2.14 - 2.03 (m, 1H), 0.92 - 0.75 (m, 4H). |
| **51-j** | ¹H NMR (500 MHz, DMSO-*d*₆) δ 11.31 (s, 1H), 10.91 (s, 1H), 9.12 (s, 1H), 8.16 (d, *J* = 8.2 Hz, 3H), 7.51 - 7.43 (m, 2H), 7.24 (t, *J =* 7.9 Hz, 1H), 5.75 (s, 1H), 4.65 (h, *J =* 5.7 Hz, 1H), 4.25 (t, *J =* 7.3 Hz, 2H), 3.73 (dd, *J =* 8.3, 4.8 Hz, 2H), 3.64 (s, 3H), 2.08 (p, *J =* 6.3 Hz, 1H), 0.82 (d, *J* = 5.0 Hz, 4H). |
| **52-j** | ¹H-NMR(DMSO-*d₆*)*δ*_{:} 11.34(1H, s), 10.97(1H, s), 9.15(1H, s), 9.07(2H, s),8.57(1H, d, *J*=3.5), 8.16(1H, s),7.86(1H, d, *J*=7), 7.63(2H, m), 7.41(1H, m), 7.35(1H, m), 3.74(3H, s), 2.55(3H, s),2.10(1H, m), 0.83(4H, m). |
| **53-j** | ¹H-NMR(DMSO-*d₆*)δ_{:} 11.31(1H, s), 10.92(1H, s), 9.12 (1H, s),8.23 (2H, m), 8.16(1H, s),7.45 (2H,m),7.25 (1H,m) 5.05(1H, s),4.45 (1H, s), 3.65(3H, s), 3.49 (1H, m), 3.45 (2H, m), 3.43 (1H, m), 2.09 (2H, m), 2.05(1H, m),0.82 (4H, m). |
| **54-j** | ¹H NMR (500 MHz, DMSO-*d*₆) δ 11.34 (s, 1H), 10.97 (s, 1H), 9.15 (d, *J* = 2.1 Hz, 3H), 8.84 (s, 1H), 8.16 (s, 1H), 7.64 (ddd, *J* = 18.5, 7.9, 1.6 Hz, 2H), 7.35 (t, *J* = 7.9 Hz, 1H), 7.20 (s, 1H), 3.75 (s, 3H), 2.57 (s, 3H), 2.13 - 2.05 (m, 1H), 0.85 - 0.80 (m, 4H). |
| **55-j** | ¹H NMR (500 MHz, DMSO-*d*₆) δ 11.35 (s, 1H), 10.99 (s, 1H), 9.16 (s, 3H), 8.17 (s, 1H), 7.64 (t, *J* = 7.8 Hz, 2H), 7.56 (s, 1H), 7.36 (t, *J* = 7.9 Hz, 1H), 7.02 (s, 1H), 3.76 (s, 3H), 2.43 (s, 3H), 2.10 (p, *J* = 6.4 Hz, 1H), 0.83 (d, *J* = 4.7 Hz, 4H). |
| **56-j** | ¹H NMR (500 MHz, DMSO-*d*₆) δ 11.35 (s, 1H), 10.98 (s, 1H), 9.22 (s, 2H), 9.16 (s, 1H), 8.16 (s, 1H), 7.95 (d*, J* = 1.3 Hz, 1H), 7.66 (dd, *J* = 7.9, 2.5 Hz, 2H), 7.41 (d, *J* = 1.2 Hz, 1H), 7.37 (t, *J* = 7.9 Hz, 1H), 3.73 (s, 3H), 2.08 (q, *J* = 7.1, 5.9 Hz, 1H), 0.83 (dd, *J* = 10.8, 6.3 Hz, 4H). |
| **57-j** | ¹H NMR (500 MHz, DMSO-*d*₆) δ 10.81 (s, 1H), 10.73 (s, 1H), 9.25 (s, 2H), 8.65 (s, 1H), 8.57 (s, 1H), 8.45 - 8.34 (m, 2H), 8.12 (s, 1H), 7.63 (d, *J* = 8.0 Hz, 1H), 7.57 (d, *J* = 7.6 Hz, 2H), 7.32 (t, *J* = 7.9 Hz, 1H), 3.79 (s, 3H), 2.03 (td, *J* = 7.7, 3.8 Hz, 1H), 0.85 - 0.76 (m, 4H). |
| **58-j** | ¹H NMR (500 MHz, DMSO-*d*₆) δ 11.42 (s, 1H), 10.94 (s, 1H), 9.44 (s, 2H), 9.38 (s, 2H), 9.31 (s, 1H), 8.87 (s, 1H), 8.51 (s, 1H), 7.66 (dd, *J* = 30.3, 9.5 Hz, 2H), 7.46 (s, 1H), 7.38 (t, *J* = 7.9 Hz, 1H), 3.75 (s, 3H), 1.91 (td, *J* = 7.6, 3.8 Hz, 1H), 0.91 - 0.86 (m, 4H). |
| **59-j** | ¹H NMR (500 MHz, DMSO-*d*₆) δ 10.75 (s, 1H), 10.62 (s, 1H), 8.59 (s, 1H), 8.52 (s, 1H), 8.23 (s, 2H), 8.07 (s, 1H), 7.44 (d, *J =* 9.6 Hz, 1H), 7.39 (d, *J =* 7.9 Hz, 1H), 7.19 (t, *J =* 7.9 Hz, 1H), 4.45 (s, 1H), 3.65 (s, 3H), 3.22 (d, *J =* 8.9 Hz, 1H), 2.53 (s, 1H), 2.08 - 2.02 (m, 1H), 1.86 (s, 5H), 0.78 (s, 4H). |
| **60-j** | ¹H-NMR(DMSO-*d₆*)*δ*_{:} 10.74 (1H, s), 10.61 (1H, s), 8.64(2H, m), 8.51-8.23(2H, m),8.01(1H,s), 7.43-7.40(2H, m), 7.19(1H, s), 5.06(1H, s),4.45(1H, s), 3.65(3H, s), 3.35(1H, s), 2.07-1.98(5H, m), 0.78 (4H, m). |
| **61-h** | ¹H NMR (500 MHz, DMSO-*d*₆) δ 10.78 (s, 1H), 10.70 (s, 1H), 9.46 (s, 2H), 8.74 (s, 1H), 8.61 (s, 1H), 8.54 (s, 1H), 8.17 (s, 1H), 8.09 (s, 1H), 7.57 (dd, *J* = 24.5, 7.9 Hz, 2H), 7.30 (t, *J =* 7.9 Hz, 1H), 3.73 (s, 3H), 1.99 (d, *J =* 5.1 Hz, 1H), 0.80 (d, *J=* 4.4 Hz, 4H). |

### Experimental Example 1: Assay for Thermal Stability of TYK2 JH2

A 0.52 mg/mL TYK2 JH2 (Beijing Sino Biological) protein stock solution was diluted to 50 ng/µL with phosphate buffered saline (PBS), while 5000× protein dye (Orange dye) was diluted to 20× with DMSO. 16 µL of TYK2 JH2 protein diluent was added to each well, and then different compounds dissolved in DMSO were added to the wells with a nanoliter pipettor, allowing the final compound concentrations to be 10 µM and 1 µM (2 concentrations in total). Blank control wells (without enzyme) and negative control wells (with enzyme, plus vehicle DMSO) were set, and the samples were tested in duplicate. Finally, 4 µL of Orange dye protein dye was added to each well, and the mixture was mixed well by centrifugation. The Roche LightCycler480 fluorescence quantitative PCR instrument was used for detection, with a running system of 20 °C 15 s; 30-90 °C 0.02 °C/s; 20 °C 15 s. Melting temperatures (Tm) were obtained by analysis using LightCycler Thermal Shift Analysis software.

The compound disclosed herein, under the condition that the final concentration is 10 µM, has a melting temperature (Tm) of greater than 40 °C, preferably greater than 45 °C, and more preferably greater than 55 °C, and under the condition that the final concentration is 1 µM, the compound has a melting temperature (Tm) of greater than 40 °C, preferably greater than 45 °C, and more preferably greater than 50 °C.

### Experimental Example 2: Detection of STAT3 Phosphorylation Using Jurkat Cells

20 µL of Jurkat cells growing at log phase were taken and counted, and the required number of cells (mL) were taken and centrifuged at 1300 rpm for 3 min. Phenol red-free 1640 base medium (Hyclone) was added to adjust the cell density to about 1.7 * 10E7 cells/mL. The cells were seeded according to the above cell density in a 384-well plate (small-volume white plate) at 8 µL per well. The compound was added with a nanoliter pipettor, and the plate was incubated for 1.5 h. IFN-α (Beijing Sino Biological) was diluted to 75 ng/mL (with a final concentration of 25 ng/mL) with phenol red-free 1640 base medium. Subsequently, 4 µL of IFN-α (3×) was added to each well according to the plate distribution. The blank group was seeded with cells, without compound and IFN-α. The control group was seeded with cells, without compound but with IFN-α. The plate was incubated at 37 °C for 30 min. 4 µL of lysis buffer (4×) containing blocking buffer was added immediately, and the cells were lysed by shaking at room temperature for 40 min. 4 µL of pre-mixed antibody (vol/vol) in assay buffer was added and the plate was covered. The mixture was mixed well by centrifugation and incubated overnight at room temperature. Signal values at 665 nm/620 nm were detected using a PE Envision multi-functional microplate reader, and IC₅₀ was calculated by four-parameter fitting.

The results are shown in Table 1.

**Table 1**

| **Compound** | **IC₅₀ (nM)** |
|---|---|
| 1-j | 4.5 |
| 5-j | 2.9 |
| 6-i | 2.4 |
| 12-j | 4.5 |
| 18-h | 5.3 |
| 26-h | 5.3 |
| 34-h | 5.7 |
| 37-h | 3.7 |
| 47-j | 2.4 |
| 49-j | 5.2 |
| 51-j | 7.1 |
| 52-j | 2.7 |
| 53-j | 5.1 |
| 54-j | 3.4 |
| 55-j | 5.2 |
| 57-j | 4.4 |
| 59-j | 5 |
| 60-j | 8.5 |

### Experimental Example 3: In Vitro Stability in Liver Microsomes

300 µL of the final incubation system contained 30 µL of liver microsomes (protein concentration: 5 mg/mL), 30 µL of NADPH + MgCl₂, 3 µL of the test compound (in acetonitrile), and 237 µL of PBS buffer (pH 7.4), wherein the proportion of the organic solvent (acetonitrile) was 1% (volume ratio). Samples were prepared in duplicate of 0.30 mL for each specie. Tubes each containing 270 µL of a mixed solution of substrate and enzyme and NADPH were pre-incubated at 37 °C for 5 min. 30 µL of NADPH + MgCh was added and the mixture was mixed. 50 µL of the mixture was taken at 0 min, 10 min, 30 min, and 60 min, and 300 µL of glacial acetonitrile containing an internal standard was added to terminate the reaction.

300 µL of glacial acetonitrile containing an internal standard (20 ng/mL) was added to 50 µL of the incubated sample for precipitation. The mixture was vortexed for 10 min and centrifuged (13,000 rpm, 20 °C) for 10 min. 70 µL of supernatant was taken and diluted with 70 µL of ultrapure water. After being mixed well, 0.5 µL of the resulting sample was subjected to LC/MS/MS analysis. The results are shown in Table 2.

**Table 2**

| Compound (1 µM) | Human | Monkey | Mouse |
|---|---|---|---|
| | Residuals at 60 min (%) | Residuals at 60 min (%) | Residuals at 60 min (%) |
| 3-j | 103.99% | 95.82% | 99.92% |
| 5-j | 107.15% | 99.31% | 99.08% |
| 6-j | 100.18% | / | / |
| 18-h | 106.77% | / | 94.80% |
| 34-h | 99.75% | / | 92.69% |
| 37-h | 101.95% | / | 97.99% |
| 38-h | 95.69% | / | 98.27% |
| 44-j | 98.44% | / | 91.31% |
| 51-j | / | / | 96.42% |
| 53-j | / | / | 94.17% |
| 54-j | / | / | 92.35% |
| 59-j | / | / | 100.66% |

### Experimental Example 4: In Vivo Pharmacokinetics in Mice

ICR mice (Shanghai Institute of Planned Parenthood Research) of 6-8 weeks old, weighing 20-24 g, were randomized into groups of 9 after 3-5 days of acclimatization, and the test compounds at a dose of 15 mg/kg or 30 mg/kg were administered intragastrically to the mice.

The animals to be tested (ICR mice) were fasted for 12 h before administration and food was provided 4 h after administration, and water was freely drunk before and after the experiment and during the experiment.

After intragastric administration, about 0.1 mL of blood was collected from the orbit at 0.25 (15 min), 0.5 (30 min), 1, 2, 4, 6, 8, and 10 h. Each mouse was subjected to blood collection at 3-4 time points, with three mice per time point. The blood samples were mixed with EDTA-K2 anticoagulant, transferred to a centrifuge within 30 min, and then centrifuged at 4000 rpm for 10 min at 4 °C to separate the plasma. All the plasma samples were collected and immediately stored at -20 °C for testing.

400 µL of acetonitrile solution containing an internal standard (20 ng/mL) was added to 20 µL of the collected plasma sample to be tested. The mixture was shaken and mixed well for 10 min, and centrifuged at 13,000 rpm for 10 min. 50 µL of supernatant was taken and diluted with 100 µL of ultrapure water. After being mixed well, 0.5 µL of the resulting sample was subjected to LC/MS/MS, and a chromatogram was recorded.

Oral exposure of the compounds disclosed herein was evaluated by *in vivo* pharmacokinetic experiments in mice. The results are shown in Table 3 below.

**Table 3**

| PK parameter | Compound 5-j IG 30 mg/kg | Compound 6-j IG 30 mg/kg | Compound 34-h IG 15 mg/kg |
|---|---|---|---|
| AUC(0-10 h) (ng*h/mL) | 60771 | 61178 | 65315 |
| AUC(0-∞) (ng*h/mL) | 62618 | 63622 | 66669 |
| MRT(0-t) (h) | 6.26 | 4.74 | 4.44 |
| t1/2(h) | 4.48 | 6.26 | 4.19 |
| Tmax (h) | 0.5 | 0.5 | 4.00 |
| Cmax (ng/mL) | 8097 | 12534 | 10192 |

### Experimental Example 5: Pharmacodynamics in Mouse Colitis Model Induced by Anti-CD40 mAb Antibody

SPF-grade male CB17-SCID mice (6-8 weeks old) were divided into groups on the first day of the experiment. On the first day of the experiment, the mice in the blank group were intraperitoneally injected with PBS, while the mice in the model group were intraperitoneally injected with anti-mouse CD40 antibody (100 µg, diluted with PBS as a modeling agent solution), so as to establish a mouse colitis model. The injection volume for all groups of mice was 0.1 mL.

**Table 4. Administration Regimen of Example Compounds in Mouse Colitis Model**

| Group | | Modeling | Compound | Dose (mg/kg) | Route of administration | Frequency of administration |
|---|---|---|---|---|---|---|
| Group i^{(a)} | Blank control group | No | - | - | i.g. | bid |
| | Model control group | Yes | - | - | i.g. | bid |
| | Treatment group | Yes | 6-j | 5mg/kg | i.g. | bid |
| Group ii^{(b)} | Blank control group | No | - | - | i.g. | bid |
| | Model control group | Yes | - | - | i.g. | bid |
| | Treatment group | Yes | 34-h | 5mg/kg | i.g. | bid |
| Group iii^{(b)} | Blank control group | No | - | - | i.g. | bid |
| | Model control group | Yes | - | - | i.g. | bid |
| | Treatment group | Yes | 52-j | 15mg/kg | i.g. | bid |

| | | | | | | |
|---|---|---|---|---|---|---|
| Note: (a) indicates that the experimental animals were sourced from Shanghai Slac Laboratory Animal Co., Ltd; (b) indicates that the experimental animals were sourced from Shanghai Lingchang Biotechnology Co., Ltd. i.g.: intragastric administration; bid: administration twice daily. | | | | | | |

The day of grouping was determined as Day 1, and the intragastric administration was started on the grouping day, with a volume of 2 mL/kg administered twice daily for 6 consecutive days. The vehicle for intragastric administration was: DMSO:PEG400 = 5:95 (volume ratio).

Animal body weights were monitored daily during the experiment. On Day 7, the end point of the experiment, all mice were euthanized and tissue samples were collected. Mice were euthanized by cervical dislocation, abdomen of the mice were cut open, and mouse spleens were collected, weighed, and photographed. The colorectum was separated from the small intestine from the end of the cecum to the anus, and the whole colorectum was removed, photographed, and then dissected longitudinally along its longitudinal axis. The intestinal contents were cleansed with a pre-cooled saline solution. The rinsed colorectum was laid flat in a large flat dish with the mucosal side thereof facing upward. Residual saline solution was removed from the colorectum with filter paper, and colonic tissues were taken for weighing.

The inhibition rate (%) was calculated according to the following formula: inhibition rate (%) = (M-T) / (M-C) * 100% (T: treatment group; C: blank control group; M: model control group).

The results are shown in Table 5.

**Table 5. Effect on colitis mouse model (Mean ± SD)**

| Group | Day 7 of the experiment | | |
|---|---|---|---|
| | Spleen weight Inhibition rate (%) | Spleen coefficient Inhibition rate (%) | Weight of intestinal tissue per unit length Inhibition rate (%) |
| Group i: treatment group | 83.38% | 81.78%^{∗∗} | 76.21%^{∗∗∗} |
| Group ii: treatment group | 82%^{∗∗∗} | 83%^{∗∗∗} | 70%^{∗∗∗} |
| Group iii: treatment group | 102%^{∗∗∗} | 101%^{∗∗∗} | 101%^{∗∗∗} |

| | | | |
|---|---|---|---|
| Note: Compared with model control group, T-test, **P*<0.05, ***P*<0.01, ****P*<0.001. | | | |

## Claims

1. A compound of formula I or a pharmaceutically acceptable salt thereof: 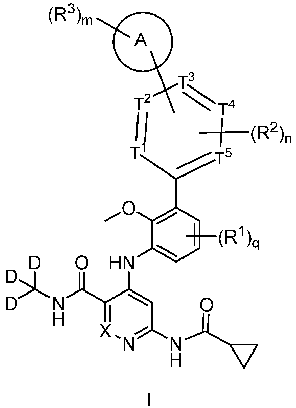 wherein,
X is selected from the group consisting of N and CH;
each R¹ is independently selected from halogen;
q is selected from the group consisting of 0, 1, and 2;
each R² is independently selected from the group consisting of halogen, hydroxy, amino, cyano, and nitro;
n is selected from the group consisting of 0, 1, and 2;
T¹, T², T³, T⁴, and T⁵ are each independently selected from the group consisting of CH and N, at least one of which is selected from CH;
ring A is selected from C₃₋₁₀ cycloalkyl, 3- to 10-membered heterocyclyl, C₆₋₁₀ aryl, and 5- to 10-membered heteroaryl;
each R³ is independently selected from the group consisting of =O, halogen, hydroxy, amino, cyano, nitro, C₁₋₈ alkyl, C₃₋₁₀ cycloalkyl, 3- to 10-membered heterocycloalkyl, 5- to 10-membered heteroaryl, C₆₋₁₀ aryl, and C₁₋₈ alkoxy, wherein the C₁₋₈ alkyl, C₃₋₁₀ cycloalkyl, 3- to 10-membered heterocycloalkyl, 5- to 10-membered heteroaryl, C₆₋₁₀ aryl, or C₁₋₈ alkoxy is optionally substituted with one or more R^{a};
m is selected from the group consisting of 0, 1, 2, 3, and 4;
each R^{a} is independently selected from the group consisting of halogen, hydroxy, amino, and cyano.

2. The compound or the pharmaceutically acceptable salt thereof according to claim 1, wherein X is N; alternatively, X is CH.

3. The compound or the pharmaceutically acceptable salt thereof according to claim 1 or 2, wherein each R¹ is independently selected from the group consisting of fluorine and chlorine;
alternatively, each R¹ is independently fluorine.

4. The compound or the pharmaceutically acceptable salt thereof according to any one of claims 1 to 3, wherein q is selected from the group consisting of 0 and 1;
alternatively, q is 0.

5. The compound or the pharmaceutically acceptable salt thereof according to any one of claims 1 to 4, wherein
each R² is independently selected from the group consisting of fluorine, chlorine, and bromine;
alternatively, each R² is independently selected from the group consisting of fluorine and chlorine;
alternatively, each R² is independently fluorine.

6. The compound or the pharmaceutically acceptable salt thereof according to any one of claims 1 to 5, wherein n is selected from the group consisting of 0 and 1;
alternatively, n is 0.

7. The compound or the pharmaceutically acceptable salt thereof according to any one of claims 1 to 6, wherein T¹, T², T³, T⁴, and T⁵ are each independently selected from the group consisting of CH and N, three of which are selected from N and two of which are selected from CH;
alternatively, T¹, T², T³, T⁴, and T⁵ are each independently selected from the group consisting of CH and N, two of which are selected from N and three of which are selected from CH;
alternatively, T¹, T², T³, T⁴, and T⁵ are each independently selected from the group consisting of CH and N, one of which is selected from N and four of which are selected from CH;
optionally, T¹, T³, and T⁵ are selected from N, and T² and T⁴ are selected from CH;
alternatively, T¹ and T³ are selected from N, and T², T⁴, and T⁵ are selected from CH;
alternatively, T¹ and T⁵ are selected from N, and T², T³, and T⁴ are selected from CH;
alternatively, T² and T⁴ are selected from N, and T¹, T³, and T⁵ are selected from CH;
alternatively, T¹ and T² are selected from N, and T³, T⁴, and T⁵ are selected from CH;
alternatively, T² and T³ are selected from N, and T¹, T⁴, and T⁵ are selected from CH;
alternatively, T¹ and T⁴ are selected from N, and T², T³, and T⁵ are selected from CH;
alternatively, T² and T³ are selected from N, and T¹, T⁴, and T⁵ are selected from CH;
alternatively, T¹ is selected from N, and T², T³, T⁴, and T⁵ are selected from CH;
alternatively, T² is selected from N, and T¹, T³, T⁴, and T⁵ are selected from CH;
alternatively, T³ is selected from N, and T¹, T², T⁴, and T⁵ are selected from CH,

8. The compound or the pharmaceutically acceptable salt thereof according to any one of claims 1 to 7, wherein ring A is selected from the group consisting of C₃₋₈ cycloalkyl, 3- to 8-membered heterocyclyl, C₆₋₁₀ aryl, and 5- to 8-membered heteroaryl;
alternatively, ring A is selected from the group consisting of C₃₋₆ cycloalkyl, 4- to 6-membered heterocyclyl, C₆₋₁₀ aryl, and 5- to 6-membered heteroaryl;
alternatively, ring A is selected from the group consisting of C₃₋₆ cycloalkyl, 4- to 6-membered heterocyclyl containing 1, 2, or 3 atoms selected from the group consisting of N, O, and S, C₆₋₁₀ aryl, and 5- to 6-membered heteroaryl containing 1, 2, or 3 atoms selected from the group consisting of N, O, and S;
alternatively, ring A is selected from the group consisting of C₃₋₈ cycloalkyl, 3- to 8-membered heterocycloalkyl, 3-to 8-membered heterocycloalkenyl, C₆₋₁₀ aryl, and 5- to 8-membered heteroaryl;
alternatively, ring A is selected from the group consisting of C₃₋₈ cycloalkyl, 4- to 6-membered heterocycloalkyl, 4-to 6-membered heterocycloalkenyl, C₆₋₁₀ aryl, and 5- to 8-membered heteroaryl;
optionally, ring A is selected from the group consisting of C₃₋₆ cycloalkyl, 4- to 6-membered heterocycloalkyl containing 1, 2, or 3 atoms selected from the group consisting of N, O, and S, 4- to 6-membered heterocycloalkenyl containing 1, 2, or 3 atoms selected from the group consisting of N, O, and S, C₆₋₁₀ aryl, and 5- to 6-membered heteroaryl containing 1, 2, or 3 atoms selected from the group consisting of N, O, and S;
alternatively, ring A is selected from the group consisting of 4- to 6-membered heterocycloalkyl containing 1, 2, 3 atoms selected from the group consisting of N, O, and S, 4- to 6-membered heterocycloalkenyl containing 1, 2, or 3 atoms selected from the group consisting of N, O, and S, and 5- to 6-membered heteroaryl containing 1, 2, or 3 atoms selected from the group consisting of N, O, and S;or
alternatively, ring A is selected from the group consisting of oxetanyl, azetidinyl, morpholinyl, dioxanyl, dihydropyridinyl, pyridinyl, pyrimidinyl, pyridazinyl, tetrahydropyrrolyl, triazolyl, tetrazolyl, pyrazolyl, furanyl, thienyl, dihydropyrimidinyl, oxazolyl, isoxazolyl, and imidazolyl;
alternatively, ring A is selected from the group consisting of oxetanyl, azetidinyl, morpholinyl, dihydropyridinyl, pyridinyl, pyrimidinyl, tetrahydropyrrolyl, triazolyl, pyrazolyl, furanyl, dihydropyrimidinyl, isoxazolyl, and imidazolyl;
alternatively, ring A is selected from the group consisting of oxetanyl, morpholinyl, dihydropyridinyl, pyridinyl, pyrimidinyl, tetrahydropyrrolyl, triazolyl, pyrazolyl, furanyl, dihydropyrimidinyl, isoxazolyl, and imidazolyl;
alternatively, ring A is selected from the group consisting of 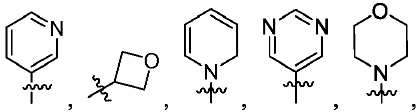 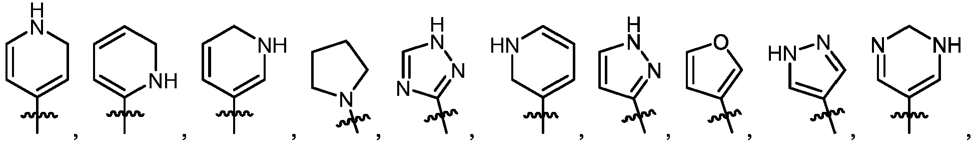 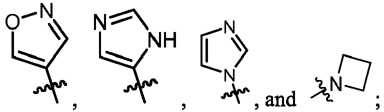
optionally, ring A is selected from the group consisting of 4- to 6-membered heterocyclyl containing 1 or 2 atoms selected from the group consisting of N and O and 6-membered heteroaryl containing 1 or 2 atoms selected from N; alternatively, ring A is selected from the group consisting of 4- to 6-membered heterocycloalkyl containing 1 or 2 atoms selected from the group consisting of N and O, 4- to 6-membered heterocycloalkenyl containing 1 or 2 atoms selected from N, and 6-membered heteroaryl containing 1 or 2 atoms selected from N;
alternatively, ring A is selected from the group consisting of oxetanyl, morpholinyl, 1,2-dihydropyridinyl, pyridinyl, and pyrimidinyl.

9. The compound or the pharmaceutically acceptable salt thereof according to any one of claims 1 to 8, wherein m is selected from the group consisting of 0, 1, 2, and 3;
optionally, m is selected from the group consisting of 0, 1, and 2.

10. The compound or the pharmaceutically acceptable salt thereof according to any one of claims 1 to 9, wherein each R³ is independently selected from the group consisting of =O, halogen, hydroxy, amino, cyano, nitro, C₁₋₆ alkyl, C₃₋₆ cycloalkyl, 3- to 6-membered heterocycloalkyl, 5- to 6-membered heteroaryl, C₆₋₁₀ aryl, and C₁₋₆ alkoxy, wherein the C₁₋₆ alkyl, C₃₋₆ cycloalkyl, 3- to 6-membered heterocycloalkyl, 5- to 6-membered heteroaryl, C₆₋₁₀ aryl, or C₁₋₆ alkoxy is optionally substituted with one or more R^{a};
alternatively, each R³ is independently selected from the group consisting of =O, halogen, hydroxy, amino, cyano, nitro, C₁₋₄ alkyl, C₃₋₆ cycloalkyl, 3- to 6-membered heterocycloalkyl, and C₁₋₄ alkoxy, wherein the C₁₋₄ alkyl, C₃₋₆ cycloalkyl, 3- to 6-membered heterocycloalkyl, or C₁₋₄ alkoxy is optionally substituted with one or more R^{a};
alternatively, each R³ is independently selected from the group consisting of =O, halogen, hydroxy, amino, cyano, nitro, C₁₋₃ alkyl, C₃₋₄ cycloalkyl, and C₁₋₃ alkoxy, wherein the C₁₋₃ alkyl, C₃₋₄ cycloalkyl, or C₁₋₃ alkoxy is optionally substituted with one or more R^{a};
alternatively, each R³ is independently selected from the group consisting of =O, fluorine, chlorine, hydroxy, amino, cyano, cyclopropyl, and C₁₋₃ alkyl optionally substituted with one or more fluorine;
alternatively, each R³ is independently selected from the group consisting of =O, fluorine, hydroxy, amino, cyano, methyl, trifluoromethyl, and cyclopropyl;
optionally, each R³ is independently selected from the group consisting of =O, halogen, hydroxy, amino, cyano, nitro, C₁₋₈ alkyl, and C₁₋₈ alkoxy, wherein the C₁₋₈ alkyl or C₁₋₈ alkoxy is optionally substituted with one or more R^{a};
alternatively, each R³ is independently selected from the group consisting of =O, halogen, hydroxy, amino, cyano, nitro, C₁₋₆ alkyl, and C₁₋₆ alkoxy, wherein the C₁₋₆ alkyl or C₁₋₆ alkoxy is optionally substituted with one or more R^{a}; alternatively, each R³ is independently selected from the group consisting of =O, halogen, hydroxy, amino, cyano, nitro, C₁₋₃ alkyl, and C₁₋₃ alkoxy, wherein the C₁₋₃ alkyl or C₁₋₃ alkoxy is optionally substituted with one or more R^{a}; alternatively, each R³ is independently selected from the group consisting of =O, fluorine, chlorine, bromine, and C₁₋₃ alkyl, wherein the C₁₋₃ alkyl is optionally substituted with one or more R^{a};
alternatively, each R³ is independently selected from the group consisting of =O, fluorine, and methyl, wherein the methyl is optionally substituted with one or more fluorine.

11. The compound or the pharmaceutically acceptable salt thereof according to any one of claims 1 to 10, wherein
each R^{a} is independently selected from the group consisting of halogen and cyano;
alternatively, each R^{a} is independently selected from the group consisting of fluorine, chlorine, and bromine;
alternatively, each R^{a} is independently fluorine.

12. The compound or the pharmaceutically acceptable salt thereof according to any one of claims 1 to 11, wherein structural unit 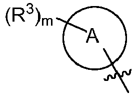 is selected from the group consisting of 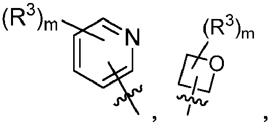 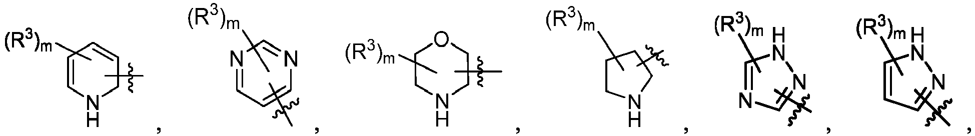 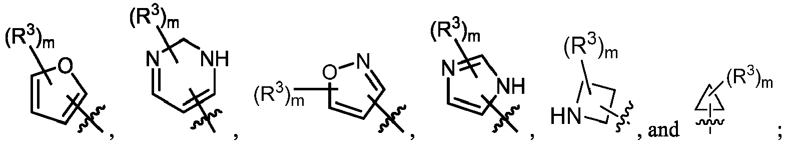 alternatively, structural unit 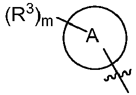 is selected from the group consisting of 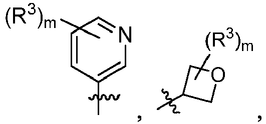 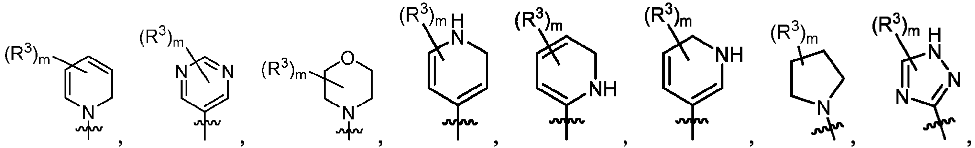 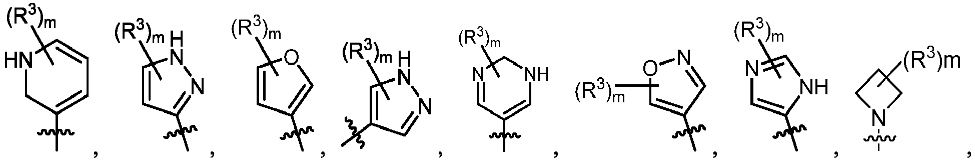 and 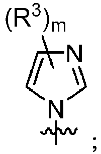 alternatively, structural unit 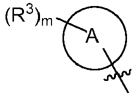 is selected from the group consisting of 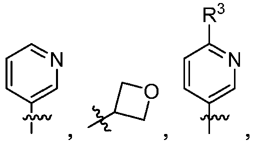 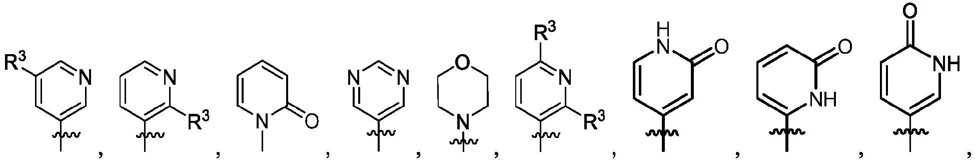 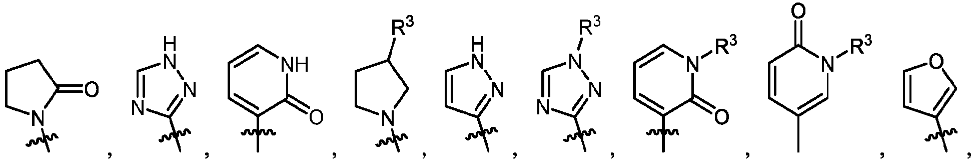 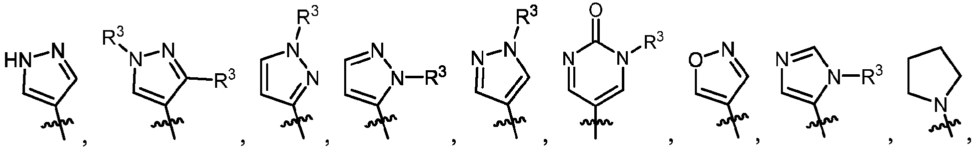 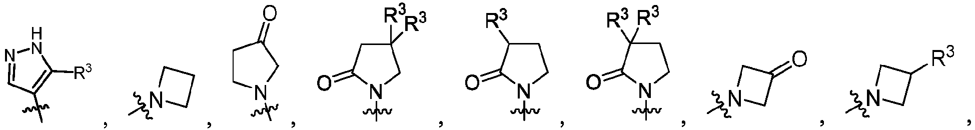 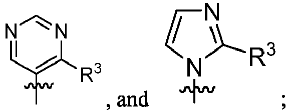 alternatively, structural unit 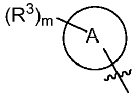 is selected from the group consisting of

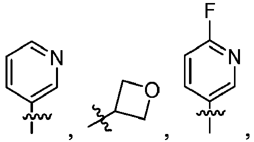

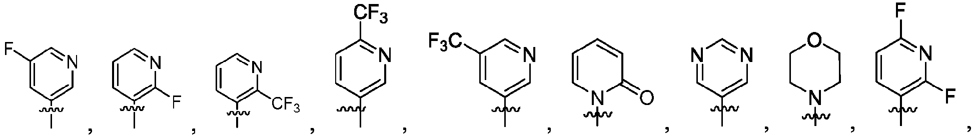

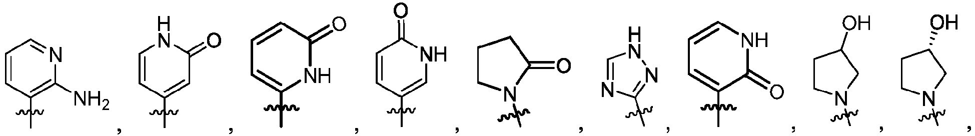

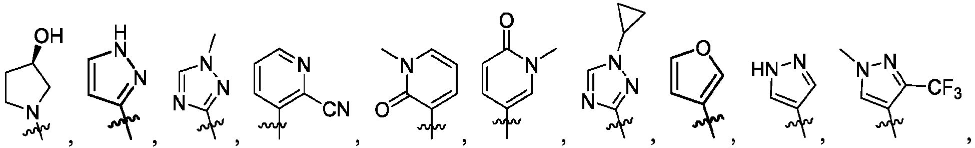

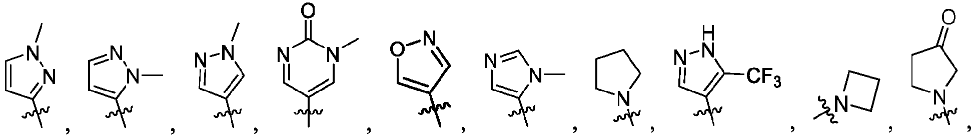

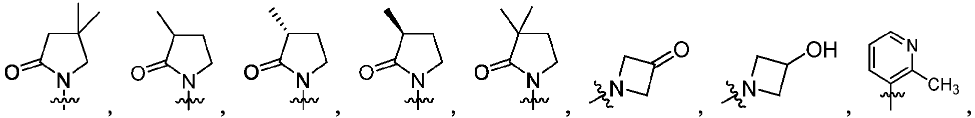

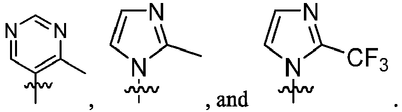

13. The compound or the pharmaceutically acceptable salt thereof according to any one of claims 1 to 12, wherein structural unit 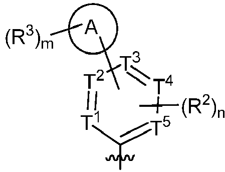 is selected from the group consisting of 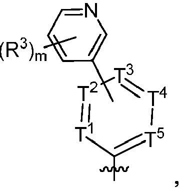 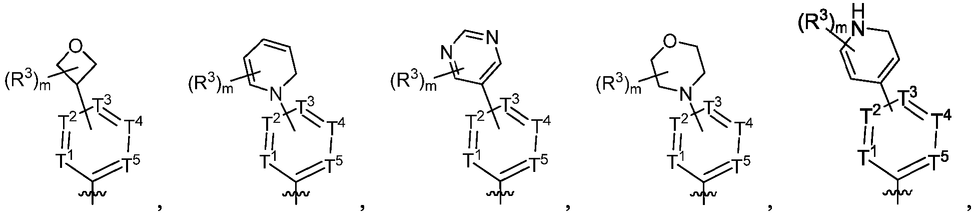 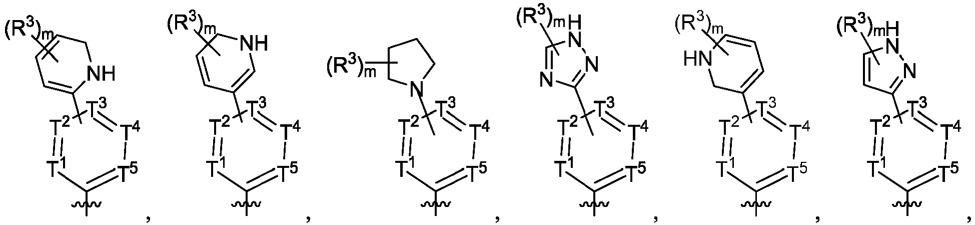 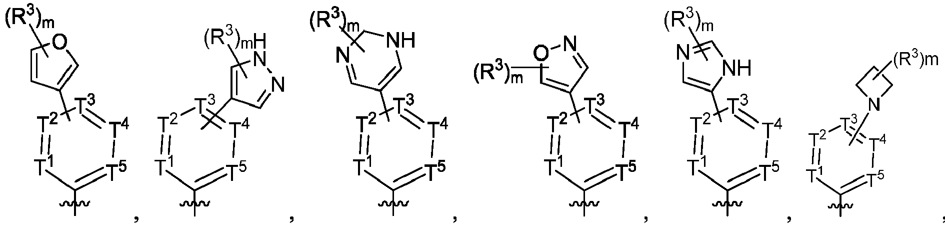 , and 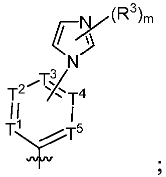 alternatively, structural unit 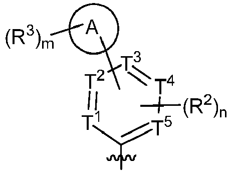 is selected from the group consisting of 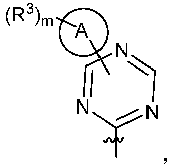 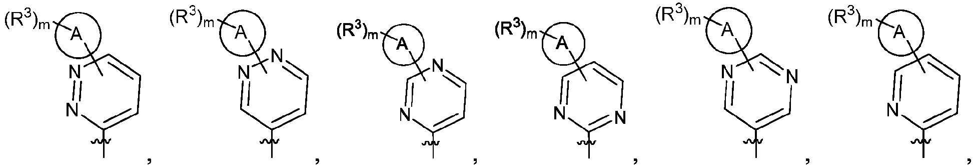 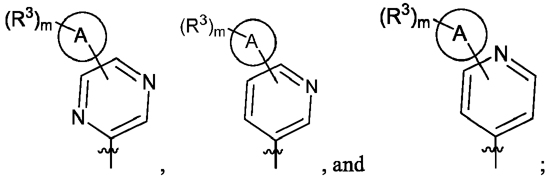 alternatively, structural unit 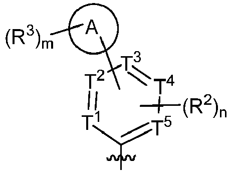 is selected from the group consisting of 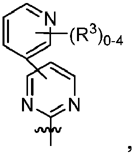 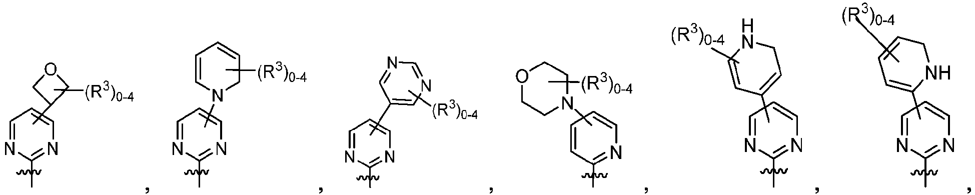 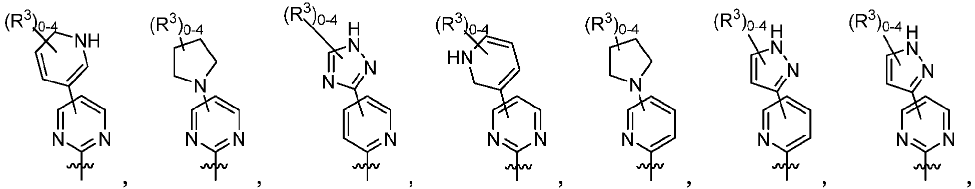 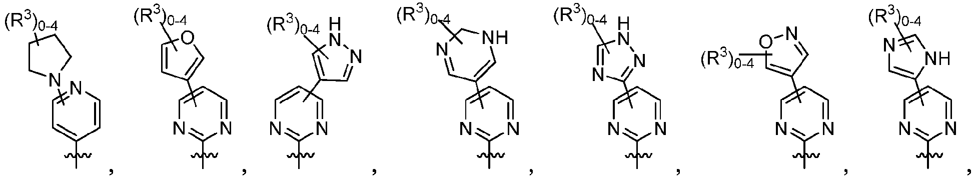 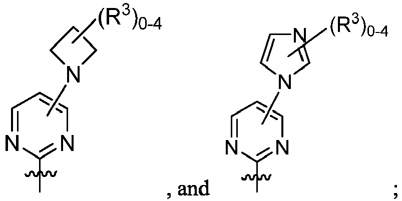 alternatively, structural unit 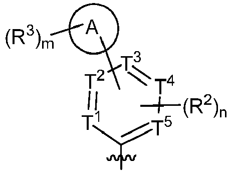 is selected from the group consisting of 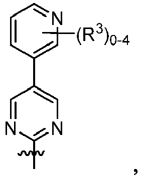 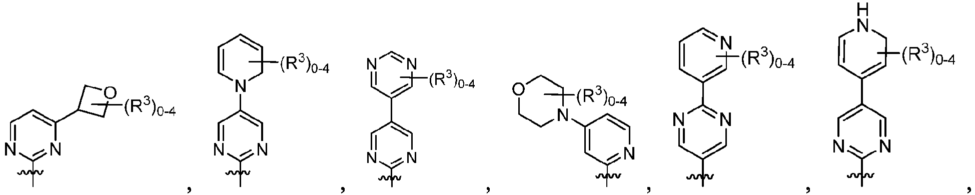 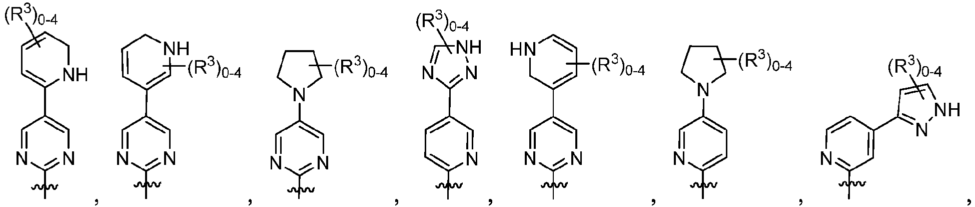 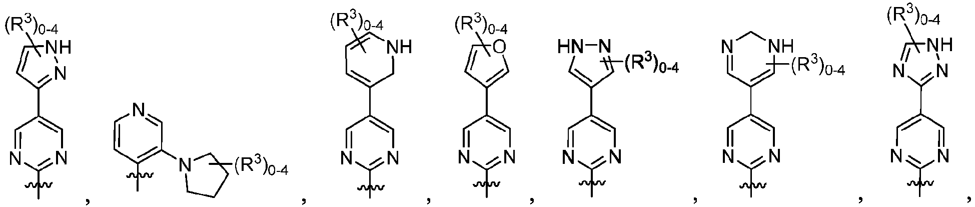 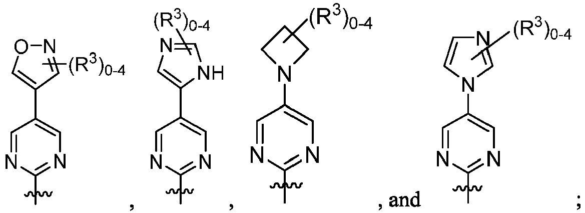 alternatively, structural unit 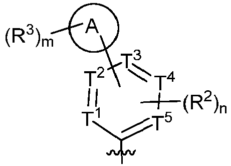 is selected from the group consisting of 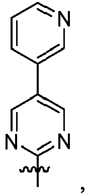 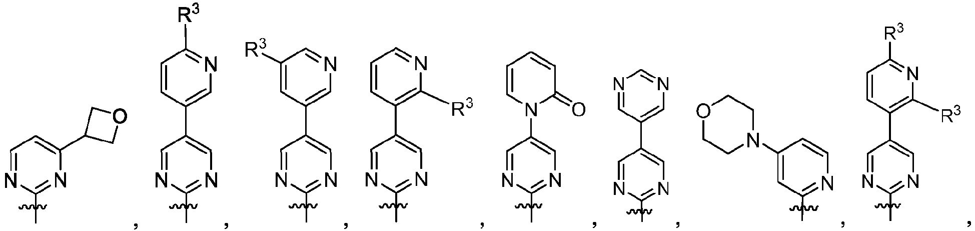 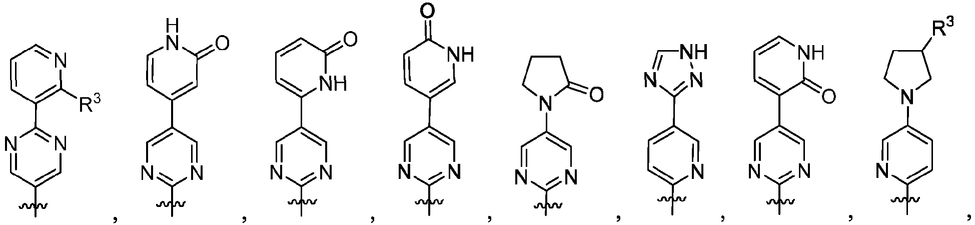 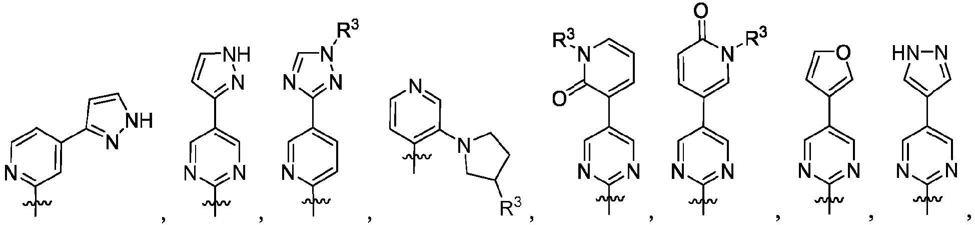 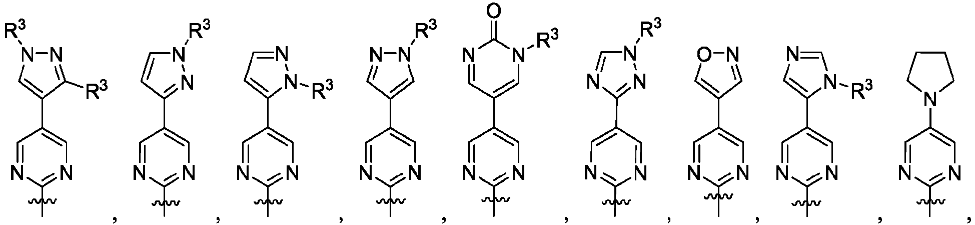 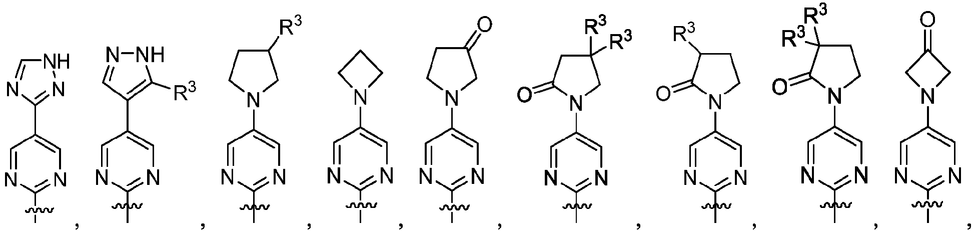 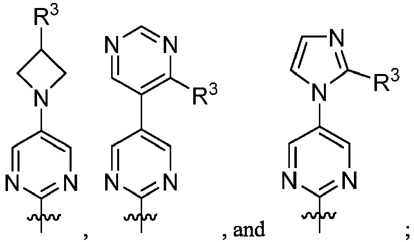 alternatively, structural unit 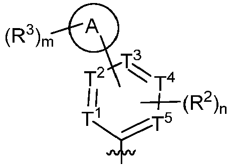 is selected from the group consisting of

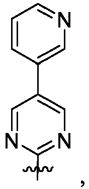

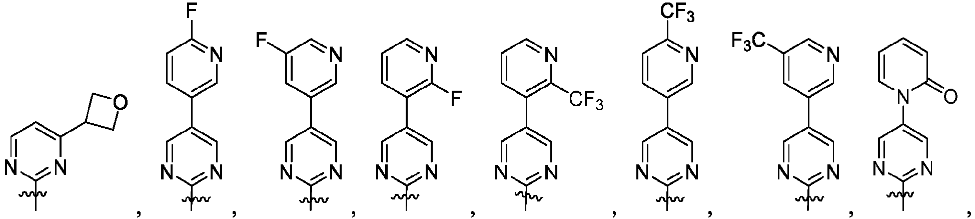

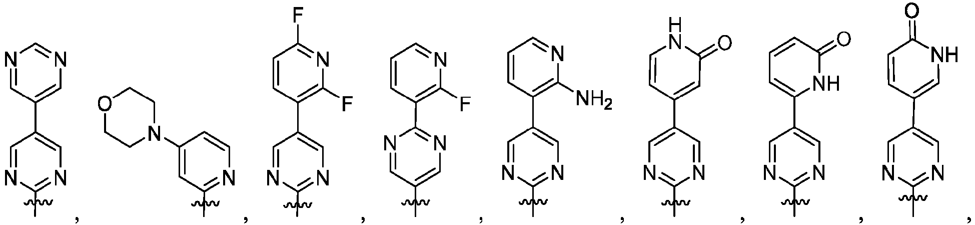

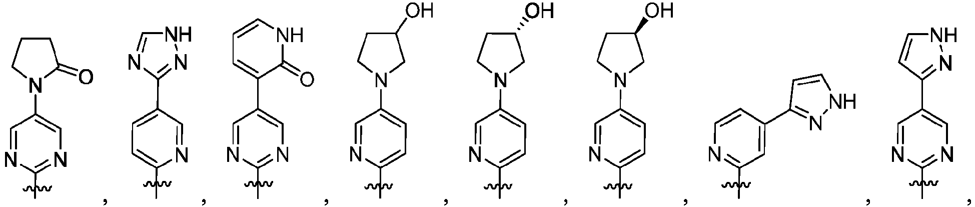

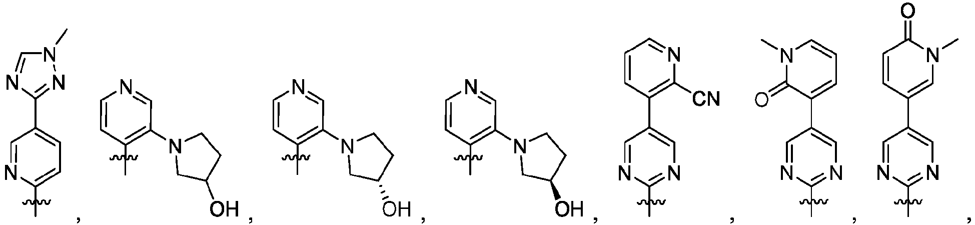

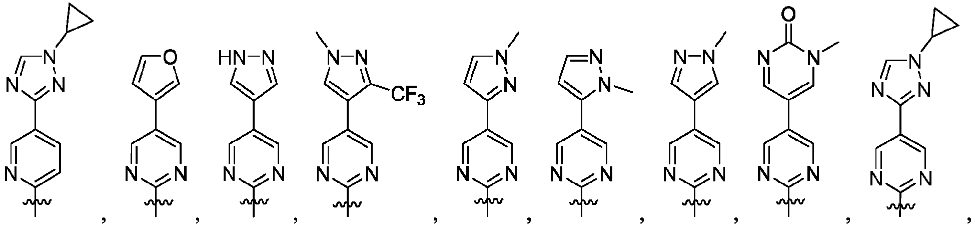

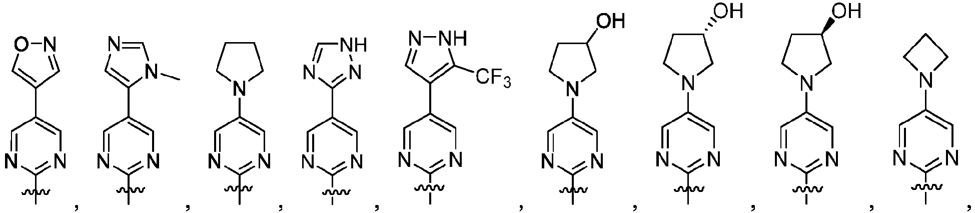

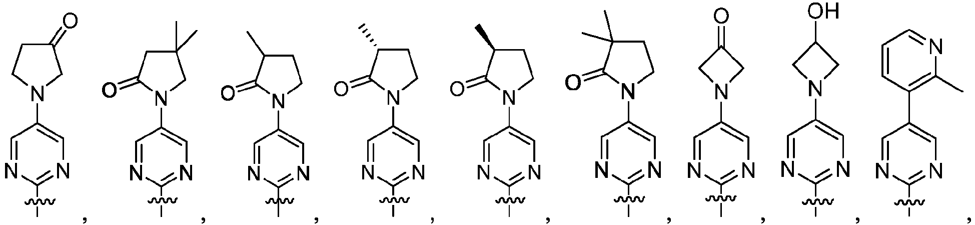

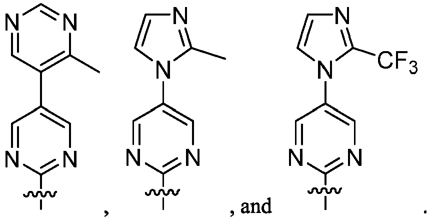

14. The compound or the pharmaceutically acceptable salt thereof according to any one of claims 1 to 13, selected from the group consisting of a compound of formula I-1, a compound of formula I-2, a compound of formula I-3, a compound of formula I-4, a compound of formula I-5, a compound of formula I-6, a compound of formula I-7, and pharmaceutically acceptable salts thereof: 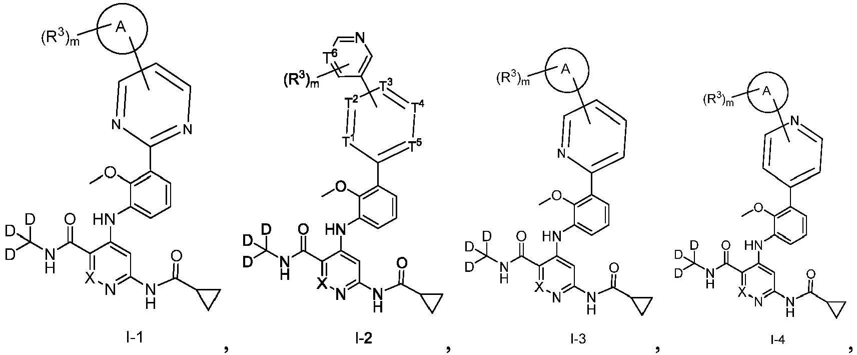 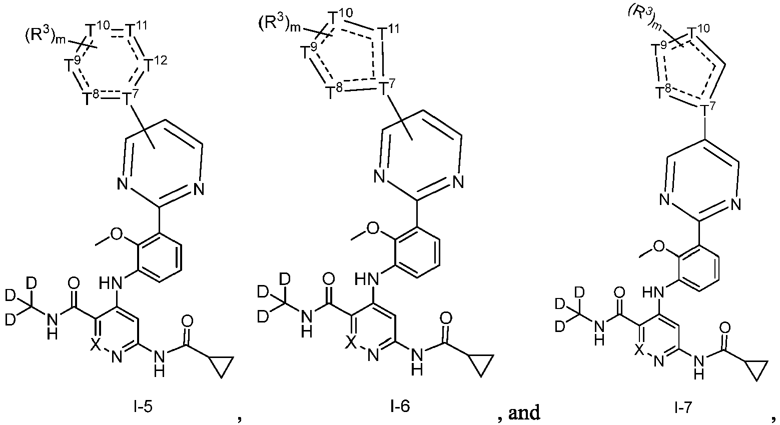
wherein, T⁶ is selected from the group consisting of CH and N;
T⁷, T⁸, T⁹, T¹⁰, T¹¹, and T¹² are each independently selected from the group consisting of CH, C, NH, N, O, and a bond, and 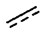 represents a single bond or double bond;
alternatively, T⁷, T⁸, T⁹, T¹⁰, T¹¹, and T¹² are each independently selected from the group consisting of CH, C, NH, N, O, and a bond, one or two of which are selected from the group consisting of NH and N;
alternatively, T⁷, T⁸, T⁹, T¹⁰, T¹¹, and T¹² are each independently selected from the group consisting of CH, C, NH, N, O, and a bond, one or two of which are selected from the group consisting of NH and N and one of which is selected from a bond;
optionally, structural unit 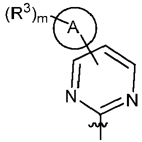 is selected from the group consisting of 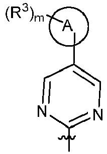 and 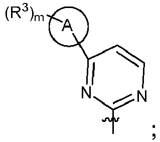
optionally, structural unit 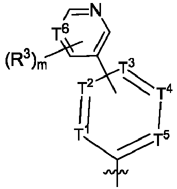 is selected from 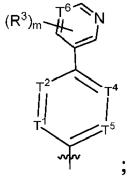
optionally, structural unit 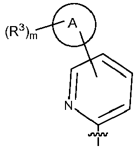 is selected from the group consisting of 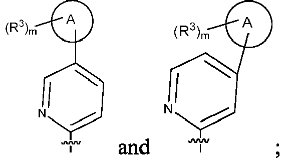
optionally, structural unit 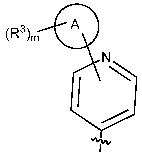 is selected from 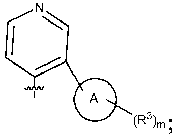
optionally, structural unit 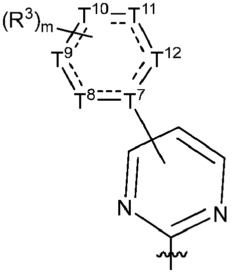 is selected from 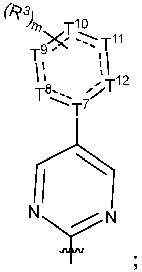
optionally, structural unit 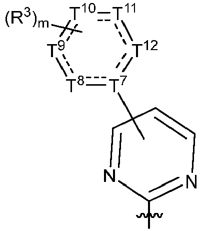 is selected from the group consisting of 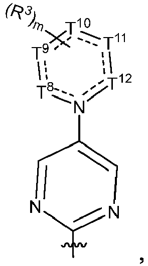 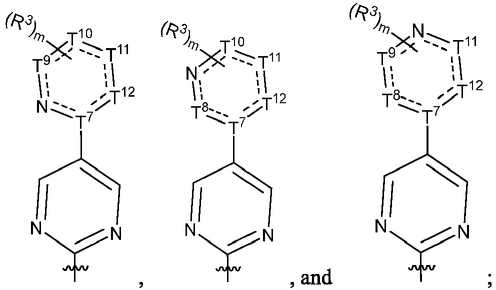
optionally, structural unit 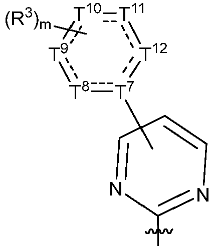 is selected from the group consisting of 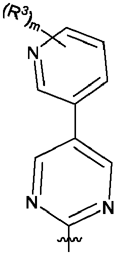 and 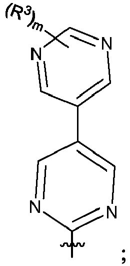
optionally, structural unit 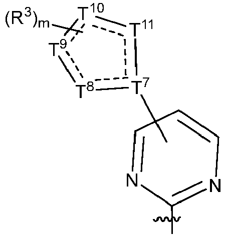 is selected from 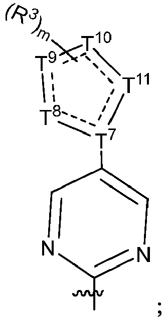
optionally, structural unit 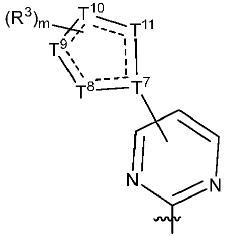 is selected from the group consisting of 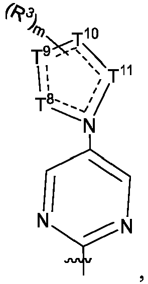 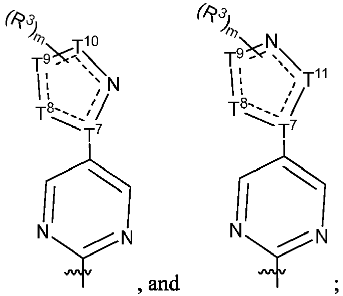
optionally, structural unit 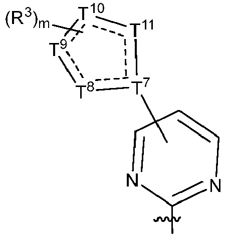
is selected from the group consisting of

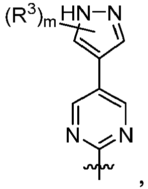

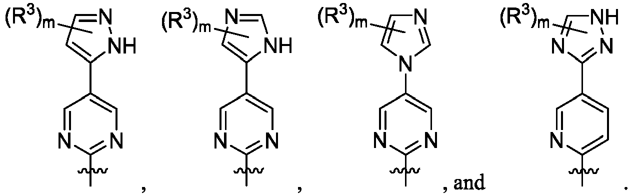

15. A compound of a formula selected from the group consisting of the following formulas or a pharmaceutically acceptable salt thereof:

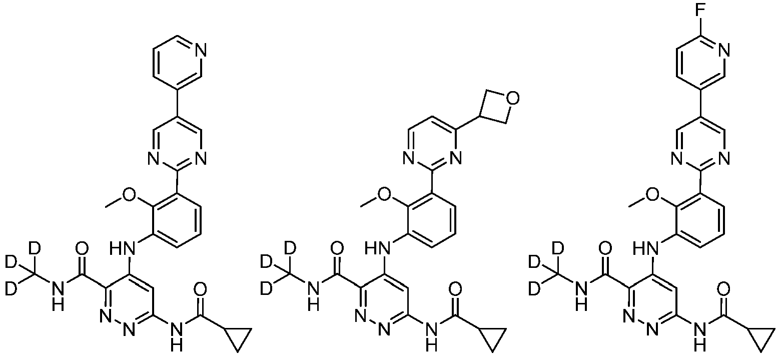

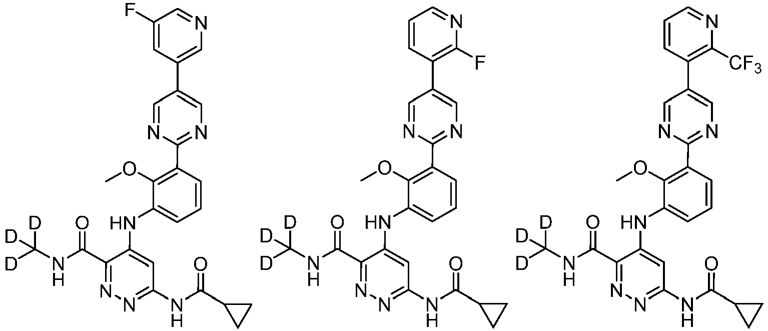

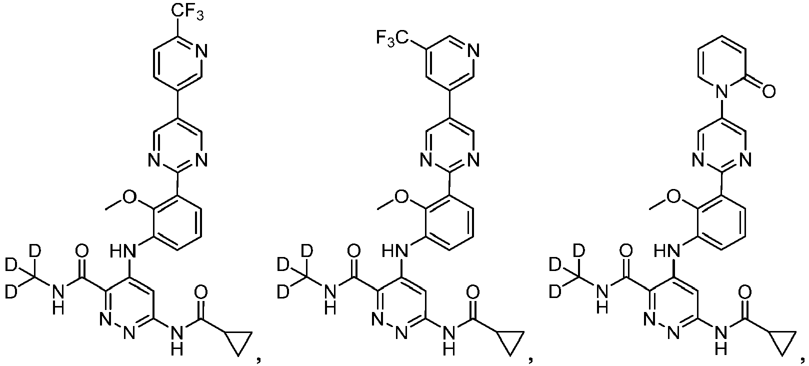

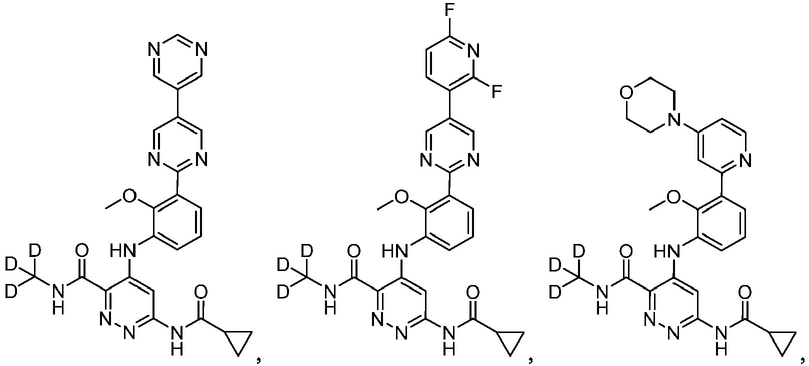

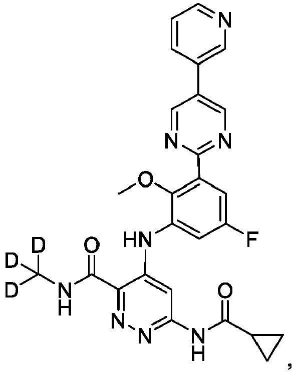

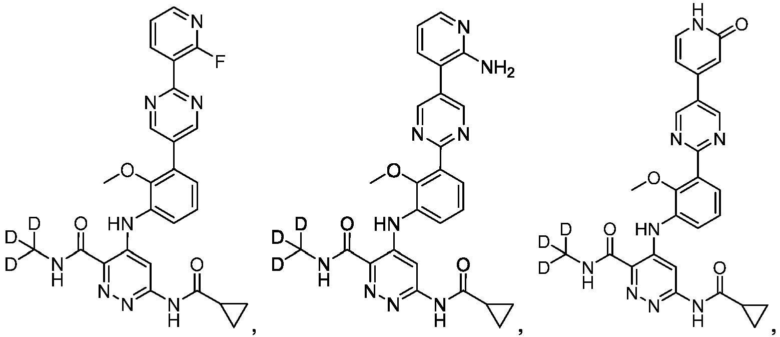

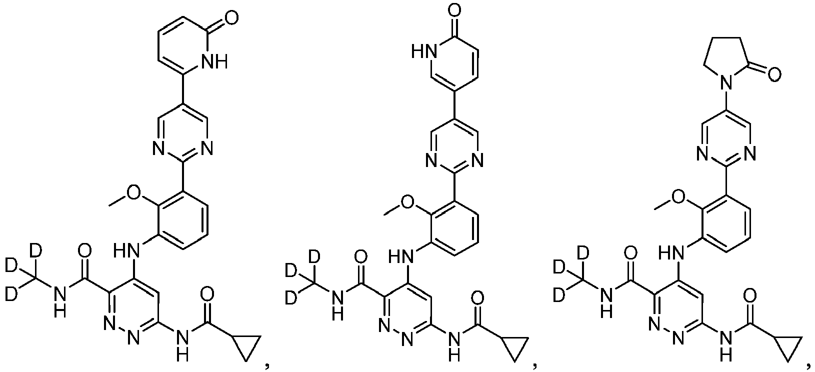

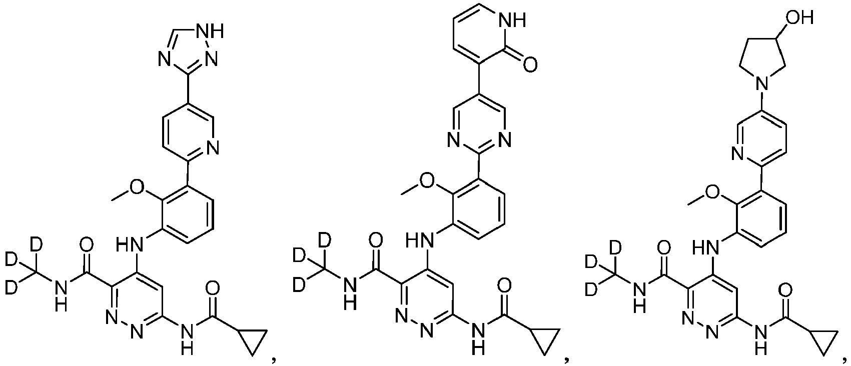

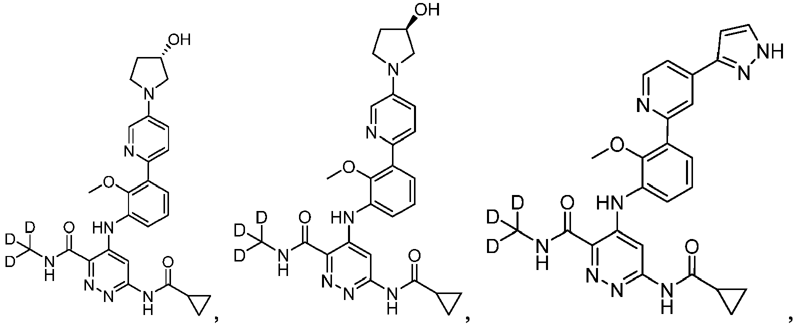

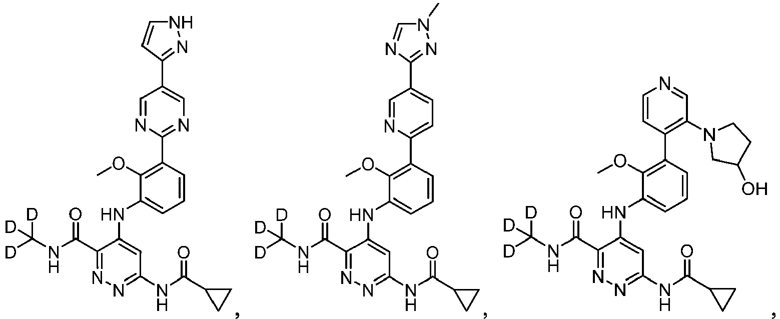

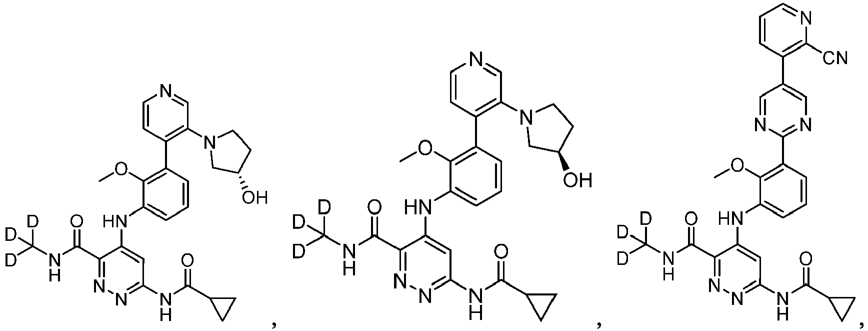

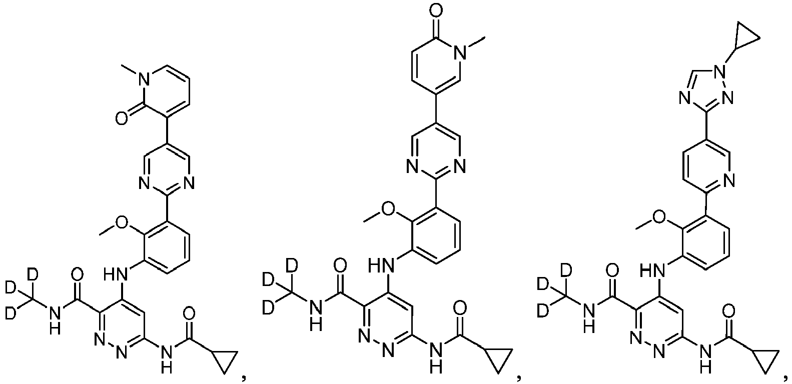

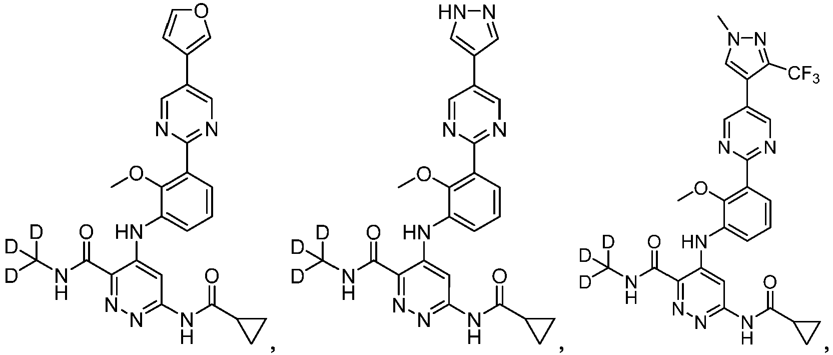

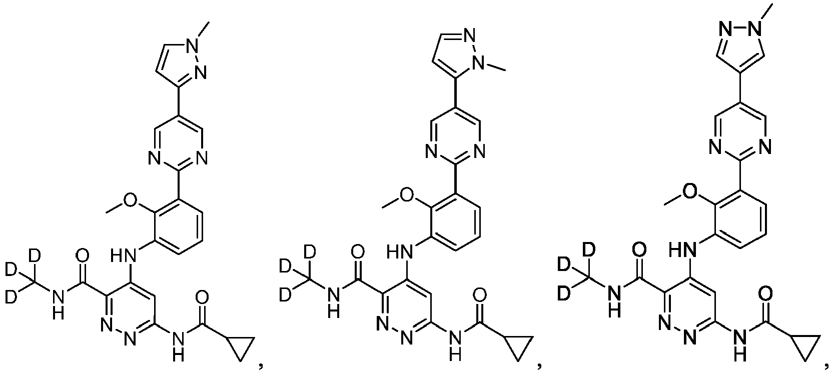

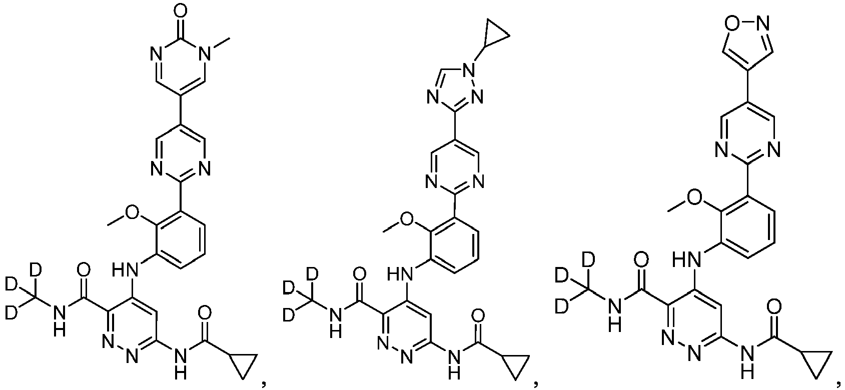

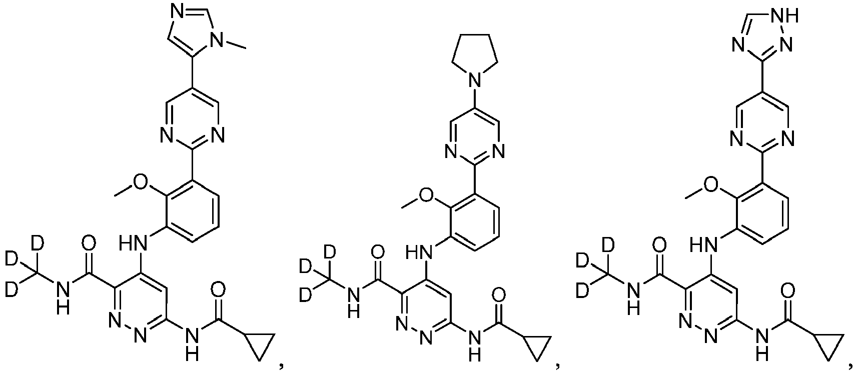

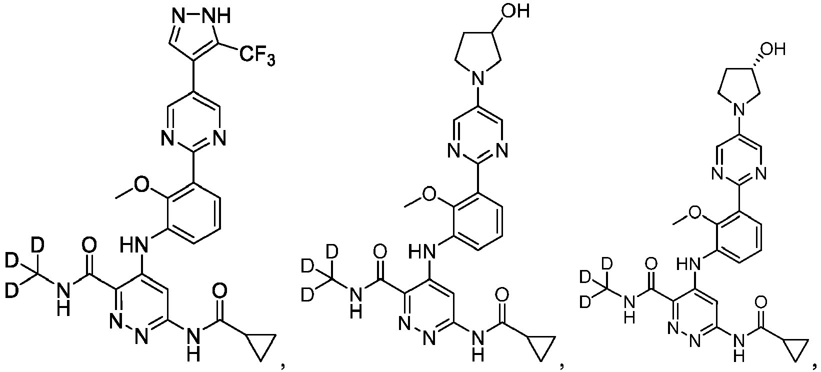

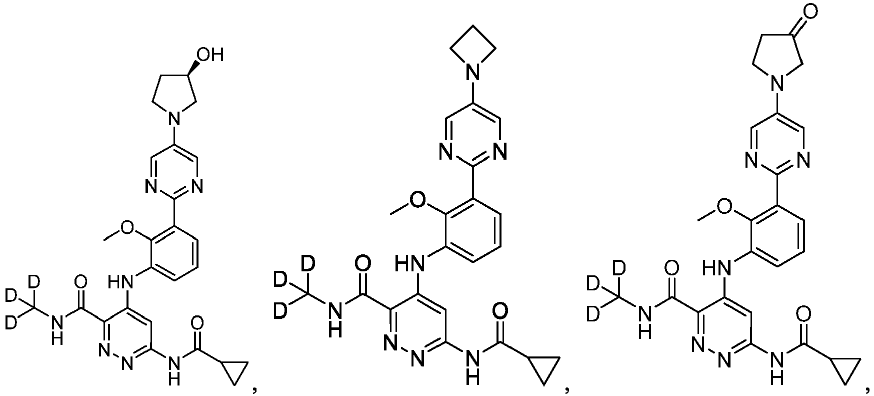

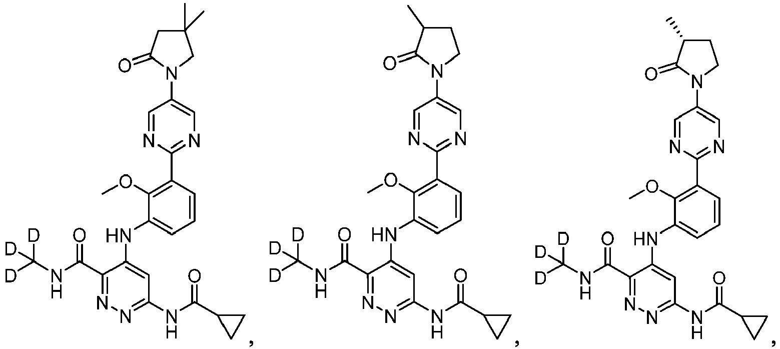

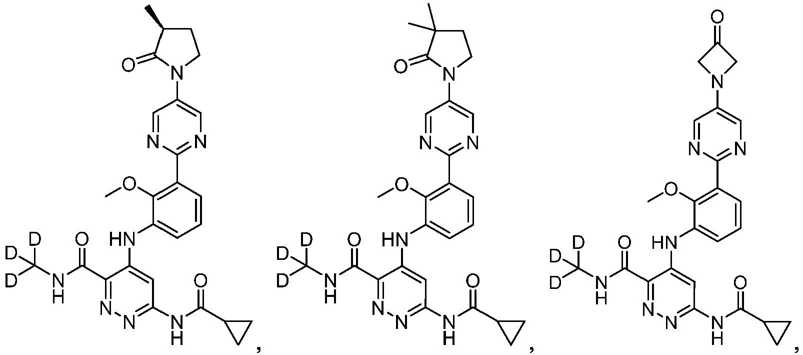

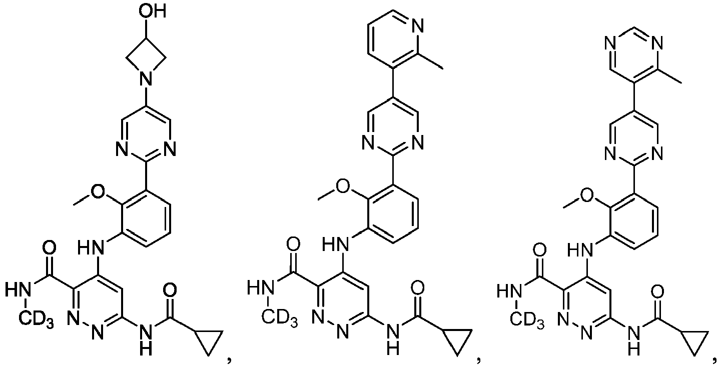

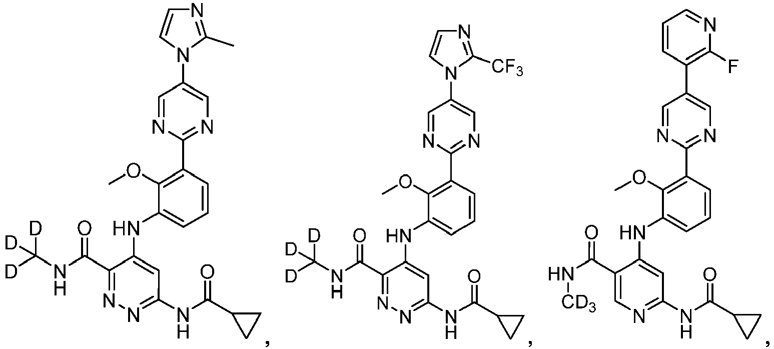

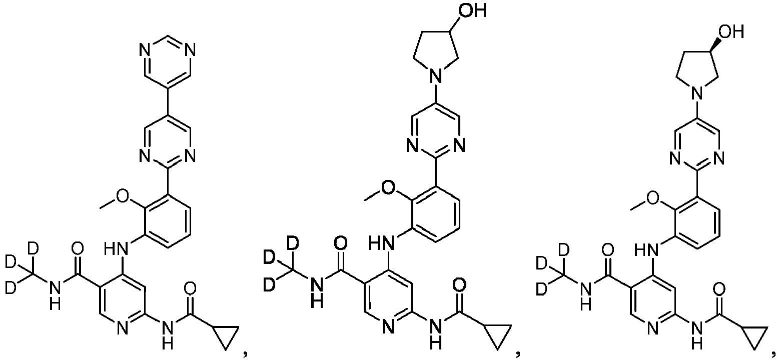

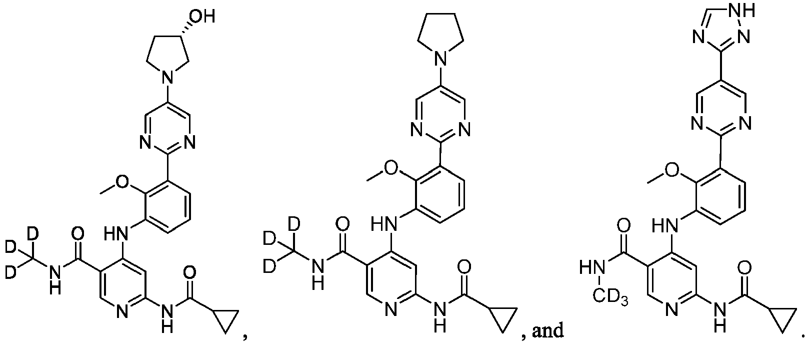

16. A pharmaceutical composition comprising the compound or the pharmaceutically acceptable salt thereof according to any one of claims 1 to 15.

17. Use of the compound or the pharmaceutically acceptable salt thereof according to any one of claims 1 to 15 and the pharmaceutical composition according to claim 16 in preparing a medicament for use in treating or preventing a TYK2-related disease.

18. The use according to claim 17, wherein the TYK2-related disease is selected from autoimmune diseases.
